# EUROPEAN PATENT SPECIFICATION

(11) **EP 3 385 376 B1**
(45) Date of publication and mention of the grant of the patent: **22.12.2021**
(21) Application number: 17164879.3
(22) Date of filing: 05.04.2017
(51) Int. Cl.: C12N 9/24, C09K 8/88, C11D 3/386, A23K 20/189

(54) **FUNGAL MANNANASES**
PILZMANNANASEN
MANNANASES FONGIQUES

(43) Date of publication of application: 10.10.2018
(73) Proprietor: AB Enzymes Oy, 05200 Rajamäki (FI)
(72) Inventor: Leinonen, Taija, 11100 Riihimäki (FI); Valtakari, Leena, 05200 Rajamäki (FI); Seefried, Michael, 63762 Großostheim (DE); Juntunen, Kari, 02680 Espoo (FI); Järvinen, Kristiina, 02940 Espoo (FI); Mäkinen, Susanna, 12600 Läyliäinen (FI); Paloheimo, Marja, 01450 Vantaa (FI); Kallio, Jarno, 04430 Järvenpää (FI); Vehmaanperä, Jari, 00980 Helsinki (FI); Ojapalo, Pentti, 04360 Tuusula (FI); Puranen, Terhi, 01900 Nurmijärvi (FI); Herbst, Daniela, 40237 Düsseldorf (DE); Wieland, Susanne, 41541 Zons/Dormagen (DE); Mussmann, Nina, 47877 Willich (DE)
(74) Representative: Espatent Oy

(56) References cited:
- EP-A1- 2 287 318
- WO-A1-2017/021515
- WO-A1-2017/021518
- WO-A2-2015/040159
- DATABASE UniProt [Online] 11 November 2015 (2015-11-11), "SubName: Full=Probable mannan endo-1,4-beta-mannosidase A {ECO:0000313|EMBL:GAO85867.1};", XP002774160, retrieved from EBI accession no. UNIPROT:A0A0K8LCG6 Database accession no. A0A0K8LCG6

## Description

### FIELD OF THE INVENTION

This invention relates to fungal mannanase enzymes. The mannanases are useful in industrial applications wherein degradation or modification of mannan is desired, such as in laundry and cleaning applications, in feed, food, pulp and oil industry. The invention also provides useful mannanase enzymes, polynucleotides encoding these enzymes, enzyme compositions and methods for their production and use.

### BACKGROUND

Mannans are mannose containing polysaccharides found in various plants. Mannans are poorly soluble in an aqueous environment and their physicochemical properties give rise to viscous dispersions. Additionally, mannans have high water binding capacity. All of these characteristics cause problems in several industries including brewing, baking, animal nutrition, and laundry and cleaning applications.

In plant based diets different β-mannans are present and, depending on their amounts and properties, they can compromise nutrient digestion, microbial colonisation and growth performance. Enzymatic degradation of mannans reduces digesta viscosity of high water soluble mannans and leads to production of manno-oligosaccharides that may form water-insoluble linear mannans present in leguminoseae. Mannanase increases average daily gain, feed efficiency, weight uniformity and livability in all monogastric animals.

For animal feed applications, such as feed for monogastric animals with cereal diets, mannan is a contributing factor to viscosity of gut contents and it thereby adversely affects the feed digestibility and animal growth rate. For ruminants, mannan represents a substantial component of fiber intake and a more complete digestion of mannan would facilitate higher feed conversion efficiencies.

For laundry and cleaning applications the present enzyme compositions comprising mannanase can be used to degrade mannan. However, providing mannanases that are stable in varying storage and use conditions while still showing good mannan degrading activity is difficult.

Database accession no. A0A0K8LCG6 (DATABASE UniProt [Online] 11 November 2015 (2015-11-11), "SubName: Full=Probable mannan endo-1,4-beta-mannosidase A {ECO:0000313|EMBL:GA085867.1};", retrieved from EBI accession no. UNIPROT:AOAOK8LCG6) discloses a protein from *Aspergillus udagawae* with a putative function as beta-1,4-endomannanase.

WO 2017/021515 A1 discloses isolated polypeptides from *Preussia aemulans* that have mannanase activity.

It is an object of the present invention to provide novel enzymes exhibiting mannanase activity when applied in different industrial processes, as well as enzyme compositions for mannan degradation or modification.

### SUMMARY

According to a first aspect is provided a recombinant polypeptide having mannanase activity and comprising an amino acid sequence, which has:
at least 92% sequence identity with Man13 (SEQ ID NO: 30).

The recombinant polypeptide is advantageous in that it can be used in various industrial processes wherein mannan degradation is needed. The recombinant polypeptide shows activity in various pH values and temperatures. The recombinant polypeptides according to the invention that have fungal origin are particularly effective in remaining stable and degrading mannan even in acidic conditions. They are effective also in processing biomasses.

According to a second aspect is provided a recombinant host cell comprising genetic elements that allow producing at least one recombinant polypeptide with mannanase activity, which has at least 92 % sequence identity with Man13 (SEQ ID NO: 30); and
wherein the host cell is selected from a group consisting of fungal cells,
filamentous fungal cells from Division *Ascomycota,* Subdivision *Pezizomycotina*; preferably from the group consisting of members of the Class *Sordariomycetes,* Subclass *Hypocreomycetidae,* Orders *Hypocreales* and *Microascales* and *Aspergillus, Chrysosporium, Myceliophthora* and *Humicola;*
more preferably from the group consisting of Families *Hypocreacea*, *Nectriaceae*, *Clavicipitaceae, Microascaceae,* and Genera *Trichoderma* (anamorph of *Hypocrea), Fusarium, Gibberella, Nectria, Stachybotrys, Claviceps, Metarhizium, Villosiclava, Ophiocordyceps, Cephalosporium,* and *Scedosporium;*
more preferably from the group consisting of *Trichoderma reesei (Hypocrea jecorina)*, *T. citrinoviridae, T. longibrachiatum, T. virens, T. harzianum, T. asperellum, T. atroviridae, T. parareesei, Fusarium oxysporum, F. gramineanum, F. pseudograminearum, F. venenatum, Gibberella fujikuroi, G. moniliformis, G. zeaea, Nectria (Haematonectria) haematococca, Stachybotrys chartarum, S. chlorohalonata, Claviceps purpurea, Metarhizium acridum, M. anisopliae, Villosiclava virens, Ophiocordyceps sinensis, Acremonium (Cephalosporium) chrysogenum,* and *Scedosporium apiospermum, and Aspergillus niger, Aspergillus awamori, Aspergillus oryzae, Chrysosporium lucknowense, Myceliophthora thermophila, Humicola insolens,* and *Humicola grisea,* bacterial cells, preferably gram positive Bacilli such as *B*. *subtilis, B. licheniformis, B. megaterium, B. amyloliquefaciens, B. pumilus,* gram negative bacteria such as *Escherichia coli,* actinomycetales such as *Streptomyces* sp., and
yeasts such as *Saccharomyces cerevisiae, Pichia pastoris, Yarrowia lipolytica,* most preferably *Trichoderma reesei or Bacillus.*

The recombinant host cell can be used to produce mannanase and to carry a polynucleotide encoding mannanase. The recombinant host cell is useful also in preparation of mannanases with different properties. For example, a host cell can be selected, which provides post-translational modifications beneficial for stability or activity, or which facilitates post-processing and formulation of mannanase produced in the host cell.

According to a third aspect is provided a recombinant polypeptide having mannanase activity and obtainable by using the host cell of the second aspect.

The recombinant polypeptide may have structural or functional properties that differentiate it from a native polypeptide having the same or similar amino acid sequence. For example, a host cell can be selected for production, which provides the produced recombinant polypeptide with post-translational modifications, a lack thereof, or localization to facilitate production and/or formulation of the recombinant polypeptide.

According to a fourth aspect is provided an enzyme composition comprising a recombinant polypeptide having mannanase activity and at least 92 % sequence identity with Man13 (SEQ ID NO: 30); .

The present enzyme composition is advantageous in having good stability and mannanase activity in detergents and in formulations. It is also suitable for various industrial applications wherein mannan degradation or modification is desired. The enzyme composition is suitable for degrading and modifying mannan containing material in various chemical environments.

According to a fifth aspect is provided a method for producing mannanase comprising
a. cultivating a recombinant host cell of the second aspect, wherein
   i. the genetic elements comprise at least one control sequence which controls the production of the recombinant polypeptide in the recombinant host cell under conditions that allow production of the polypeptide;
   ii. the genetic elements optionally comprise at least one sequence encoding a signal sequence for transporting the polypeptide outside the host cell; and
   iii. cultivating is carried out in conditions allowing production of the polypeptide; and
b. recovering the mannanase.

The method provides an efficient way to produce mannanase. Because the mannanase is produced in a recombinant host cell, a mannanase production system is provided which can be optimized, tailored, and controlled in a desired manner. The mannanase produced by the method may differ from natural mannanases at a structural level. The mannanase produced by the method can e.g. have a glycosylation pattern, or other post translational modification, which causes differences in the structure and/or function when compared to a natural mannanase, such as a mannanase having similar or the same amino acid sequence, or compared to a mannanase having the same amino acid sequence but produced in another host cell. The mannanase produced by the method can be used as such or formulated into a selected formulation.

According to another aspect is provided an enzyme preparation comprising a recombinant polypeptide having mannanase activity and obtainable by using the host cell of the second aspect.

The enzyme preparation or composition may further comprise other enzyme(s) selected from the group consisting of proteases, amylases, cellulases, lipases, xylanases, mannanases, cutinases, esterases, phytases, DNAses, pectinases, pectinolytic enzymes, xanthanases, xyloglucanases, laccases, peroxidases and oxidases with or without a mediator, as well as suitable additives selected from the group consisting of stabilizers, buffers, surfactants, bleaching agents, mediators, anti-corrosion agents, builders, anti-redeposition agents, optical brighteners, dyes, pigments, perfumes, caustics, abrasives and preservatives.

According to a sixth aspect is provided a method for degrading or modifying mannan containing material comprising treating the mannan containing material with an effective amount of the recombinant polypeptide of the first aspect or the enzyme composition of the fourth aspect.

According to a seventh aspect is provided an animal feed comprising the recombinant polypeptide of the first aspect or the enzyme composition of the fourth aspect, at least one protein source of plant origin or a mannan containing product or by-product, and
a. Optionally at least one enzyme selected from protease, amylase, phytase, xylanase, endoglucanase, beta-glucanase, or a combination thereof; and
b. Optionally at least one filler selected from maltodextrin, flour, salt, sodium chloride, sulfate, sodium sulfate, or a combination thereof.

According to an eighth aspect is provided a feed supplement comprising the recombinant polypeptide of the first aspect or the enzyme composition of the fourth aspect; and
a. Optionally at least one enzyme selected from protease, amylase, phytase, xylanase, endoglucanase, beta-glucanase, or a combination thereof; and
b. Optionally at least one filler selected from maltodextrin, flour, salt, sodium chloride, sulfate, sodium sulfate or a combination thereof.

The feed and the feed supplement improve nutritional value of feed compared to a feed without mannanase. The present enzyme composition degrades mannan present in the feed and thereby makes it more easily digestible for the animal. In particular for soybean meal containing feeds mannan-oligosaccharides that result from enzymatic digestion have beneficial effects on the intestinal microbes, and consequently on the performance of the animals. The effect of the mannanases can be enhanced by including xylanase to digest arabinoxylans present in corn soybean based diets. Mannanase can also be used to modify rheological properties of wet feeds.

In an embodiment the feed may comprise animal protein, such as meat meal or bone meal.

According to a ninth aspect is provided a use of, and a method of using, the animal feed of the seventh aspect or the feed supplement of the eighth aspect in:
a. feeding animals, preferably monogastric animals; and/or
b. improving weight gain of animals.

According to a tenth aspect is provided a use of the recombinant polypeptide of the first aspect or the enzyme composition of the fourth aspect in a detergent.

In one embodiment of the present invention the detergent composition further comprises one or more additional enzymes selected from the group consisting of protease, lipase, cutinase, amylase, carbohydrase, cellulase, pectinase, pectatelyase, pectinolytic enzyme, esterase, mannanase, arabinase, galactanase, xylanase, oxidase, xanthanase, laccase, DNAse and/or peroxidase, preferably selected from the group consisting of proteases, amylases, cellulases and lipases.

In a further embodiment of the present invention the detergent composition, which is used in the method, is in a form of a bar, a homogenous tablet, a tablet having two or more layers, a pouch having one or more compartments, a regular or compact powder, a granule, a paste, a gel, or a regular, compact or concentrated liquid. In one embodiment the detergent composition can be a laundry detergent composition, preferably a liquid or solid laundry detergent composition.

The present invention furthermore relates to the use of the recombinant polypeptide, the enzyme composition and detergent composition as herein disclosed for degrading mannan.

In a further aspect the present invention relates to the use of the recombinant polypeptide, the enzyme composition and/or detergent composition as herein disclosed in a laundry process.

The present invention furthermore relates to a method for removing a stain from a surface, comprising contacting the surface with the recombinant polypeptide, the enzyme composition and/or detergent composition as herein disclosed.

The present invention also relates to a method for degrading mannan comprising applying the recombinant polypeptide, the enzyme composition or detergent composition as herein disclosed to mannan, preferably wherein the mannan is on a surface of a textile or at least partially embedded in a textile.

According to an eleventh aspect is provided a use of the recombinant polypeptide of the first aspect or the enzyme composition of the fourth aspect in oil drilling.

The present enzyme composition is advantageous in modifying rheological properties of oil drilling fluids and to improve oil recovery.

According to a twelfth aspect is provided a use of the recombinant polypeptide of the first aspect or the enzyme composition of the fourth aspect in processing coffee extract, fruit juice, pineapple juice, or soya milk.

Using the present enzyme composition or the enzyme obtainable from the host cell is advantageous in processing coffee extract because it reduces viscosity of the coffee extract.

Using the present enzyme composition or the enzyme obtainable from the host cell is advantageous in processing and manufacturing fruit juice because it lowers viscosity and improves filtration rate, stability and helps to extract fruit components.

Using the present enzyme composition or the enzyme obtainable from the host cell is advantageous in processing and manufacturing soya milk because it improves yield, colour, protein content and taste of soya milk.

In one embodiment of the invention the recombinant polypeptide has an amino acid sequence having at least 92%, at least 93%, at least 94%, at least 95%, at least 96%, at least 97%, at least 98%, or at least 99% sequence. identity to Man13, SEQ ID NO: 30.

In one embodiment of the invention the recombinant polypeptide has an amino acid sequence of Man13 (SEQ ID NO: 30).

In an embodiment the recombinant polypeptide does not have 100% sequence identity with the amino acid sequence of Man13 (SEQ ID NO: 30).

In another embodiment of the invention the recombinant polypeptide has an amino acid sequence having sequence identity to the sequence Man13 (SEQ ID NO: 30).

As evidenced by the Examples, the fungal mannanases comprised in the enzyme composition according to the invention has a structure and properties that allow production in recombinant host cells and which is useful in enzyme compositions for industrial applications. The inventors tested several mannanases from various sources and found that not all of them produce sufficient yields, or have a stability, which allows them to be taken into industrial production and application. Thus, the above groups of the recombinant polypeptides and enzyme compositions define a limited set of mannanases and mannanase containing products that can be used in applications wherein mannan degradation is desired. The fungal mannanases of the present invention are particularly suitable for production in fungal host, such as in filamentous fungi. They can also be produced in bacteria such as Bacillus.

A common structural element shared by the mannanases is mannan endo-1,4-betamannosidase domain. Another common structural element with the GH5 family mannanases is a sequence identity of at least 35% with Man29 (SEQ ID NO: 39). These structural elements are characteristic for the mannanases of the invention.

The present fungal mannanases are particularly suitable for feed applications and for processing biomass, because they perform well even in acidic environment.

A common structural characteristic for fungal mannanases Man1 (SEQ ID NO: 3), Man3 (SEQ ID NO: 9), Man5 (SEQ ID NO: 12) particularly suitable for detergents is at least 65% identity with Man1 (SEQ ID NO: 3), the presence of GH5 domain and the absence of a CBM domain. This group of enzymes is particularly good at degrading mannan and removing stains in bleach containing detergents.

Another common property shared by the fungal mannanases of the invention is their good performance even in acidic environment.

In an embodiment the mannanase has a molecular weight between about 30 and about 90 kDa, preferably between about 40 and about 70kDa.

In another aspect the disclosed sequence information herein relating to a polynucleotide sequence encoding a mannanase of the invention can be used as a tool to identify other homologous mannanases. For instance, polymerase chain reaction (PCR) can be used to amplify sequences encoding other homologous mannanases from a variety of biological sources. In addition, genome mining approaches can be used to identify sequences encoding other homologous mannanases from genome databases.

### BRIEF DESCRIPTION OF THE FIGURES

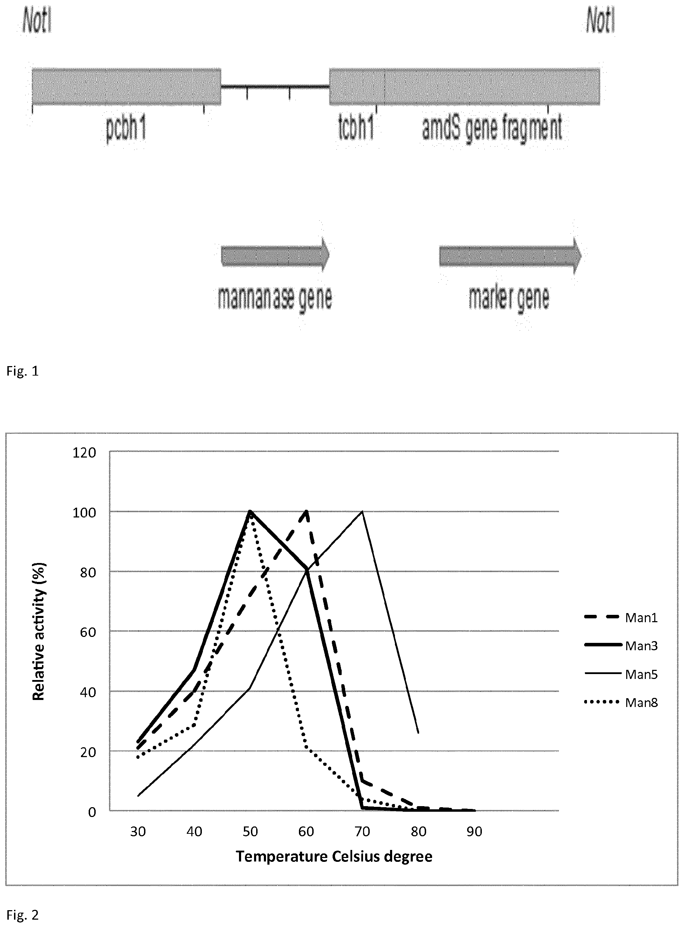

Fig 1 schematically shows the expression cassettes used in the transformation of *Trichoderma reesei* protoplasts for overproducing the recombinant mannanase proteins. The mannanase genes were under the control of *T. reesei cel7A*/*cbh1* promoter (pcbh1) and the termination of the transcription was ensured by using T. *reesei cel7A*/*cbh1* terminator sequence (tcbh1). The *amdS* gene was included as a transformation marker.
Figure 2 describes the temperature profile of recombinant Man1 (SEQ ID NO: 3), Man3 (SEQ ID NO: 9), Man5 (SEQ ID NO: 12) and Man8 (SEQ ID NO: 15) mannanase assayed in 40 mM Britton-Robinson buffer pH 7 using 10 min reaction time, Azurine-crosslinked carob galactomannan was used as a substrate. All measurements were made at least duplicates. The data points are averages of separate measurements.

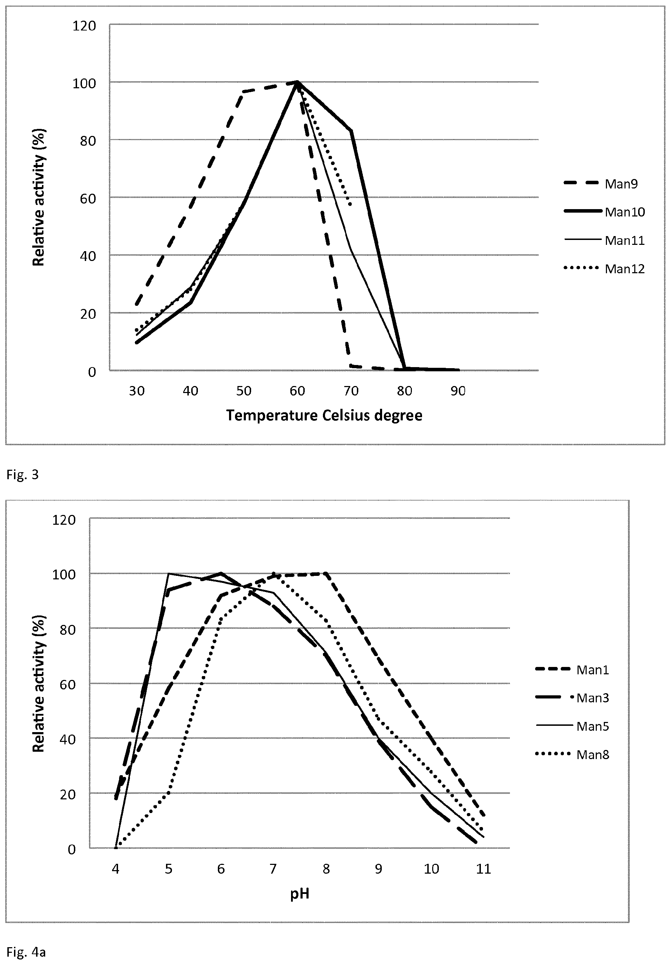

Fig 3 describes the temperature profile of recombinant Man9 (SEQ ID NO: 18), Man10 (SEQ ID NO: 21), Man11 (SEQ ID NO: 24) and Man13 (SEQ ID NO: 30) mannanase assayed in 40 mM Britton-Robinson buffer pH 7 using 10 min reaction time, Azurine-crosslinked carob galactomannan was used as a substrate. All measurements were made at least duplicates. The data points are averages of separate measurements.

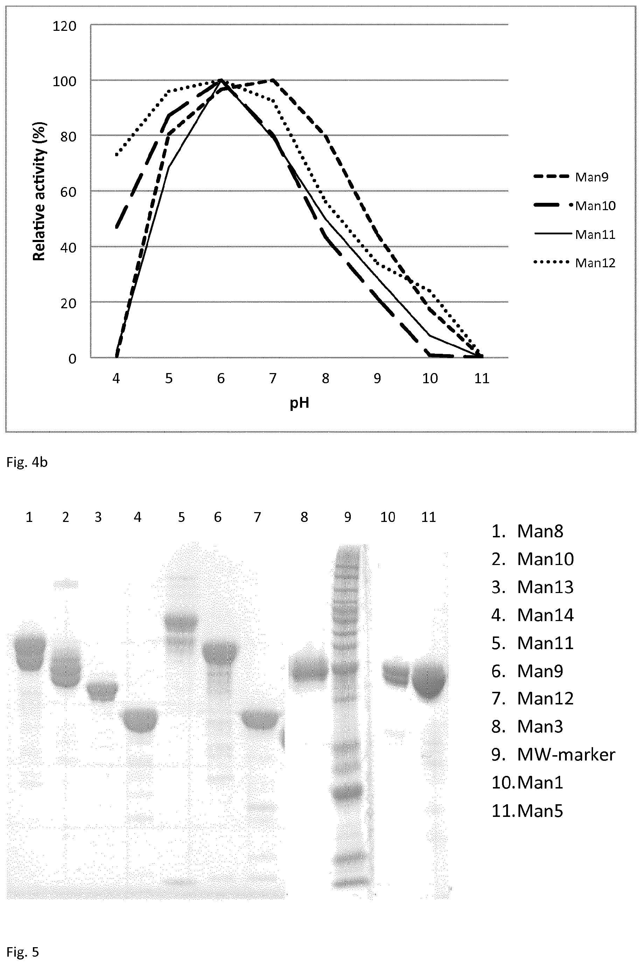

Figure 4a and 4b describes the effect of pH on the activity of recombinant Man1 (SEQ ID NO: 3), Man3 (SEQ ID NO: 9), Man5 (SEQ ID NO: 12) and Man8 (SEQ ID NO: 15) mannanases and Man9 (SEQ ID NO: 18), Man10 (SEQ ID NO: 21), Man11 (SEQ ID NO: 24) and Man12 (SEQ ID NO: 27) mannanases, respectively, in 40 mM Britton-Robinson buffer at pH 4 to pH 11. Reaction temperature was 50°C and the reaction time was 10 min. Azurine-crosslinked carob galactomannan was used as a substrate. All measurements were made at least duplicates. The data points are averages of separate measurements.
Fig 5 shows SDS PAGE analysis of fungal mannanases.

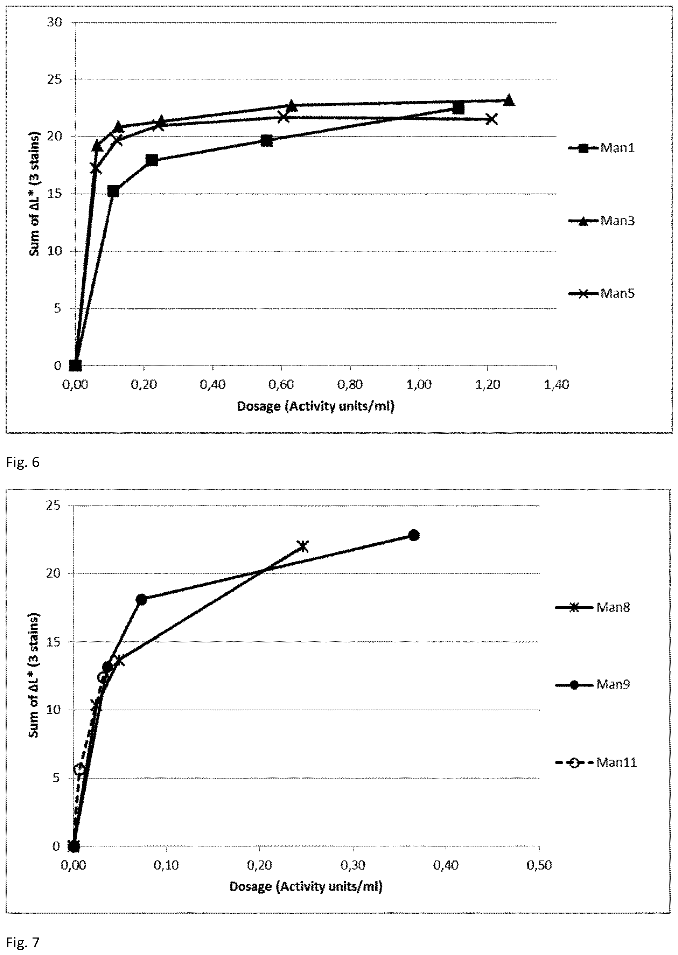

Fig 6 describes the stain removal performance of Man1 (SEQ ID NO: 3), Man3 (SEQ ID NO: 9) and Man5 (SEQ ID NO: 12) as an increase of lightness (sum of ΔL*of 3 stains) in the presence of 4.4 g/l of Commercial heavy duty liquid detergent A at 40°C, 16 °dH, 60 min, pH approximately. 8.2 and enzymes dosed as activity units
Fig 7 describes the stain removal performance of Man8 (SEQ ID NO: 15), Man9 (SEQ ID NO: 18) and Man11 (SEQ ID NO: 24) as an increase of lightness (sum of ΔL*of 3 stains) in the presence of 4.4 g/l of Commercial heavy duty liquid detergent A at 40°C, 16 °dH, 60 min, pH approximately. 8.2 and enzymes dosed as activity units

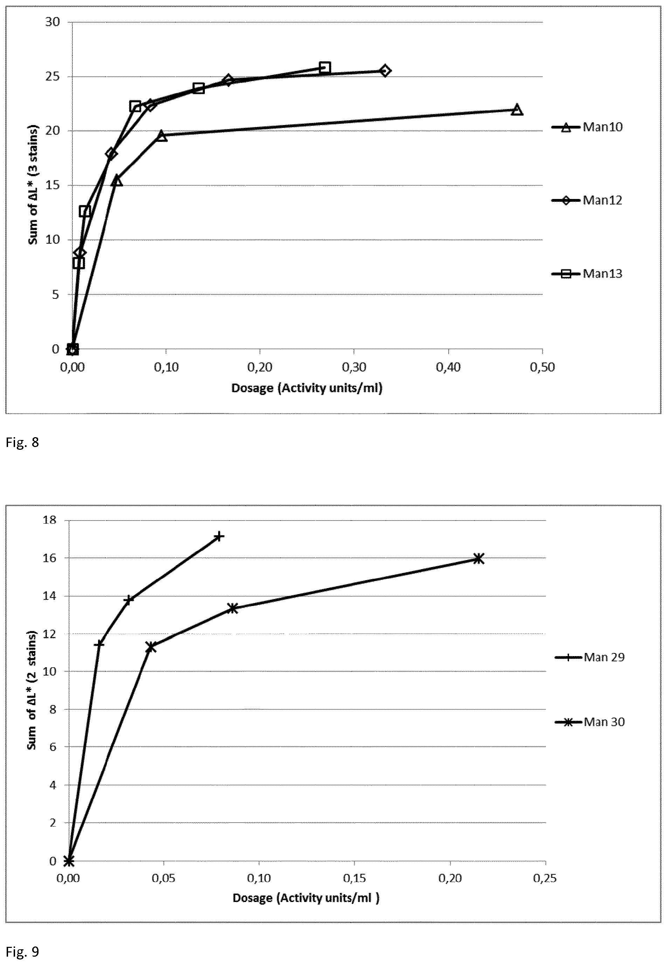

Fig 8 describes the stain removal performance of Man10 (SEQ ID NO: 21), Man12 (SEQ ID NO: 27) and Man13 (SEQ ID NO: 30) as an increase of lightness (sum of ΔL*of 3 stains) in the presence of 4.4 g/l of Commercial heavy duty liquid detergent A at 40°C, 16 °dH, 60 min, pH approximately. 8.2 and enzymes dosed as enzymes dosed as activity units.
Fig 9 describes the stain removal performance of Man29 (SEQ ID NO: 39) and Man30 (SEQ ID NO: 42) as an increase of lightness (sum of ΔL*of 2 stains) in the presence of 5 g/l of Commercial heavy duty liquid detergent at 40°C, 16 °dH, 60 min, pH approximately. 8.2 -8.3 and enzymes dosed as activity units.

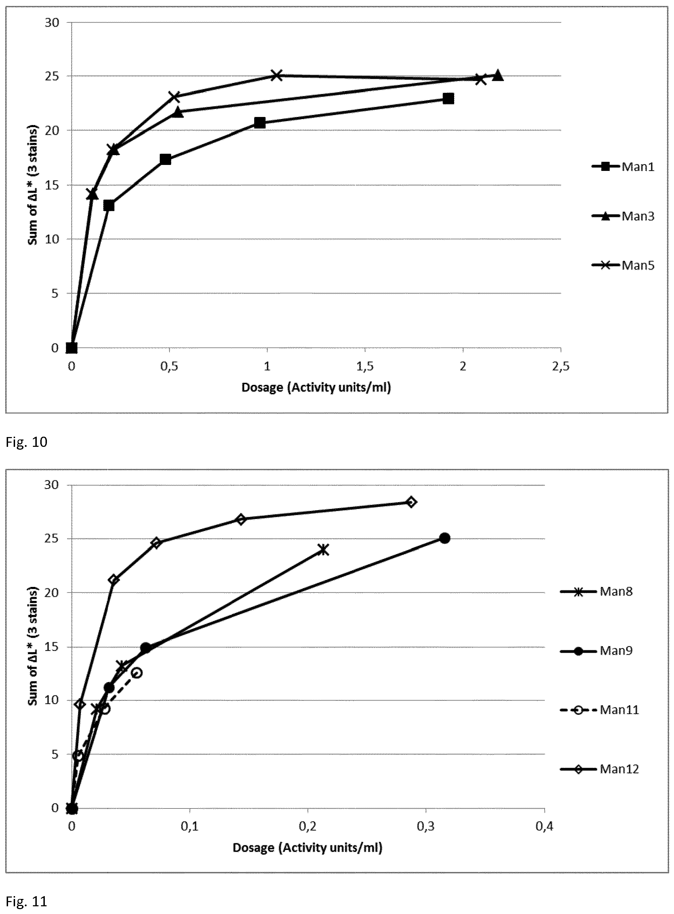

Fig 10 describes the stain removal performance of Man1 (SEQ ID NO: 3), Man3 (SEQ ID NO: 9) and Man5 (SEQ ID NO: 12) as an increase of lightness (sum of ΔL*of 3 stains) in the presence of 3.8 g/l of Commercial color detergent powder at 40°C, 16 °dH, 60 min, pH approximately. 10 and enzymes dosed as activity units.
Fig 11 describes the stain removal performance of Man8 (SEQ ID NO: 15), Man9 (SEQ ID NO: 18), Man11 (SEQ ID NO: 24) and Man12 (SEQ ID NO: 27) as an increase of lightness (sum of ΔL*of 3 stains) in the presence of 3.8 g/l of Commercial color detergent powder at 40°C, 16 °dH, 60 min, pH approximately. 10 and enzymes dosed as activity units.

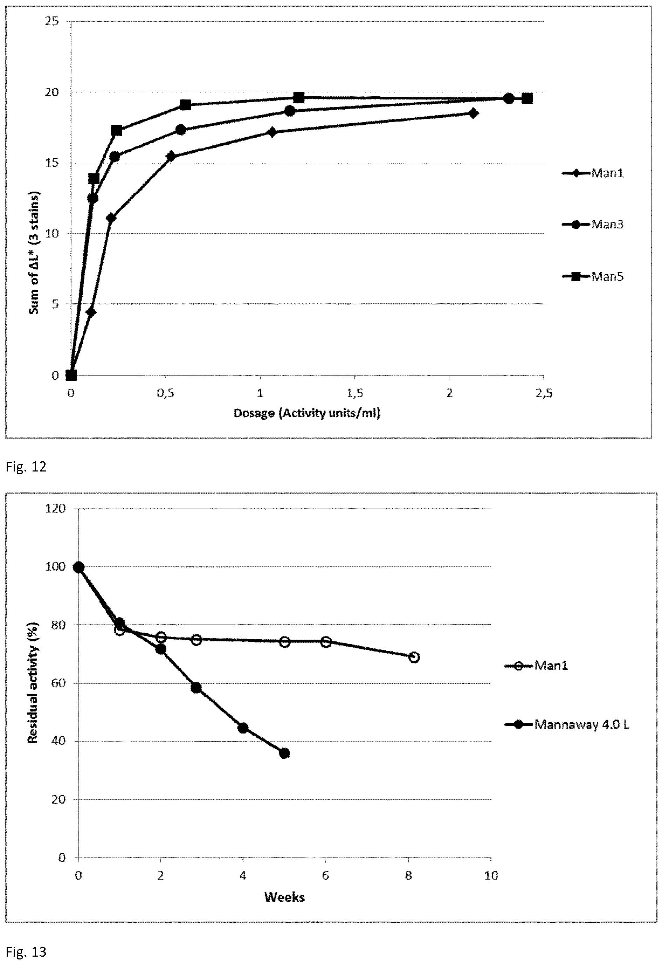

Fig 12describes the stain removal performance of Man1 (SEQ ID NO: 3), Man3 (SEQ ID NO: 9) and Man5 (SEQ ID NO: 12) as an increase of lightness (sum of ΔL* of 3 stains) in the presence of 4,2 g/l of Commercial bleach detergent powder at 40°C, 16 °dH, 60 min, pH approximately. 9.5 and enzymes dosed as activity units.
Fig 13 describes the stability of Man1 (SEQ ID NO: 3) in liquid detergent (OMO Color) at 37°C. Commercial preparation Mannaway^{®} 4,0 L was used for comparison.

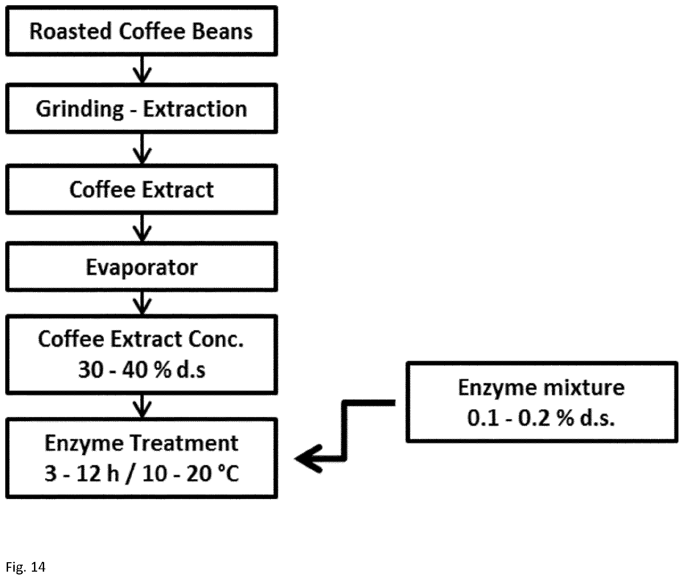

Fig 14 shows a flow chart of instant coffee production involving use of the mannanase of the invention.

### SEQUENCE LISTINGS

SEQ ID NO: 1 nucleotide sequence of *man*1 gene
SEQ ID NO: 2 cDNA sequence of *man1*
SEQ ID NO: 3 deduced amino acid sequence of Man1
SEQ ID NO: 4 nucleotide sequence of *man2* gene
SEQ ID NO: 5 cDNA sequence of *man2*
SEQ ID NO: 6 deduced amino acid sequence of Man2
SEQ ID NO: 7 nucleotide sequence of *man3* gene
SEQ ID NO: 8 cDNA sequence of *man3*
SEQ ID NO: 9 deduced amino acid sequence of Man3
SEQ ID NO: 10 nucleotide sequence of *man5* gene
SEQ ID NO: 11 cDNA sequence of *man5*
SEQ ID NO: 12 deduced amino acid sequence of Man5
SEQ ID NO: 13 nucleotide sequence of *man*8 gene
SEQ ID NO: 14 codon optimized cDNA sequence of *man*8
SEQ ID NO: 15 deduced amino acid sequence of Man8
SEQ ID NO: 16 nucleotide sequence of *man*9 gene
SEQ ID NO: 17 codon optimized cDNA sequence of *man*9
SEQ ID NO: 18 deduced amino acid sequence of Man9
SEQ ID NO: 19 nucleotide sequence of *man*10 gene
SEQ ID NO: 20 codon optimized cDNA sequence of *man*10
SEQ ID NO: 21 deduced amino acid sequence of Man10
SEQ ID NO: 22 nucleotide sequence of *man*11 gene
SEQ ID NO: 23 codon optimized cDNA sequence of *man*11
SEQ ID NO: 24 deduced amino acid sequence of Man11
SEQ ID NO: 25 nucleotide sequence of *man*12 gene
SEQ ID NO: 26 cDNA sequence of *man12*
SEQ ID NO: 27 deduced amino acid sequence of Man12
SEQ ID NO: 28 nucleotide sequence of *man*13 gene
SEQ ID NO: 29 codon optimized cDNA sequence of *man*13
SEQ ID NO: 30 deduced amino acid sequence of Man13
SEQ ID NO: 31 nucleotide sequence of *man*17 gene
SEQ ID NO: 32 cDNA sequence of *man17*
SEQ ID NO: 33 deduced amino acid sequence of Man17
SEQ ID NO: 34 nucleotide sequence of *man*27 gene
SEQ ID NO: 35 cDNA sequence of *man*27
SEQ ID NO: 36 deduced amino acid sequence of Man27
SEQ ID NO: 37 nucleotide sequence of *man*29 gene
SEQ ID NO: 38 codon optimized cDNA sequence of *man*29
SEQ ID NO: 39 deduced amino acid sequence of Man29
SEQ ID NO: 40 nucleotide sequence of *man30* gene
SEQ ID NO: 41 codon optimized cDNA sequence of *man30*
SEQ ID NO: 42 deduced amino acid sequence of Man30
SEQ ID NO: 43 oligonucleotide Mc_Man for
SEQ ID NO: 44 oligonucleotide Mc_Man rev
SEQ ID NO: 45 oligonucleotide MANP5
SEQ ID NO: 46 oligonucleotide MANP6
SEQ ID NO: 47 oligonucleotide Vd_Man3_for
SEQ ID NO: 48 oligonucleotide Vd_Man3_rev
SEQ ID NO: 49 oligonucleotide Vd_Man2_for
SEQ ID NO: 50 oligonucleotide Vd_Man2_rev
SEQ ID NO: 51 oligonucleotide Ma_man2_for
SEQ ID NO: 52 oligonucleotide Ma_Man2_rev
SEQ ID NO: 53 oligonucleotide Ma_Man1for2
SEQ ID NO: 54 oligonucleotide Ma_Man1rev
SEQ ID NO: 55 oligonucleotide MANP3
SEQ ID NO: 56 oligonucleotide MANP4

### DEPOSITS

The following strain depositions according to the Budapest Treaty on the International Recognition of Deposit of Microorganisms for the Purposes of Patent Procedure were made:
*Acremonium thermophilum* ALKO4245 was deposited at the Westerdijk Fungalbio Diversity Institute (formerly Centraalbureau Voor Schimmelcultures) at Uppsalalaan 8, 3584 CT, Utrecht, the Netherlands on 27 February 2017 and assigned accession number CBS 142385.

*Verticillium dahliae* RF8261 was deposited at the Westerdijk Fungalbio Diversity Institute (formerly Centraalbureau Voor Schimmelcultures) at Uppsalalaan 8, 3584 CT, Utrecht, the Netherlands on 27 February 2017 and assigned accession number CBS 142387.

*Malbranchea cinnamomea* ALKO4122 was deposited at the Centraalbureau Voor Schimmelcultures at Uppsalalaan 8, 3584 CT, Utrecht, the Netherlands on 4 January 2011 and assigned accession number CBS 128533.

*Melanocarpus albomyces* ALKO4237 was deposited at the Centraalbureau Voor Schimmelcultures at Uppsalalaan 8, 3584 CT, Utrecht, the Netherlands on 26 March 2012 and assigned accession number CBS 132099.

The *E.coli* strain RF12388 including the plasmid pALK4443 was deposited at the Deutsche Sammlung von Mikroorganismen und Zellkulturen GmbH (DSMZ), Inhoffenstrasse 7 b, D-38124 Braunschweig, Germany on 2 March, 2017 and assigned accession number DSM 32434.

The *E.coli* strain RF12389 including the plasmid pALK4444 was deposited at the Deutsche Sammlung von Mikroorganismen und Zellkulturen GmbH (DSMZ), Inhoffenstrasse 7 b, D-38124 Braunschweig, Germany on 2 March, 2017 and assigned accession number DSM 32435.

The *E.coli* strain RF12390 including the plasmid pALK4445 was deposited at the Deutsche Sammlung von Mikroorganismen und Zellkulturen GmbH (DSMZ), Inhoffenstrasse 7 b, D-38124 Braunschweig, Germany on 2 March, 2017 and assigned accession number DSM 32436.

The *E.coli* strain RF12391 including the plasmid pALK4446 was deposited at the Deutsche Sammlung von Mikroorganismen und Zellkulturen GmbH (DSMZ), Inhoffenstrasse 7 b, D-38124 Braunschweig, Germany on 2 March, 2017 and assigned accession number DSM 32437.

The *E.coli* strain RF12392 including the plasmid pALK4447 was deposited at the Deutsche Sammlung von Mikroorganismen und Zellkulturen GmbH (DSMZ), Inhoffenstrasse 7 b, D-38124 Braunschweig, Germany on 2 March, 2017 and assigned accession number DSM 32438.

The *E.coli* strain RF12393 including the plasmid pALK4448 was deposited at the Deutsche Sammlung von Mikroorganismen und Zellkulturen GmbH (DSMZ), Inhoffenstrasse 7 b, D-38124 Braunschweig, Germany on 2 March, 2017 and assigned accession number DSM 32439.

The *E.coli* strain RF12394 including the plasmid pALK4449 was deposited at the Deutsche Sammlung von Mikroorganismen und Zellkulturen GmbH (DSMZ), Inhoffenstrasse 7 b, D-38124 Braunschweig, Germany on 2 March, 2017 and assigned accession number DSM 32440.

### DETAILED DESCRIPTION

Mannan refers to polysaccharides consisting of a mannose backbone linked together by β-1,4-linkages with side-chains of galactose attached to the backbone by α-1,6-linkages. Mannans comprise plant-based material such as guar gum and locust bean gum. Glucomannans are polysaccharides having a backbone of more or less regularly alternating β-1,4 linked mannose and glucose, galactomannans and galactoglucomannans are mannans and glucomannans with alpha-1,6 linked galactose side branches.

As used herein, the term "mannanase" or "galactomannanase" denotes a mannanase enzyme defined according to that known in the art as mannan endo-1,4-beta-mannosidase and having the alternative names beta-mannanase and endo-1,4-mannanase and catalysing hydrolysis of 1,4-beta-D-mannosidic linkages in mannans, galactomannans, glucomannans, and galactoglucomannans. Mannanases are classified according to the Enzyme Nomenclature as EC 3.2.1.78.

As used herein, "isolated" means a substance in a form or environment that does not occur in nature. Non-limiting examples of isolated substances include (1) any non-naturally occurring substance, (2) any substance including any enzyme, variant, nucleic acid, protein, peptide or cofactor, that is at least partially removed from one or more or all of the naturally occurring constituents with which it is associated in nature; (3) any substance modified by the hand of man relative to that substance found in nature; or (4) any substance modified by increasing or decreasing the amount of the substance relative to other components with which it is naturally associated (e.g., recombinant production in a host cell; one or multiple copies of a gene encoding the substance; and use of an alternative promoter to the promoter naturally associated with the gene encoding the substance). In an embodiment a polypeptide, enzyme, polynucleotide, host cell or composition of the invention is isolated.

As used herein, the term "comprising" includes the broader meanings of "including", "containing", and "comprehending", as well as the narrower expressions "consisting of" and "consisting only of".

As used herein, "fragment" means a protein or a polynucleotide having one or more amino acids or nucleotides deleted. In the context of DNA, a fragment includes both single stranded and double stranded DNA of any length. A fragment may be an active fragment, which has the biological function, such as enzyme activity or regulatory activity, of the protein or the polynucleotide. A fragment may also be an inactive fragment, i.e. it does not have one or more biological effects of the native protein or polynucleotide.

As used herein, a "peptide" and a "polypeptide" are amino acid sequences including a plurality of consecutive polymerized amino acid residues. For purpose of this invention, peptides are molecules including up to 20 amino acid residues, and polypeptides include more than 20 amino acid residues. The peptide or polypeptide may include modified amino acid residues, naturally occurring amino acid residues not encoded by a codon, and non-naturally occurring amino acid residues. As used herein, a "protein" may refer to a peptide or a polypeptide of any size. A protein may be an enzyme, a protein, an antibody, a membrane protein, a peptide hormone, regulator, or any other protein.

The term "polynucleotide" denotes a single- or double-stranded polymer of deoxyribonucleotide or ribonucleotide bases read from the 5' to the 3' end. Polynucleotides include RNA and DNA, and may be isolated from natural sources, synthesized in vitro, or prepared from a combination of natural and synthetic molecules.

As used herein, "modification", "modified", and similar terms in the context of polynucleotides refer to modification in a coding or a non-coding region of the polynucleotide, such as a regulatory sequence, 5' untranslated region, 3' untranslated region, up-regulating genetic element, down-regulating genetic element, enhancer, suppressor, promoter, exon, or intron region. The modification may in some embodiments be only structural, having no effect on the biological effect, action or function of the polynucleotide. In other embodiments the modification is a structural modification, which provides a change in the biological effect, action or function of the polynucleotide. Such a modification may enhance, suppress or change the biological function of the polynucleotide.

As used herein, "identity" means the percentage of exact matches of amino acid residues between two aligned sequences over the number of positions where there are residues present in both sequences. When one sequence has a residue with no corresponding residue in the other sequence, the alignment program allows a gap in the alignment, and that position is not counted in the denominator of the identity calculation. Identity is a value determined with the Pairwise Sequence Alignment tool EMBOSS Needle at the EMBL-EBI website (www.ebi.ac.uk/Tools/psa/emboss_needle/).

As used herein, "host cell" means any cell type that is susceptible to transformation, transfection, transduction, mating, crossing or the like with a nucleic acid construct or expression vector comprising a polynucleotide. The term "host cell" encompasses any progeny that is not identical due to mutations that occur during replication. Non-limiting examples of a host cell are fungal cells, filamentous fungal cells from Division *Ascomycota,* Subdivision *Pezizomycotina*; preferably from the group consisting of members of the Class *Sordariomycetes,* Subclass *Hypocreomycetidae,* Orders *Hypocreales* and *Microascales* and *Aspergillus, Chrysosporium, Myceliophthora and Humicola;* more preferably from the group consisting of Families *Hypocreacea, Nectriaceae, Clavicipitaceae, Microascaceae,* and Genera *Trichoderma* (anamorph of *Hypocrea), Fusarium, Gibberella, Nectria, Stachybotrys, Claviceps, Metarhizium, Villosiclava, Ophiocordyceps, Cephalosporium,* and *Scedosporium*; more preferably from the group consisting of *Trichoderma reesei (Hypocrea jecorina), T. citrinoviridae, T. longibrachiatum, T. virens, T. harzianum, T. asperellum, T. atroviridae, T. parareesei, , Fusarium oxysporum, F. gramineanum, F. pseudograminearum, F. venenatum, Gibberella fujikuroi, G. moniliformis, G. zeaea, Nectria (Haematonectria) haematococca, Stachybotrys chartarum, S. chlorohalonata, Claviceps purpurea, Metarhizium acridum, M. anisopliae, Villosiclava virens, Ophiocordyceps sinensis, Acremonium (Cephalosporium) chrysogenum,* and *Scedosporium apiospermum, and Aspergillus niger, Aspergillus awamori, Aspergillus oryzae, Chrysosporium lucknowense, Myceliophthora thermophila, Humicola insolens,* and *Humicola grisea,* most preferably *Trichoderma reesei.* Non-limiting examples of a host cell are bacterial cells, preferably gram positive Bacilli (e.g. *Bacillus subtilis, B. licheniformis, B. megaterium, B. amyloliquefaciens, B. pumilus*), gram negative bacteria (e.g. *Escherichia coli*), actinomycetales (e.g. *Streptomyces* sp.) and yeasts (e.g. *Saccharomyces cerevisiae, Pichia pastoris, Yarrowia lipolytica).*

In an embodiment the host cell is a fungal cell, preferably a filamentous fungal cell, such as *Trichoderma* or *Trichoderma reesei.* In an embodiment the host cell is a bacterial cell, preferably a gram positive *Bacillus* cell, such as *B*. *subtilis, B. licheniformis, B. megaterium, B. amyloliquefaciens, B. pumilus.*

A "recombinant cell" or "recombinant host cell" refers to a cell or host cell that has been genetically modified or altered to comprise a nucleic acid sequence which is not native to said cell or host cell. In an embodiment the genetic modification comprises integrating the polynucleotide in the genome of the host cell. In another embodiment the polynucleotide is exogenous in the host cell.

As used herein, "expression" includes any step involved in the production of a polypeptide in a host cell including, but not limited to, transcription, translation, post-translational modification, and secretion. Expression may be followed by harvesting, i.e. recovering, the host cells or the expressed product.

The term "expression vector" denotes a DNA molecule, linear or circular, that comprises a segment encoding a polypeptide of interest operably linked to additional segments that provide for its transcription. Such additional segments may include promoter and terminator sequences, and may optionally include one or more origins of replication, one or more selectable markers, an enhancer, a polyadenylation signal, carrier and the like. Expression vectors are generally derived from plasmid or viral DNA, or may contain elements of both. The expression vector may be any expression vector that is conveniently subjected to recombinant DNA procedures, and the choice of vector will often depend on the host cell into which the vector is to be introduced. Thus, the vector may be an autonomously replicating vector, i.e. a vector, which exists as an extrachromosomal entity, the replication of which is independent of chromosomal replication, e.g. a plasmid. Alternatively, the vector may be one which, when introduced into a host cell, is integrated into the host cell genome and replicated together with the chromosome(s) into which it has been integrated.

The term "recombinant produced" or "recombinantly produced" used herein in connection with production of a polypeptide or protein is defined according to the standard definition in the art.

The term "obtained from" and "obtainable" as used herein in connection with a specific microbial source means that the polynucleotide is expressed by the specific source (homologous expression), or by a cell in which a gene from the source has been inserted (heterologous expression).

The term "enzyme composition" means either a conventional enzymatic fermentation product, possibly isolated and purified, from a single species of a microorganism, such preparation usually comprising a number of different enzymatic activities; or a mixture of monocomponent enzymes, preferably enzymes derived from bacterial or fungal species by using conventional recombinant techniques, which enzymes have been fermented and possibly isolated and purified separately and which may originate from different species, preferably fungal or bacterial species or the fermentation product of a microorganism which acts as a host cell for production of a recombinant mannanase, but which microorganism simultaneously produces other enzymes.

The term "operably linked", when referring to DNA segments, denotes that the segments are arranged so that they function in concert for their intended purposes, e.g. transcription initiates in the promoter and proceeds through the coding segment to the terminator

The term "promoter" denotes a portion of a gene containing DNA sequences that provide for the binding of RNA polymerase and initiation of transcription. Promoter sequences are commonly, but not always, found in the 5' non-coding regions of genes.

The term "secretory signal sequence" denotes a DNA sequence that encodes a polypeptide (a "secretory peptide") that, as a component of a larger polypeptide, directs the larger polypeptide through a secretory pathway of a host cell in which it is produced. The secretory signal sequence can be native or it can be replaced with secretory signal sequence or carrier sequence from another source. Depending on the host cell, the larger peptide may be cleaved to remove the secretory peptide during transit through the secretory pathway.

The term "core region" denotes a domain of an enzyme, which may or may not have been modified or altered, but which has retained its original activity; the catalytic domain as known in the art has remained functional. The core region of a mannanase according to the invention corresponds to the amino acids aligned with the amino acids shown in the Tables 5 and 8.

By the term "linker" or "spacer" is meant a polypeptide comprising at least two amino acids which may be present between the domains of a multidomain protein, for example an enzyme comprising an enzyme core and a binding domain such as a carbohydrate binding module (CBM) or any other enzyme hybrid, or between two proteins or polypeptides produced as a fusion polypeptide, for example a fusion protein comprising two core enzymes. For example, the fusion protein of an enzyme core with a CBM is provided by fusing a DNA sequence encoding the enzyme core, a DNA sequence encoding the linker and a DNA sequence encoding the CBM sequentially into one open reading frame and expressing this construct.

Efficient amount means an amount, which is sufficient to degrade mannan in the selected application.

The terms "detergent composition" and "detergent" include, unless otherwise indicated, granular or powder-form all-purpose or heavy-duty washing agents, especially cleaning detergents; liquid, gel or paste-form all-purpose washing agents, especially the so- called heavy-duty liquid (HDL) types; liquid fine-fabric detergents; hand dishwashing agents or light duty dishwashing agents, especially those of the high-foaming type; machine dishwashing agents, including the various tablet, granular, liquid and rinse-aid types for household and institutional use; liquid cleaning and disinfecting agents, car or carpet shampoos, bathroom cleaners; metal cleaners; as well as cleaning auxiliaries such as bleach additives and "stain-stick" or pre-treat types. The terms "detergent", "detergent composition" and "detergent formulation" are used in reference to mixtures, which are intended for use in a wash medium for the cleaning of soiled objects. In some embodiments, the term is used in reference to laundering fabrics and/or garments (e.g., "laundry detergents"). In alternative embodiments, the term refers to other detergents, such as those used to clean dishes, cutlery, etc. (e.g., "dishwashing detergents"). It is not intended that the present invention be limited to any particular detergent formulation or composition. It is intended that in addition to the mannanases according to the invention, the term encompasses detergents that may contain e.g., surfactants, builders, chelators or chelating agents, bleach system or bleach components, polymers, fabric conditioners, foam boosters, suds suppressors, dyes, perfume, tannish inhibitors, optical brighteners, bactericides, fungicides, soil suspending agents, anticorrosion agents, hydrotropes, fabric hueing agents, dispersants, dye transfer inhibiting agents, fluorescent whitening agents, soil release polymers, anti-redepositions agents, anti-shrink agents, anti-wrinkling agents, bactericides, binders, carriers, dyes, enzyme stabilizers, fabric softeners, fillers, foam regulators, perfumes, pigments, sod suppressors, solvents, and structurants for liquid detergents, structure elasticizing agents, enzyme inhibitors or stabilizers, enzyme activators, transferase(s), hydrolytic enzymes, oxido reductases, bluing agents and fluorescent dyes, antioxidants, and solubilizers.

The term "textile" means any textile material including yarns, yarn intermediates, fibers, non-woven materials, natural materials, synthetic materials, and any other textile material, fabrics made of these materials and products made from fabrics (e.g., garments and other articles). The textile or fabric may be in the form of knits, wovens, denims, non-wovens, felts, yarns, and towelling. The textile may be cellulose based, such as natural cellulosics including cotton, flax/linen, jute, ramie, sisal or coir or manmade cellulosics (e.g. originating from wood pulp) including viscose/rayon, ramie, cellulose acetate fibers (tricell), lyocell or blends thereof. The textile or fabric may also be non-cellulose based such as natural polyamides including wool, camel, cashmere, mohair, rabit and silk or synthetic polymer such as nylon, aramid, polyester, acrylic, polypropylen and spandex/elastane, or blends thereof as well as blend of cellulose based and non-cellulose based fibers. Examples of blends are blends of cotton and/or rayon/viscose with one or more companion material such as wool, synthetic fibers (e.g. polyamide fibers, acrylic fibers, polyester fibers, polyvinyl alcohol fibers, polyvinyl chloride fibers, polyurethane fibers, polyurea fibers, aramid fibers), and cellulose-containing fibers (e.g. rayon/viscose, ramie, flax/linen, jute, cellulose acetate fibers, lyocell). Fabric may be conventional washable laundry, for example stained household laundry. When the term fabric or garment is used it is intended to include the broader term textiles as well.

The term "stability" includes storage stability and stability during use, e.g. during a wash process (in wash stability) and reflects the stability of the mannanase according to the invention as a function of time, e.g. how much activity is retained when the mannanase is kept in solution, in particular in a detergent solution. The stability is influenced by many factors, e.g. pH, temperature, detergent composition e.g. proteases, stabilizers, builders, surfactants etc. The mannanase stability may be measured using the 'activity assay' as described in examples.

"Mannanase activity" as used herein refers to the mannan degrading activity of a polypeptide. Degrading or modifying as used herein means that mannose units are hydrolyzed from the mannan polysaccharide by the mannanase. The mannan degrading activity of the polypeptides according to present invention can be tested according to standard test procedures known in the art. Example 6 provides an example of a standard method for determining mannanase activity.

In a further embodiment of the invention the at least one enzyme has mannanase activity. The mannanases comprised in the enzyme composition of the invention are suitable for degrading and modifying mannan containing material in various chemical environments, preferably in detergent compositions.

In one embodiment of the present invention the enzyme composition further comprises one or more additional enzymes selected from the group consisting of protease, lipase, cutinase, amylase, carbohydrase, cellulase, pectinase, pectatelyase, pectinolytic enzyme, esterase, phytase, mannanase, arabinase, galactanase, xylanase, oxidase, xanthanase, DNAse, laccase, and/or peroxidase, preferably selected from the group consisting of proteases, amylases, cellulases and lipases.

In general the properties of the selected enzyme(s) should be compatible with the selected detergent, (*i.e.*, pH-optimum, compatibility with other enzymatic and non-enzymatic ingredients, etc.), and the enzyme(s) should be present in effective amounts.

A composition for use in solid laundry detergent, for example, may include 0.000001% - 5%, such as 0.000005%-2%, such as 0.00001%-1%, such as 0.00001%-0.1% of enzyme protein by weight of the composition.

A composition for use in laundry liquid, for example, may include 0.000001%-3%, such as 0.000005%-1%, such as 0.00001%-0.1% of enzyme protein by weight of the composition.

A composition for use in automatic dishwash, for example, may include 0.000001%-5%, such as 0.000005%-2%, such as 0.00001%-1%, such as 0.00001%-0.1% of enzyme protein by weight of the composition.

In a further embodiment of the present invention the detergent composition is in the form of a bar, a homogenous tablet, a tablet having two or more layers, a pouch having one or more compartments, a regular or compact powder, a granule, a paste, a gel, or a regular, compact or concentrated liquid. In one embodiment the detergent composition can be a laundry detergent composition, preferably a liquid or solid laundry detergent composition. There are a number of detergent formulation forms such as layers (same or different phases), pouches, as well as forms for machine dosing unit.

In an embodiment the recombinant polypeptide is a fusion protein which, in addition to having the amino acid sequence having mannanase activity, comprises at least one of:
an amino acid sequence providing a secretory signal sequence;
an amino acid sequence which facilitates purification, such as an affinity tag or His-tag;
an amino acid sequence which enhances production, such as an amino acid sequence which is a carrier or CBM;
an amino acid sequence having an enzyme activity; and
an amino acid sequence providing for the fusion protein with binding affinity, such as a carbohydrate binding moiety.

The CBM, carbohydrate binding moiety, as a carrier is advantageous e.g. in *Trichoderma* production. The CBM may also be a fragment of another protein or enzyme derived from the same or different organism as the mannanase.

The amino acid sequence having mannanase activity may be connected to the other functionality of the fusion protein via a linker sequence.

In an embodiment the host cell is non-pathogenic. This is particularly advantageous for using the host cell in feed, and in detergent applications such as in home laundry detergents.

Fusion proteins can be engineered to modify properties or production of the mannanase. In an embodiment the mannanase part is connected to the other amino acid sequence with a linker.

In an embodiment of the fourth aspect the present enzyme composition further comprises
a. at least one preservative selected from benzoic acid, sodium benzoate, hydroxybenzoate, citric acid, ascorbic acid, or a combination thereof;
b.optionally at least one polyol selected from propylene glycol, glycerol, a sugar, sugar alcohol, lactic acid, boric acid, boric acid derivative, aromatic borate ester, phenyl boronic acid derivative, peptide, or a combination thereof;
c. optionally at least one enzyme selected from proteases, amylases, cellulases, lipases, xylanases, mannanases, cutinases, esterases, phytases, DNAses, pectinases, pectinolytic enzymes, pectate lyases, carbohydrases, arabinases, galactanases, xanthanases, laccases, peroxidases and oxidases with or without a mediator, or a combination thereof; and
d. optionally at least one filler selected from maltodextrin, flour, sodium chloride, sulfate, sodium sulfate, or a combination thereof.

The additional components a-d provide improved or modified properties for the present enzyme composition. The enzyme composition is compatible with the additional components and improves applicability of the enzyme composition in various uses.

Salts, such as sodium chloride and sodium sulfate function as drying aids.

In an embodiment of the fourth aspect the present enzyme composition is in the form of a liquid composition or a solid composition such as solution, dispersion, paste, powder, granulate, coated granulate, tablet, cake, crystal, crystal slurry, gel, or pellet.

In an embodiment of the sixth aspect the mannan containing material is plant based material, textile, waste water, sewage, oil, or a combination thereof.

In another embodiment the mannan containing material is recycled waste paper; mechanical pulp, chemical pulp, semi chemical pulp, Kraft or other paper-making pulps; fibres subjected to a retting process; or guar gum or locust bean gum containing material.

In another embodiment degradation or modifying is carried out in an aqueous environment wherein mannanase shows activity.

In a preferred embodiment the mannan containing material, which is degraded or modified in the method, is on a textile or a fabric optionally with mannan stains. By degrading mannan attached to the textile or fabric, dirt or soil bound to mannan is released and not capable of binding again to the mannan or mannan stains. The textile or fabric can be of any material, for example cotton, flax/linen, jute, ramie, sisal or coir or manmade cellulosics (e.g. originating from wood pulp) including viscose/rayon, modal, cellulose acetate fibers (tricell), lyocell, cupro or blends thereof.

In an embodiment of the first aspect the recombinant polypeptide has an amino acid sequence which has:
at least 92% sequence identity with Man13 (SEQ ID NO: 30).

This embodiment is particularly advantageous in that the mannanases comprised therein can be produced in hosts suitable for industrial production and the resulting mannanases show activity and stability in detergent compositions.

In an embodiment the recombinant polypeptide is obtainable by expressing in a host cell a polynucleotide having a sequence selected from SEQ ID NO: 29. In another embodiment the sequence is at least 92%, 93%, 94%, 95%, 96%, 97%, 98%, or 99% identical with the above sequence ID NO. In another embodiment the sequence is not 100% identical with the above SEQ ID NO.

In an embodiment the detergent, wherein the recombinant polypeptide is used, is a liquid detergent or a dry detergent, preferably in a form of a powder, bar, tablet, pouch, paste, gel, liquid, granule or granulate.

The present invention furthermore relates to different uses of the enzyme composition as herein disclosed, such as for degrading mannan and for use in a laundry process.

The recombinant polypeptide and the enzyme composition can also be used in cleaning agents or boosters that are added on top of the detergent during or before the wash and that are for example in the form of liquid, gel, powder, granules or tablets. Enzyme composition and detergent components may also be soaked in a carrier like textiles.

In a further embodiment of the present invention the detergent is in the form of a bar, a homogenous tablet, a tablet having two or more layers, a pouch having one or more compartments, a regular or compact powder, a granule, a paste, a gel, or a regular, compact or concentrated liquid. In one embodiment the detergent can be a laundry detergent composition, preferably a liquid or solid laundry detergent composition. There are a number of detergent formulation forms such as layers (same or different phases), pouches, as well as forms for machine dosing unit.

Providing mannanases that retain activity in temperatures above ambient temperature and in acidic, neutral and alkaline pH is advantageous for applications wherein mannan degradation is required in such conditions.

In an embodiment the present enzyme composition comprises the recombinant host cell of the second aspect, or the polypeptide obtainable from the recombinant host cell.

In an embodiment of the sixth aspect the animal is a monogastric animal or a ruminant. In another embodiment the animal is a broiler chicken, egg-laying chicken, swine, turkey, or an aquaculture organism such as fish. In another embodiment the animal is a ruminant.

In an embodiment the feed comprises or consists of maize and soybean meal.

In an embodiment the protein source of plant origin comprises or consist of soy, cereal such as barley, wheat, rye, oats, or maize.

In an embodiment the mannan containing product or by-product comprises or consists of palm kernel, guar meal or copra meal.

In an embodiment of the sixth or seventh aspect the animal feed or the feed supplement is formulated in the form of a wet composition or a dry composition.

In an embodiment of the seventh aspect the animal is a monogastric animal. In another embodiment the animal is a broiler chicken, egg-laying chicken, swine, turkey, or an aquaculture organism such as fish.

In an embodiment the feed comprises or consists of maize and soybean meal.

In an embodiment the protein source of plant origin comprises or consist of soy, cereal such as barley, wheat, rye, oats, or maize.

In an embodiment the mannan containing product or by-product comprises or consists of palm kernel or guar meal.

In an embodiment of the sixth or seventh aspect the animal feed or the feed supplement is formulated in the form of a wet composition or a dry composition.

In an embodiment or the tenth aspect the detergent is a liquid detergent or a dry detergent preferably in a form of a powder or granulate.

In an embodiment the mannanase is used in pulp and paper industry, biobleaching, fiber modification, drainage improvement, in the oil industry, e.g. in oil drilling or oil-servicing industry for hydro-fracturing or controlling the viscosity of drilling fluids.

In an embodiment the mannanase is used in textile and detergent industry, biomass processing, preferably in biofuel, starch, pulp and paper, food, baking, feed or beverage industries.

In an embodiment the mannanase hydrolyses endo-beta-1,4-mannosidic linkages randomly.

In an embodiment the mannanase is obtainable or derivable from a fungal source.

In an embodiment is provided a process for machine treatment of fabrics which process comprises treating fabric during a washing cycle of a machine washing process with a washing solution containing the enzyme composition of the first aspect, the recombinant host cell of the second aspect or the recombinant polypeptide of the third aspect.

In an embodiment is provided a use of the enzyme composition of the fourth aspect, the enzyme obtainable from the recombinant host cell of the second aspect, or the polypeptide of the first aspect together with an enzyme selected from cellulase, protease, lipase, amylase, esterase, laccase, peroxidase, pectin degrading enzyme and xyloglucanase in a cleaning composition for fabric cleaning and/or fabric stain removal.

In an embodiment is provided a use of the enzyme composition of the fourth aspect, the enzyme obtainable from the recombinant host cell of the second aspect, or the polypeptide of the first aspect together with an enzyme selected from cellulase, amylase, protease, lipase, esterase, peroxidase, pectin degrading enzyme and xyloglucanase in a cleaning composition for cleaning hard surfaces such as floors, walls, bathroom tile and the like.

In an embodiment is provided a use of the enzyme composition of the fourth aspect, the enzyme obtainable from the recombinant host cell of the second aspect, or the polypeptide of the fist aspect together with an enzyme selected from cellulase, amylase, protease, lipase, esterase, peroxidase, pectin degrading enzyme and xyloglucanase in a cleaning composition for hand and machine dishwashing.

### EXAMPLES

The following examples are provided to illustrate various aspects of the present invention. They are not intended to limit the invention, which is defined by the accompanying claims.

### Example 1: Screening

For identification of new beta-1,4-mannanases public databases (NCBI, EBI) and selected proprietary and public genomes were screened. All proprietary and public genomes used in this work are shown in Table 1. All hits were grouped and finally 14 genes of fungal origin were selected for cloning in *Trichoderma reesei* (Table 2).

**Table 1: List of proprietary and public genomes used for screening of beta-1,4-mannanases. Identification numbers of proprietary strains, genome sources and identification of strains used for public genomes are shown.**

| Species | Strain | Genome | Genome origin |
|---|---|---|---|
| *Melanocarpus albomyces* | ALKO4237 | ROAL | |
| *Acremonium thermophilum* | ALKO4245 | ROAL | |
| *Fusarium equiseti* | RF6318 | ROAL | |
| *Verticillium dahliae* | RF8261 | JGI^{(a} | VdLs.17 |
| *Malbranchea cinnamomea* | ALKO4122 | Genozymes^{(b} | CBS 343.55 |
| *Aspergillus nishimurae* | RH3949 | AB Enzymes | |
| *Acremonium alcalophilum* | | JGI^{(a} | |
| *Gymnascella citrina* | | JGI^{(a} | NRRL 5970 |
| *Plectosphaerella cucumerina* | | JGI^{(a} | DS2psM2a2 |

| | | | |
|---|---|---|---|
| ^{(a} Joint Genome Institute (United States Department of Energy) at http://genome.jgi.doe.gov/ ^{(b} Genozymes project (Genozymes for Bioproducts and Bioprocesses Development) at http://www.fungalgenomics.ca | | | |

**Table 2: List of genes selected for cloning in Trichoderma reesei. Predicted PFAM domains and amino acid lengths of the proteins are shown.**

| **Sequence ID** | **Species** | **GH family** | **Length** |
|---|---|---|---|
| Man1, SEQ ID NO: 3 | *Malbranchea cinnamomea* | 5 | 410 aa |
| Man2, SEQ ID NO: 6 | *Verticillium dahliae* | 5 | 397 aa |
| Man3, SEQ ID NO: 9 | *Verticillium dahliae* | 5 | 401 aa |
| Man5, SEQ ID NO: 12 | *Melanocarpus albomyces* | 5 | 416 aa |
| Man8, SEQ ID NO:15 | *Acremonium alcalophilum* | 5 | 469 aa |
| Man9, SEQ ID NO: 18 | *Fusarium equisetii* | 5 | 446 aa |
| Man10, SEQ ID NO:21 | *Acremonium thermophilum* | 5 | 416 aa |
| Man11, SEQ ID NO:24 | *Verticillium dahliae* | 5 | 492 aa |
| Man12, SEQ ID NO:27 | *Malbranchea cinnamomea* | 5 | 355 aa |
| Man13, SEQ ID NO: 30 | *Aspergillus nishimurae* | 5 | 376 aa |
| Man17, SEQ ID NO: 33 | *Melanocarpus albomyces* | 26 | 476 aa |
| Man27, SEQ ID NO: 36 | *Acremonium thermophilum* | 5 | 379 aa |
| Man29, SEQ ID NO: 39 | *Plectosphaerella cucumerina* | 5 | 401 aa |
| Man30, SEQ ID NO: 42 | *Gymnascella citrina* | 5 | 411 aa |

### Example 2: PCR-cloning of Malbranchea cinnamomea ALKO4122 man1 and man12, Verticillium dahliae RF8261 man2 and man3, Melanocarpus albomyces ALKO4237 man5 and man17, and Acremonium thermophilum ALKO4245 man27 mannanases.

Standard molecular biology methods were used in the isolation and enzyme treatments of DNA (e.g. isolation of plasmid DNA, digestion of DNA to produce DNA fragments), in *E*. *coli* transformations, sequencing etc. The basic methods used were either as described by the enzyme, reagent or kit manufacturer or as described in the standard molecular biology handbook, e.g. Sambrook and Russell (2001).

After analyzing the sequence data from the fungal strains *Malbranchea cinnamomea* ALKO4122, *Verticillium dahliae* RF8261, and *Melanocarpus albomyces* ALKO4237 and *Acremonium thermophilum* ALKO4245 (see Example 1) 7 mannanase genes from the analyzed genomes were selected for PCR-cloning. The genes encoding mannanases *man*2 and *man*3 were PCR-cloned using genomic DNA of RF8261 as a template. Mannanase genes *man*5 and *man*17 were cloned using ALKO4237 genomic DNA as template, *Man*27 was cloned using ALKO4245 genomic DNA and *man*12 using ALKO4122 genomic DNA as templates. The gene encoding mannanase *man*1 was PCR-cloned using plasmid pALK4042 as a template. Plasmid pALK4042 consists of pBluescript II KS+ vector and about 4.5 kb DNA fragment from ALKO4122 including the mannanase gene. The genes were amplified by PCR with primers described in Table 3. The PCR mixture contained 1x HF Buffer for Phusion HF Polymerase (NEB/BioNordika, Finland), 0.2 mM dNTP mix (Thermo Fisher Scientific, Finland), 1 µM each primer, 3% DMSO (Thermo Fisher Scientific), 1 unit of Phusion High-Fidelity Polymerase (NEB/BioNordika, Finland) and 1-2 µg genomic DNA or 50 - 200 ng plasmid DNA as templates. The conditions for the PCR reactions were the following: 30 sec initial denaturation at 98 °C, followed by 28 cycles of 10 sec at 98 °C, 30 sec annealing at one of the following 45/50/55/60 °C, 45 sec extension at 72 °C and the final extension at 72 °C for 7 min.

Primer combination described in Table 3 produced specific DNA product having the expected size. The PCR product was isolated from agarose gel with GenJet Gel Extraction Kit (Thermo Fisher Scientific) according to manufacturer's instructions, digested with Sacll and BamHI restriction enzymes (Thermo Fisher Scientific) and cloned into an expression vector cleaved with Sacll and *Bam*HI. Ligation mixtures were transformed into *Escherichia coli* XL1-Blue (AH Diagnostics) or XL10-Gold (Agilent) cells and plated on LB (Luria-Bertani) plates containing 50-100 µg/ml ampicillin. Several *E. coli* colonies were collected from the plates and DNA was isolated with GenJet Plasmid Miniprep Kit (Thermo Fisher Scientific). Positive clones were screened using restriction digestions and they were shown to contain inserts of expected sizes. The full-length genes encoding the mannanases *man*1*, man*2*, man*3*, man*5*, man*12*, man*17 *and man*27 were sequenced and the plasmids were named pALK4073, pALK4070, pALK4071, pALK4271, pALK4080 and pALK4270, respectively (For details see Example 4). The relevant information on the genes and the deduced amino protein sequences (SEQ ID NOs: 1, 3, 4, 6, 7, 9, 10, 12, 25, 27, 31, 33, 34, 36) are summarized in Table 4 and Table 5, respectively.

**Table 3. The oligonucleotides used as PCR primers to amplify mannanase genes from Malbranchea cinnamomea ALKO4122, Verticillium dahliae RF8261, Melanocarpus albomyces ALKO4237, Acremonium thermophilum ALKO4245 and plasmid pALK4042.**

| **Template, DNA from** | **Oligo-nucleotides** | **Length (bp)** | **Sequence^{(a}** | **SEQ ID NO:** |
|---|---|---|---|---|
| pALK4042 | Mc_Man for | 53 | | 43 |
| pALK4042 | Mc_Man rev | 36 | | 44 |
| ALK04122 *Malbranchea cinnamomea* | MANP5 | 60 | | 45 |
| ALK04122 *Malbranchea cinnamomea* | MANP6 | 52 | | 46 |
| RF8261 *Verticillium dahliae* | Vd_Man3_for | 48 | | 47 |
| RF8261 *Verticillium dahliae* | Vd_Man3_rev | 37 | | 48 |
| RF8261 *Verticillium dahliae* | Vd_Man2_for | 47 | | 49 |
| RF8261 *Verticillium dahliae* | Vd_Man2_rev | 38 | | 50 |
| ALKO4237 *Melanocarpus albomyces* | Ma_man2_for | 53 | | 51 |
| ALKO4237 *Melanocarpus albomyces* | Ma_Man2_rev | 39 | | 52 |
| ALKO4237 *Melanocarpus albomyces* | Ma_Man1for2 | 47 | | 53 |
| ALKO4237 *Melanocarpus albomyces* | Ma_Man1rev | 41 | | 54 |
| ALK04245 *Acremonium thermophilum* | MANP3 | 60 | | 55 |
| ALK04245 *Acremonium thermophilum* | MANP4 | 54 | | 56 |

| | | | | |
|---|---|---|---|---|
| ^{(a}"s" in the parenthesis = sense strand, "as" = antisense strand. | | | | |

**Table 4. The summary on the mannanase encoding genes of Malbranchea cinnamomea ALKO4122, Verticillium dahliae RF8261, Melanocarpus albomyces ALKO4237 and Acremonium thermophilum ALKO4245.**

| **Gene** | **Origin** | **Length with introns (bp)^{(a}** | **Coding region (bp)^{(b}** | **No of putative introns** | **Lengths of putative introns (bp)** | **SEQ ID NO** |
|---|---|---|---|---|---|---|
| *man1* | *M. cinnamomea* | 1686 | 1230 | 4 | 119, 124, 121,89 | 1 |
| *man2* | *V. dahliae* | 1330 | 1191 | 2 | 65, 71 | 4 |
| *man3* | *V. dahliae* | 1388 | 1203 | 2 | 83, 99 | 7 |
| *man5* | *M.albomyces* | 1397 | 1248 | 2 | 81, 65 | 10 |
| *man12* | *M. cinnamomea* | 1275 | 1065 | 3 | 73, 63, 71 | 25 |
| *man17* | *M. albomyces* | 1761 | 1428 | 4 | 63, 69, 103, 95 | 31 |
| *man27* | *A. thermophilum* | 1360 | 1137 | 2 | 107, 113 | 34 |

| | | | | | | |
|---|---|---|---|---|---|---|
| ^{(a} The STOP codon is included ^{(b} The STOP codon is not included | | | | | | |

**Table 5. The summary of the amino acid sequences deduced from the mannanase encoding gene sequences from Malbranchea cinnamomea ALKO4122, Verticillium dahliae RF8261, Melanocarpus albomyces ALKO4237 and Acremonium thermophilum ALKO4245.**

| **Man protein** | **Origin** | **No of aas** | **Length of ss ^{(a}** | **CBM** | **Core (aa-aa)** | **Predicted (Da), ss not included (b** | **Predicted pl, ss not included** | **SEQ ID NO** |
|---|---|---|---|---|---|---|---|---|
| Man1 (SEQ ID NO: 3) | *M. cinnamomea* | 410 | 18 | No | | 44487 | 5.37 | 3 |
| Man2 (SEQ ID NO: 6) | *V. dahliae* | 397 | 22 | No | | 41589 | 4.56 | 6 |
| Man3 (SEQ ID NO: 9) | *V. dahliae* | 401 | 17 | No | | 43488 | 4.71 | 9 |
| Man5 (SEQ ID NO: 12) | *M. albomyces* | 416 | 20 | No | | 44670 | 4.51 | 12 |
| Man12 (SEQ ID NO: 27) | *M. cinnamomea* | 355 | 17 | No | | 37959 | 4.26 | 27 |
| Man17 (SEQ ID NO: 33) | *M. albomyces* | 476 | 22 | Yes | 143-476 | 50420 | 4.42 | 33 |
| Man27 (SEQ ID NO: 36) | *A. thermophilum* | 379 | 18 | No | | 39046 | 4.86 | 36 |

| | | | | | | | | |
|---|---|---|---|---|---|---|---|---|
| ^{(a} The prediction on the signal sequence was made using the program SignalP v3.0, NN/HMM (Nielsen *et al.*, 1997; Nielsen & Krogh, 1998; Bendtsen *et al.*, 2004). ^{(b} The predicted signal sequence was not included. The prediction was made using Clone Manager Professional version 9 for Windows, Sci-Ed Software. | | | | | | | | |

The comparison of the deduced mannanase sequences Man1 (SEQ ID NO: 3) and Man12 (SEQ ID NO: 27) from *Malbranchea cinnamomea* ALKO4122, Man2 (SEQ ID NO: 6) and Man3 (SEQ ID NO: 9) from *Verticillium dahliae* RF8261, Man5 (SEQ ID NO: 12) and Man17 (SEQ ID NO: 33) from *Melanocarpus albomyces* ALKO4237 and Man27 (SEQ ID NO: 36) from *Acremonium thermophilum* ALKO4245 to the databases is shown in Table 6.

**Table 6. The highest identity sequences to the deduced Man1 (SEQ ID NO: 3), Man2 (SEQ ID NO: 6), Man3 (SEQ ID NO: 9) Man5 (SEQ ID NO: 12), Man12 (SEQ ID NO: 27), Man17 (SEQ ID NO: 33) and Man27 (SEQ ID NO: 36) amino acid sequences from *Malbranchea cinnamomea* ALKO4122, *Verticillium dahliae* RF8261, *Melanocarpus albomyces* ALKO4237 and *Acremonium thermophilum* ALKO4245. The full-length amino acid sequences including the signal sequences were aligned. The database searches were performed at** https://www.ebi.ac.uk/Tools/sss/ncbiblast/https://www.ebi.ac.uk/Tools/psa/emboss needle / **using NCBI BLAST+ (EMBL-EBI, NCBI BLAST+** - **Sequence Similarity Search, UniProt Knowledgebase, BLOSUM62, Gap open 11, Gap extend 1), and EMBOSS Needle (EMBL-EBI, EMBOSS-Needle** - **Pairwise Sequence Alignment, BLOSUM62, Gap open 10, Gap extend 0.5) for determining the degree of identity. Searches were also performed with https://usgene.sequencebase.com/ using Matrix PAM30, Gap open 9 and Gap extend 1.**

| **Organism and accession number** | **Identity (%)** |
|---|---|
| Man1 (SEQ ID NO: 3) *Aspergillus terreus,* Q0C8J3 ⁽¹ | 78.9 |
| *Malbranchea cinnamomea,* BBN39104 ⁽² | 99.5 |
| Man2 (SEQ ID NO: 6) *Verticillium dahliae, G2XHW3_VERDV* ⁽¹ | 100 |
| *Myriococcum thermophilum,* BBA64714 ⁽² | 63.4 |
| Man3 (SEQ ID NO: 9) *Verticillium dahliae,* G2XBE5_VERDV (VDAG 07477) ⁽¹ | 100 |
| *Aspergillus terreus,* BBW96507 ⁽² | 69.4 |
| Man5 (SEQ ID NO: 12) *Chaetomium* sp. CQ31, G9BZJ2_9PEZI ⁽¹ | 99.3 |
| *Chaetomium* sp., BAL41820 ⁽² | 99.3 |
| Man12 (SEQ ID NO: 27) *Pseudogymnoascus* sp, A0A1B8DBN0_9PEZI ⁽¹ | 46.1 |
| *Malbranchea cinnamomea,* BBN39199 ⁽² | 100 |
| Man17 (SEQ ID NO: 33) *Madurella mycetomatis*, A0A175W047_9PEZI ⁽¹ | 81.5 |
| *Melanocarpus albomyces*, BDB24260 ⁽² | 99.8 |
| Man27 (SEQ ID NO: 36) *Coniochaeta ligniaria*, A0A1J7JVW4_9PEZI ⁽¹ | 75.5 |
| *Acremonium thermophilum,* BDB18590 ⁽² | 100 |

| | |
|---|---|
| ⁽¹ Search performed using NCBI BLAST+ (EMBL-EBI, NCBI BLAST+ - Sequence Similarity Search, UniProt Knowledgebase, BLOSUM62, Gap open 11, Gap extend 1), and EMBOSS Needle (EMBL-EBI, EMBOSS-Needle - Pairwise Sequence Alignment, BLOSUM62, Gap open 10, Gap extend 0.5) for determining the degree of identity. ⁽² Search performed with SequenceBase database https://usgene.sequencebase.com/ | |

### Example 3: Cloning of synthetic fungal mannanase genes

Standard molecular biology methods were used in the isolation and enzyme treatments of DNA (e.g. isolation of plasmid DNA, digestion of DNA to produce DNA fragments), in *E. coli* transformations, sequencing etc. The basic methods used were either as described by the enzyme, reagent or kit manufacturer or as described in the standard molecular biology handbook, e.g. Sambrook and Russell (2001).

*Man8, man9, man10, man11, man13, man29 and man30* from *Acremonium alcalophilum, Fusarium equiseti* RF6318, *Acremonium thermophilum* ALKO4245, *Verticillium dahliae* RF8261, *Aspergillus nishimurae* RH3949, *Plectosphaerella cucmerina* and *Gymnascella citrina* respectively, were ordered from GenScript as synthetic constructs without introns and with codon optimization for *Trichoderma reesei.*

Plasmid DNAs obtained from GenScript including the genes *man*8, *man*9, *man*10, *man*11, *man*13, *man*29 and *man*30 were re-suspended in sterile water, digested with Sacll and BamHI restriction enzymes (Thermo Fisher Scientific) according to manufacturer's instructions and cloned into an expression vector cleaved with Sacll and *Bam*HI. Ligation mixtures were transformed into *Escherichia coli* XL1-Blue (AH Diagnostics) and plated on LB (Luria-Bertani) plates containing 50-100 µg/ml ampicillin. Several *E. coli* colonies were collected from the plates and DNA was isolated with GenJet Plasmid Miniprep Kit (Thermo Fisher Scientific). Positive clones were screened using restriction digestions and they were shown to contain inserts of expected sizes. The full-length genes encoding the *Acremonium alcalophilum man*8, *Fusarium equiseti man*9, *Acremonium thermophilum man*10 and *Verticillium dahliae man*11 mannanases, and fusion sites to the expression plasmid of *Aspergillus nishimurae man*13, *Plectosphaerella cucumerina man*29 and *Gymnascella citrina man*30 mannanase genes were sequenced and the plasmids were named pALK4363, pALK4091, pALK4084, pALK4090, pALK4413, pALK4411 and pALK4412 respectively (For details see Example 4). The plasmid DNAs including the aforementioned genes delivered by GenScript were also transformed into XL10-Gold *E*. *coli* cells (Agilent) and deposited into DSMZ strain collections. The relevant information on the genes and the deduced amino protein sequences (SEQ ID NOs: 14, 17, 20, 23, 29, 38 and 41) are summarized in Table 7 and Table 8, respectively. The *E. coli* strains RF12388, RF12389, RF12390, RF12391, RF12392 and RF12393 including the plasmids pALK4443, pALK4444, pALK4445, pALK4446, pALK4447 and pALK4448, respectively were deposited to the DSM collection under the accession numbers DSM 32434, DSM 32435, DSM 32436,DSM 32437, DSM 32438 and DSM 32439 respectively.

**Table 7. The summary on the mannanase encoding synthetic genes designed based on sequences obtained from Acremonium alcalophilum, Fusarium equiseti RF6318, Acremonium thermophilum ALKO4245, Verticillium dahliae RF8261, Aspergillus nishimurae RH3949, Plectosphaerella cucumerina and Gymnascella citrina.**

| **Gene** | **Length without introns (bp)^{(a}** | **SEQ ID NO** |
|---|---|---|
| *man*8 | 1410 | 14 |
| *man*9 | 1341 | 17 |
| *man*10 | 1251 | 20 |
| *man11* | 1479 | 23 |
| *man*13 | 1131 | 29 |
| *man29* | 1206 | 38 |
| *man*30 | 1236 | 41 |

| | | |
|---|---|---|
| ^{(a} The STOP codon is included | | |

**Table 8. The summary of the amino acid sequences deduced from the mannanase encoding gene sequences from Acremonium alcalophilum, Fusarium equiseti RF6318, Acremonium thermophilum ALKO4245, Verticillium dahliae RF8261, Aspergillus nishimurae RH3949, Plectosphaerella cucumerina and Gymnascella citrina.**

| **Man protein** | **No of aas** | **Length of ss ^{(a}** | **CBM** | **Core (aa-aa)** | **Predicted (Da), ss not included ^{(b}** | **Predicted pl, ss not included ^{(b}** | **SEQ ID NO** |
|---|---|---|---|---|---|---|---|
| Man8 (SEQ ID NO: 15) | 469 | 21 | Yes | 22-392 | 49497 | 4.36 | 15 |
| Man9 (SEQ ID NO: 18) | 446 | 18 | Yes | 19-387 | 47728 | 5.05 | 18 |
| Man10 (SEQ ID NO: 21) | 416 | 19 | No | | 44403 | 4.97 | 21 |
| Man11 (SEQ ID NO: 24) | 492 | 19 | Yes | 20-401 | 51903 | 5.16 | 24 |
| Man13 (SEQ ID NO: 30) | 376 | 21 | No | | 39351 | 5.09 | 30 |
| Man29 (SEQ ID NO: 39) | 401 | 18 | No | | 43738 | 4.55 | 39 |
| Man30 (SEQ ID NO: 42) | 411 | 18 | No | | 44747 | 5.83 | 42 |

| | | | | | | | |
|---|---|---|---|---|---|---|---|
| ^{(a} The prediction on the signal sequence was made using the program SignalP v3.0, NN/HMM (Nielsen *et al.*, 1997; Nielsen & Krogh, 1998; Bendtsen *et al.*, 2004). ^{(b} The predicted signal sequence was not included. The prediction was made using Clone Manager Professional version 9 for Windows, Sci-Ed Software. | | | | | | | |

The comparison of the deduced mannanase sequences Man8 (SEQ ID NO: 15), Man9 (SEQ ID NO: 18), Man10 (SEQ ID NO: 21), Man11 (SEQ ID NO: 24), Man13 (SEQ ID NO: 30), Man29 (SEQ ID NO: 39) and Man30 (SEQ ID NO: 42) from *Acremonium alcalophilum, Fusarium equiseti* RF6318, *Acremonium thermophilum* ALKO4245, *Verticillium dahliae* RF8261, *Aspergillus nishimurae* RH3949, *Plectosphaerella cucumerina* and *Gymnascella citrina* respectively, to the databases is shown in Table 9.

**Table 9. The highest identity sequences to the deduced Man8 (SEQ ID NO: 15), Man9 (SEQ ID NO: 18), Man10 (SEQ ID NO: 21), Man11 (SEQ ID NO: 24), Man13 (SEQ ID NO: 30), Man29 (SEQ ID NO: 39) and Man30 (SEQ ID NO: 42) amino acid sequences from *Acremonium alcalophilum, Fusarium equiseti RF6318, Acremonium thermophilum ALKO4245, Verticillium dahliae RF8261, Aspergillus nishimurae* RH3949, *Plectosphaerella cucumerina* and *Gymnascella citrina.* The full-length amino acid sequence including the signal sequenceswere aligned. The database searches were performed at** https://www.ebi.ac.uk/Tools/sss/ncbiblast/ and https://www.ebi.ac.uk/Tools/psa/emboss_needle/ **using NCBI BLAST+ (EMBL-EBI, NCBI BLAST+** - **Sequence Similarity Search, UniProt Knowledgebase, BLOSUM62, Gap open 11, Gap extend 1), and EMBOSS Needle (EMBL-EBI, EMBOSS-Needle** - **Pairwise Sequence Alignment, BLOSUM62, Gap open 10, Gap extend 0.5) for determining the degree of identity. Searches were also performed with https://usgene.sequencebase.com/ using Matrix PAM30, Gap open 9 and Gap extend 1.**

| **Organism and accession number** | **Identity (%)** |
|---|---|
| Man8 (SEQ ID NO: 15) *Verticillium longisporum* , A0A0G4LAG4_9PEZI ⁽¹ | 69.0 |
| *Nectria haematococca*, BBX16347 ⁽² | 67.7 |
| Man9 (SEQ ID NO: 18) *Fusarium langsethiae*, A0A0M9ES31_9HYPO ⁽¹ | 92.2 |
| *Fusarium graminearum*, CEF88482.1 ⁽² | 91.1 |
| Man10 (SEQ ID NO: 21) *Coniochaeta ligniaria*, A0A1J7I5A7_9PEZI ⁽¹ | 69.6 |
| *Thielavia australiensis*, BBN39719 ⁽² | 67.0 |
| Man11 (SEQ ID NO: 24) *Verticillium longisporum*, A0A0G4MTC5_9PEZI ⁽¹ | 99.8 |
| *Nectria haematococca*, BBX16347 ⁽² | 64.7 |
| Man13 (SEQ ID NO: 30) *Aspergillus udagawae,* A0A0K8LCG6_9EURO ⁽¹ | 90.7 |
| *Neosartorya fischeri*, BBX16429 ⁽² | 87.8 |
| Man29 (SEQ ID NO: 39) *Verticillium dahliae,* G2XBE5_VERDV ⁽¹ | 79.4 |
| *Aspergillus terreus*, BBW96507 ⁽² | 66.9 |
| Man30 (SEQ ID NO: 42) *Aspergillus calidoustus*, A0A0U5GVM4_9EURO ⁽¹ | 77.4 |
| *Malbranchea cinnamomea*, BBN39104 ⁽² | 88.6 |

| | |
|---|---|
| ⁽¹ Search performed using NCBI BLAST+ (EMBL-EBI, NCBI BLAST+ - Sequence Similarity Search, UniProt Knowledgebase, BLOSUM62, Gap open 11, Gap extend 1), and EMBOSS Needle (EMBL-EBI, EMBOSS-Needle - Pairwise Sequence Alignment, BLOSUM62, Gap open 10, Gap extend 0.5) for determining the degree of identity. ⁽² Search performed with SequenceBase database https://usgene.sequencebase.com/ | |

### Example 4: Production of recombinant fungal mannanase proteins in Trichoderma reesei

Expression plasmids were constructed for production of recombinant mannanase Man1 (SEQ ID NO: 3), Man2 (SEQ ID NO: 6), Man3 (SEQ ID NO: 9), Man5 (SEQ ID NO: 12), Man8 (SEQ ID NO: 15), Man9 (SEQ ID NO: 18), Man10 (SEQ ID NO: 21), Man11 (SEQ ID NO: 24), Man12 (SEQ ID NO: 27) (SEQ ID NO: 27), Man13 (SEQ ID NO: 30), Man17 (SEQ ID NO: 33), Man27 (SEQ ID NO: 36), Man29 (SEQ ID NO: 39) and Man30 (SEQ ID NO: 42) proteins from *Malbranchea cinnamomea* ALKO4122, *Verticillium dahliae* RF8261, *Melanocarpus albomyces* ALKO4237, *Acremonium alcalophilum, Fusarium equiseti* RF6318, *Acremonium thermophilum* ALKO4245, *Verticillium dahliae* RF8261, *Aspergillus nishimurae* RH3949, *Plectosphaerella cucumerina* and *Gymnascella citrina* (See Examples 2, 3), respectively in *Trichoderma reesei.* The expression plasmids constructed are listed in Table 10. The recombinant mannanase genes including their own signal sequences, were fused to the *T. reesei cel7A*/*cbh1* promoter. The transcription termination was ensured by the *T. reesei cel7A*/*cbh1* terminator and the *A. nidulans amd*S marker gene was used for selection of the transformants as described in Paloheimo *et al.* (2003). The linear expression cassettes (Fig. 1) were isolated from the vector backbones after *Not*I or *Eco*RI digestions and were transformed into *T. reesei* protoplasts. The host strains used, do not produce any of the four major *T. reesei* cellulases (CBHI, CBHII, EGI, EGII). The transformations were performed as in Penttilä *et al.* (1987) with the modifications described in Karhunen *et al.* (1993), selecting acetamidase as a sole nitrogen source (amdS marker gene). The transformants were purified on selection plates through single conidia prior to sporulating them on PD.

**Table 10. The expression cassettes constructed to produce Man1 (SEQ ID NO: 3), Man2 (SEQ ID NO: 6), Man3 (SEQ ID NO: 9), Man5 (SEQ ID NO: 12), Man8 (SEQ ID NO: 15), Man9 (SEQ ID NO: 18), Man10 (SEQ ID NO: 21), Man11 (SEQ ID NO: 24), Man12 (SEQ ID NO: 27) (SEQ ID NO: 27), Man13 (SEQ ID NO: 30), Man17 (SEQ ID NO: 33), Man27 (SEQ ID NO: 36), Man29 (SEQ ID NO: 39) and Man30 (SEQ ID NO: 42) recombinant proteins from *Malbranchea cinnamomea* ALKO4122, *Verticillium dahliae* RF8261, *Melanocarpus albomyces* ALKO4237, *Acremonium alcalophilum, Fusarium equiseti* RF6318, *Acremonium thermophilum* ALKO4245, , *Aspergillus nishimurae* RH3949*, Plectosphaerella cucumerina* and *Gymnascella citrina* in *Trichoderma reesei.* The overall structure of the expression cassettes was as described in** **Fig. 1****.**

| **Mannanase protein** | **Expression plasmid** | **Expression cassette ^{(a}** |
|---|---|---|
| Man1 (SEQ ID NO: 3) | pALK4073 | 7.0 kb *Not*I |
| Man3 (SEQ ID NO: 9) | pALK4071 | 6.7 kb *Not*I |
| Man5 (SEQ ID NO: 12) | pALK4081 | 6.8 kb *Not*I |
| Man8 (SEQ ID NO: 15) | pALK4363 | 6.8 kb *Not*I |
| Man9 (SEQ ID NO: 18) | pALK4091 | 6.7 kb *Not*I |
| Man10 (SEQ ID NO: 21) | pALK4084 | 6.6 kb *Not*I |
| Man11 (SEQ ID NO: 24) | pALK4090 | 6.8 kb *Not*I |
| Man12 (SEQ ID NO: 27) | pALK4271 | 6.6 kb *Not*I |
| Man13 (SEQ ID NO: 30) | pALK4413 | 6.5 kb *Not*I |
| Man17 (SEQ ID NO: 33) | pALK4080 | 7.1 kb *Eco*RI |
| Man27 (SEQ ID NO: 36) | pALK4270 | 6.7 kb *Not*I |
| Man29 (SEQ ID NO: 39) | pALK4411 | 6.6 *Not*I |
| Man30 (SEQ ID NO: 42) | pALK4412 | 6.6 *Not*I |

| | | |
|---|---|---|
| ^{(a} The expression cassette for *T. reesei* transformation was isolated from vector backbone by using *Not*I or *Eco*RI digestions. | | |

The mannanase production of the transformants was analyzed from the culture supernatants of the shake flask cultivations. The transformants were inoculated from the PD slants to shake flasks containing 50 ml of complex lactose-based cellulase inducing medium (Joutsjoki *at al.* 1993) buffered with 5% KH₂PO₄. The mannanase protein production of the transformants was analyzed from culture supernatants after growing them for 7 days at 30 °C, 250 rpm. Heterologous production of recombinant proteins was analyzed by SDS-PAGE with subsequent Coomassie staining.

The best producing transformants were chosen to be cultivated in laboratory scale bioreactors. The transformants were cultivated in bioreactors either on batch or by additional feeding type of process under protein inducing conditions at a typical mesophilic fungal cultivation temperature and slightly acidic conditions. The cultivation was continued until depletion of the medium sugars or until suitable yield was reached. The supernatants were recovered for application tests by centrifugation or by filtration.

### EXAMPLE 5: Purification of Man1 (SEQ ID NO: 3) mannanase, not according to the invention

Cells and solids were removed from the fermentation culture medium by centrifugation for 10 min, 4000 g at 4°C. The supernatant of 10 ml was used for protein purification. The sample was filtered through 0.44 µm PVDF membrane (Millex-HV, Merck Millipore Ltd,Carrigtwohill, IRL). The filtrate was loaded onto a HiPrep 26/10 Desalting column (GE Healthcare, Uppsala, Sweden) equilibrated in 20 mM Tris pH 7. The desalted sample was then loaded onto a 5 ml HiTrap Q HP column (GE Healthcare, Uppsala, Sweden) pre-equilibrated with 20 mM Tris pH 7. After sample loading, the column was washed with 20 ml of the same buffer . Proteins were eluted with linear salt gradient 20 mM Tris, 500 mM NaCl pH 7 in 15 CVs. Fractions of 5 ml were collected and analyzed by SDS-PAGE. The fractions containing target protein were combined and concentrated to 2 ml using Vivaspin 20, 10 kDa MWCO ultrafiltration devices (GE Healthcare). The concentrated sample was further fractionated using Superdex 75 26/60 gel-filtration column equilibrated with 20 mM MES, 200 mM NaCl pH 6,5. Fractions of 2 ml were collected and analyzed by SDS-PAGE. Fractions containing pure mannanase were combined. Enzyme content of the purified sample was determined using UV absorbance 280 nm measurements. Excitation coefficients for each mannanases were calculated on the bases of amino acid sequence of the enzyme by using ExPASy Server (http://web.expasy.org/protparam/). (Gasteiger et al. 2005).

Other mannanases were purified using the same protocol but changing the buffer composition in desalting and ion exchange steps. Buffer compositions are shown in Table 11.

**Table 11.**

| **Mannanase** | **Buffers used in ion exchange chromatography** |
|---|---|
| **Man1 (SEQ ID NO: 3)** | 20 mM Tris pH 7 |
| **Man3 (SEQ ID NO: 9)** | 20 mM MES pH 6 |
| **Man5 (SEQ ID NO: 12)** | 20 mM Hepes pH 7 |
| **Man8 (SEQ ID NO: 15)** | 20 mM MES pH 6 |
| **Man9 (SEQ ID NO: 18)** | 20 mM MES pH 6,5 |
| **Man10 (SEQ ID NO: 21)** | 20 mM MES pH 6,5 |
| **Man11 (SEQ ID NO: 24)** | 20 mM MES pH 6 |
| **Man12 (SEQ ID NO: 27)** | 20 mM MES pH 6 |
| **Man13 (SEQ ID NO: 30)** | 20 mM MES pH 6,5 |

Purified samples were above 95% pure.

### pH profiles of mannanases

The pH profiles of purified mannanases were determined using the beta-mannazyme tablet assay Azurine-crosslinked carob galactomannan (T-MNZ 11/14) from Megazyme with minor modifications to suggested protocol. The linearity of the assay was checked with each purified enzymes. The assay was performed in 40 mM Britton-Robinson buffer adjusted to pH values between 4 and 11. The enzyme solution was diluted into the assay buffer and 500µl of enzyme solution was equilibrated at 50°C water bath for 5 min before adding one substrate tablet. After 10 minutes, the reaction was stopped by adding 10 ml 2% Tris pH 12. The reaction tubes were left at room temperature for 5 min, stirred and the liquid filtered through a Whatman No.1 paper filter. Release of blue dye from the substrate was quantified by measuring the absorbance at 595 nm. Enzyme activity at each pH was reported as relative activity where the activity at the pH optimum was set to 100%. The pH profiles were shown in Figures 4a and 4b.

Relative activity (%) of mannanase is calculated by dividing mannanase activity of a sample by the mannanase activity of a reference sample. In the case of pH profile, the reference sample is a sample at the optimal pH. In the case of temperature profile the reference sample is a sample at the optimal temperature.

### Temperature profiles of mannanases

The temperature optimum of purifed mannanases was determined using the beta-mannazyme tablet assay Azurine-crosslinked carob galactomannan (T-MNZ 11/14) from Megazyme with minor modifications to suggested protocol. The assay was performed at temperatures varying between 30-90°C for 10 minutes in 40 mM Britton-Robinson buffer pH7. Enzyme activity was reported as relative activity where the activity at temperature optimum was set to 100%. The temperature profiles were shown in Figures 2-3.

Molecule weight determined in SDS PAGE gel, temperature and pH optimum of enzymes are shown in Table 12.

**Table 12**

| | **MW SDS PAGE** | **pH optimum** | **Temperature optimum** |
|---|---|---|---|
| **Man1 (SEQ ID NO: 3)** | 40-60 kDa | 5-7 | 50°C - 60°C |
| **Man3 (SEQ ID NO: 9)** | 40-60 kDa | 5-8 | 40°C - 60°C |
| **Man5 (SEQ ID NO: 12)** | 40-60 kDa | 5-8 | 50°C - 70°C |
| **Man8 (SEQ ID NO: 15)** | 50-70 kDa | 6-9 | 40°C - 60°C |
| **Man9 (SEQ ID NO: 18)** | 50-70 kDa | 5-9 | 40°C - 60°C |
| **Man10 (SEQ ID NO: 21)** | 45-60 kDa | 5-8 | 50°C - 70°C |
| **Man11 (SEQ ID NO: 24)** | 50-90 kDa | 5-8 | 50°C - 70°C |
| **Man12 (SEQ ID NO: 27)** | 30-40 kDa | 4-8 | 50°C - 70°C |

### EXAMPLE 6 Assay of galactomannanase activity by DNS -method

Mannanase activity (MNU) was measured as the release of reducing sugars from galactomannan (0.3 w/w-%) at 50°C and pH 7.0 using 5 min reaction time. The amount of released reducing carbohydrates was determined spectrophotometrically using dinitrosalicylic acid.

Subtrate (0,3 w/w-%) used in the assay was prepared as follows: 0.6 g of locust bean gum (Sigma G-0753) was blended in 50 mM sodium citrate buffer pH 7 (or citrate phosphate buffer pH 7) at about 80 °C using a heating magnetic stirrer and heated up to boiling point. The solution was cooled and let to dissolve overnight in a cold room (2 - 8 °C) with continuous stirring after which insoluble residues were removed by centrifugation. After that solution was filled up to 200 ml volume with the buffer. Substrate was stored as frozen and melted by heating in a boiling water bath to about 80 °C, cooled to room temperature and mixed carefully before use.

DNS reagent used in the assay was prepared by dissolving 50 g of 3.5-dinitrosalisylic acid (Sigma D-550) in about 4 liter of water. With continuous magnetic stirring 80.0 g of NaOH was gradually added and let to dissolve. An amount of 1500 g of Rochelle Salt (K-Na-tartrate, Merck 8087) was added in small portions with continuous stirring. The solution that might have been cautiously warmed to a maximum temperature of 45 °C was cooled to room temperature and filled up to 5000 ml. After that it was filtered through Whatman 1 filter paper and stored in a dark bottle at room temperature.

The reaction was started by adding 1.8 ml of substrate solution to two test tubes and let to equilibrate at 50 °C for 5 minutes, after which 200 µl of suitably diluted enzyme solution was added to one of the tubes, mixed well with vortex mixer and incubated exactly for 5 min at 50 °C. Enzyme blanks didn't need to be equilibrated or incubated. The reaction was stopped by adding 3.0 ml of DNS reagent into both tubes and mixed. 200 µl of sample solution was added to the enzyme blank tubes. Both tubes were placed in a boiling water bath. After boiling for exactly 5 minutes, the tubes were placed in a cooling water bath and allowed to cool to room temperature. The absorbance of sample was measured against the enzyme blank at 540 nm and activity was read from the calibration curve and multiplied by the dilution factor. A suitable diluted sample yielded an absorbance difference of 0.15 - 0.4.

Standard curve was prepared from 20 mM mannose stock solution by dissolving 360 mg of mannose (SigmaM-6020, stored in a desiccator) to 100 ml of assay buffer and diluted to solutions containing 3, 6, 10 and 14 µmol/ml of mannose. Standards were handled like the samples except for incubation at 50°C. The absorbances were measured against the reagent blank (containing buffer instead of mannose) at 540nm. A calibration curve was prepared for every series of assays.

One mannanase unit (MNU) was defined as the amount of enzyme that produces reductive carbohydrates having a reductive power corresponding to one nmol of mannose from galactomannan in one second under the assay conditions (1 MNU = 1 nkat).

### EXAMPLE 7 Stain removal performance of fungal mannanases with commercial detergents without bleaching agents

Cultivation samples of fungal mannanases (Man1 (SEQ ID NO: 3), Man3 (SEQ ID NO: 9), Man5 (SEQ ID NO: 12), Man8 (SEQ ID NO: 15), Man9 (SEQ ID NO: 18), Man10 (SEQ ID NO: 21), Man11 (SEQ ID NO: 24), Man12 (SEQ ID NO: 27),Man13 (SEQ ID NO: 30), Man29 (SEQ ID NO: 39) and Man30 (SEQ ID NO: 42)) produced in *Trichoderma* (as described in Example 4), were tested for their ability to remove mannanase sensitive standard stains at 40 °C and water hardness of 16°dH with commercial detergents without bleaching agents. The following artificially soiled test cloths from Center for testmaterial B.V. (the Netherlands) were used with Man1 (SEQ ID NO: 3), Man3 (SEQ ID NO: 9), Man5 (SEQ ID NO: 12), Man8 (SEQ ID NO: 15), Man9 (SEQ ID NO: 18), Man10 (SEQ ID NO: 21), Man11 (SEQ ID NO: 24), Man12 (SEQ ID NO: 27) and Man13 (SEQ ID NO: 30) cultivation samples: Chocolate pudding mannanase sensitive on cotton (E-165), Locust bean gum, with pigment on cotton (C-S-73) and Guar gum with carbon black on cotton (C-S-43). With Man29 (SEQ ID NO: 39) and Man30 (SEQ ID NO: 42) cultivation samples stains E-165 and C-S-73 were used. The fabric was cut in 6 cm × 6 cm swatches and 2 pieces of each were used in test.

Commercial heavy duty liquid detergent A containing all other enzymes except mannanase was used at concentration of 4.4 g per liter of wash liquor, commercial heavy duty liquid detergent B was used 5.0 g/l and commercial color detergent powder without enzymes was used at 3.8 g/l. Detergent containing wash liquors we prepared in synthetic tap water with hardness of 16 °dH. Protease Savinase^{®} 16 L (0.5 w/w %) and amylase Stainzyme^{®} 12 L (0.4 w/w %) was added into hard water used with commercial heavy duty liquid detergent B and commercial color detergent powder. The liquid detergent already contained amylase and protease. The pH of the wash liquor of Color detergent powder was approximately 10 and with the liquid detergent approximately 8.2 - 8.3.

Mannanase dosages were in range 0 - 0.4 % of detergent weight, the amount depending on test and the strength of enzyme preparation. For the evaluation the dosages were calculated as enzyme activity units (MNU) per ml wash liquor. Activity was measured as described in Example 6. Control sample contained the detergent solution but no mannanase.

For synthetic tap water with hardness of 16 °dH the following stock solutions were prepared in deionized water (Milli-Q or equivalent):
Stock solution with 1000 °d Calcium-hardness: CaCl2 × 2 H2O (1.02382.1000, Merck KGaA, Germany) 26,22 g/l
Stock solution with 200 °d Magnesium-hardness: MgSO4 × 7 H2O (1.05886.1000, Merck KGaA, Germany) 8,79 g/l H2O
NaHCO3 stock solution: NaHCO3 (1.06329.1000 Merck KGaA, Germany) 29,6 g/l

13,3 ml CaCl2 solution, 13,3 ml MgSO4 solution and 10,0 ml of freshly made NaHCO3 solution were added in volumetric flask in the given order, made up to 1 liter with deionized water and mixed. The hardness of water was determined by complexometric titration and found correct.

Stain removal treatments were performed in Atlas LP-2 Launder-Ometer as follows. Launder-Ometer was first preheated to 40°C. Then detergent, 250 ml synthetic tap water with hardness of 16 °dH and diluted enzyme (<1.0 ml) were added into 1.2 liter containers. Stains were added and the Launder-Ometer was run at 40°C for 60 min with a rotation speed of 42 rpm. After that the swatches were carefully rinsed under running water and dried overnight at indoor air, on a grid protected against daylight.

The stain removal effect was evaluated by measuring the colour as reflectance values with Konica Minolta CM-3610A spectrophotometer using L*a*b* color space coordinates (illuminant D65/10°, 420 nm cut). Fading of the stains, indicating mannanase performance (stain removal efficiency) was calculated as ΔL* (delta L*), which means lightness value L* of enzyme treated fabric minus lightness value L* of fabric treated with washing liquor without mannanase (control). Final results (total stain removal effect) were shown as sum of ΔL* of each stains. Color values of each stain were average of 2 swatches.

Mannanase cultivation samples (Man1, Man3, Man5, Man8, Man9, Man10, Man11, Man12, Man13) were tested with 3 different stains in commercial liquid detergent A (Figs. 6-8). Man29 and Man30 cultivation samples were tested with 2 different stains in commercial liquid detergent B (Fig 9). All the mannanases according to the invention increased considerably the stain removal efficiency in liquid detergent.

The results obtained with commercial color detergent powder (Figs 10 -11) show that mannanases Man1 (SEQ ID NO: 3), Man3 (SEQ ID NO: 9), Man5 (SEQ ID NO: 12), Man8 (SEQ ID NO: 15), Man9 (SEQ ID NO: 18), Man11 (SEQ ID NO: 24) and Man12 (SEQ ID NO: 27) are performing well also in commercial detergent powder.

### EXAMPLE 8 Stain removal performance of fungal mannanases with bleach containing detergent, not according to the invention

Fungal mannanases Man1 (SEQ ID NO: 3), Man3 (SEQ ID NO: 9) and Man5 (SEQ ID NO: 12) produced in *Trichoderma* (as described in Example 4) were tested for their ability to remove mannanase sensitive standard stains at 40 °C and water hardness of 16°dH with commercial bleach detergent powder. Test system was similar to that described in Example 7, except commercial bleach detergent powder was used at concentration of 4,2 g per liter of wash liquor and pH of the wash liquor was approximately 9.5. Protease Savinase^{®} 16 L (0.5 w/w %) and amylase Stainzyme^{®} 12 L (0.4 w/w %) were added into hard water used in test, since the detergent didn't contain any enzymes.

The color of the swatches after treatment was measured and results calculated as sum of ΔL* of each 3 stains as described in Example 7.

The results obtained with commercial bleach containing detergent (Fig. 12) show that mannanases Man1 (SEQ ID NO: 3), Man3 (SEQ ID NO: 9) and Man5 (SEQ ID NO: 12) increased the stain removal efficiency considerably. In previous tests also cultivations samples of Man8 (SEQ ID NO: 15), Man9 (SEQ ID NO: 18) and Man11 (SEQ ID NO: 24) and Man12 (SEQ ID NO: 27) were performing with bleach detergent (data not shown).

### EXAMPLE 9 Stability of Man1 (SEQ ID NO: 3) mannanase (not according to the invention) in commercial liquid detergent

The stability of Man1 (SEQ ID NO: 3) preparation produced in *Trichoderma* (as described in Example 4) was tested in OMO Color liquid obtained from local super market and compared to commercial mannanase preparation Mannaway^{®} 4,0 L. Mannanase preparations were added 0.5s w/w-% in detergents and samples were incubated in plastic tubes with caps at 37°C for 5 or 8 weeks. The activity was measured at certain intervals by activity assay described in Example 6 except using 30 min incubation time.

The results in Omo Color (Fig.13) show that Man1 (SEQ ID NO: 3) had considerably better stability compared to Mannaway^{®} 4.0 L. The results of the stability experiments show that the mannanase according to the invention was stable in detergents for several weeks even when stored at high temperature like 37°C. The stability of the mannanase according to the invention (Man1 (SEQ ID NO: 3)) in liquid detergent is considerably improved compared to a commercial bacterial mannanase.

### Example 10: Efficiency study with mannanase alone and in combination with a non-starch polysaccharide (NSP) degrading enzyme in broilers

Effects of recombinant mannanases of the invention are studied on growth in broilers. Ultrafiltrate of the fermentation broth including the recombinant mannanase is dried and target levels applied to a pelleted broiler diet alone or in combination with a commercial available xylanase based product.

A control diet based on corn and dehulled solvent extracted soybean meal is fed without enzyme or added by different levels of the recombinant mannanase of the invention alone or in combination with a standard dose of a commercial xylanase. Initial weight of the broilers is between 30 g and 50 g. The trial lasts between three and five weeks. Each treatment consists at minimum of six replicates with 10 broilers each. In each case the diet is analysed for moisture, crude protein, crude fibre, fat, ash, and enzyme activity.

Five diets are prepared:
1) unsupplemented control (BD, )
2) BD + mannanase 1-500 mg/kg
3) BD + mannanase 1-1000 mg/kg
4) BD + mannanase 1-500 mg/kg + xylanase 1-10 mg/kg
5) BD + xylanase 1-10 mg/kg

Health status and mortality of the animals is checked daily by visual inspection. At days 0, 14, and 35 body weight gain (BW), feed intake (FI), and feed-conversion ratio (FCR) are measured. FCR is calculated as the total feed consumed divided by the weight gain during the same period. Determination of the effect of the recombinant mannanases is based on the comparison to those animals fed the same diet or the same diet but added by xylanase.

### Example 11: Instant coffee production

Pure mannan is the main storage polysaccharide component of coffee endosperms and is responsible for their high viscosity, which negatively affects the technological processing of instant coffee and increases energy consumption during drying. Those effects are attributed to mannan forming hard, insoluble crystalline structures. β-mannanase, often together with other enzymes such as pectinase and cellulase, is added during the concentration step of instant coffee production to reduce viscosity in coffee extracts. Mannanase is also being employed for hydrolyzing galactomannans present in a liquid coffee extract in order to inhibit gel formation during freeze drying of instant coffee. Furthermore, due to the use of enzymatic treatment the coffee bean extracts can be concentrated by a low cost procedure such as evaporation.

The test is performed according the following flow-chart of Fig. 14 at temperature of 10°C and an enzyme dosage of 0,15% d.s.

Mannanases of the invention are tested in mixture composed of different enzymes, such as pectinases and cellulases.

The viscosity of the coffee extract increases significantly over time under standard process conditions. However, with the application of Enzyme mixture the viscosity is significantly reduced which results in an improved downstream processing such as spray- or freeze drying.

### Example 12: Pineapple processing with Mannanase

In particular, mannanase is useful for pineapple mill juice extraction and clarification, as pineapple contains a large fraction of mannans, including glucomannans and galactomannans.

Mannanase helps to improve extraction of valuable fruit components, lower the viscosity of fruit juice prior to concentration, and increase filtration rate and stability of the final product.

The pineapples are crushed in a meat grinder and fill 500g mash in a 1000ml beaker. The enzyme is applied at 21°C with a reaction time of 60 minutes. The mash is then pressed with a small Hafico press according to the press protocol: 0 bar 2 min - 50 bar 2 min - 100 bar 2 min - 150 bar 2 min - 200 bar 1 min - 300 bar 1 min - 400 bar 1min. The obtained juice is then centrifuged at 4500 rpm for 5 minutes and analyzed for turbidity and viscosity.

Enzyme materials used in the tests are three enzyme mixtures, Mixture A, Mixture B and Mixture C (Table 13).

The enzymes are first diluted with tab water before being added to the pineapple mash.

**Table 13. Enzyme mixtures**

| | **Enzyme activity** | **dosage[ppm]** | **5 ml of [% enzyme solution]** |
|---|---|---|---|
| blank | | | 5 ml H2O |
| Mixture A | Pectinase | 50 | 0,50% |
| Mixture B | Pectinase + Arabanase | 50 | 0,50% |
| Mixture C | Pectinase + Mannanase | 50 | 0,50% |

Applying a mannanase enzyme in pineapple processing leads to higher juice yield and to lower juice turbidity.

### Example 13: Mannanase treatment of soya beans for soya milk production

For the enzymatic treatment of soya beans to get soya milk the "hot process" is commonly used. For the hot soya milk process the dried soya beans were mixed and crushed in a mixer with boiling tap water in a ratio of 1:7 (soaked beans: water). The whole soya slurry is cooled down to 50-55 °C before enzyme addition. The pH level for the soya slurry should be around pH 6,5 and can be adjusted with NaHCO₃. The mannanase enzyme is dosed at 1kg /t of dried soya beans into the slurry and stirred for 30 min. After completion of the reaction time, the slurry is pressed to obtain the final product: soya milk. In order to ensure the same pressing profile, the pressure as well as the corresponding pressing time is specified, as shown in Table 14. Besides the sample for enzymatic reaction, a control sample without any enzyme is prepared, in which the enzyme solution was replaced with water.

**Table 14. Press scheme**

| | | | | |
|---|---|---|---|---|
| **pressure [bar]** | 0 | 50 | 100 | 300 |
| **time [min]** | 2 | 2 | 2 | 1 |

After pressing the soya milk is heated in a microwave until boiling to stop the enzyme reaction. Analysis of the soya milk:
- Yield in gram/time
- °Brix, which gives a direct correlation of the amount of sugar in the soy milk, is determined with a refractometer
- The turbidity of the juice is measured with a NTU-photometer, which measures the nephelometric turbidity.
- The brightness will be measured with a LAB-measurement
- Protein content is determined with a CN-Analyser (combustion method)
- Flavour

The enzymatic treated soya milk shows a higher yield, brighter colour, increased °Brix, a lower turbidity, a higher protein content and a better taste (off flavour removal).

### Example 14: Wash performance of liquid detergent compositions not according to the invention

The wash performance of liquid detergent compositions according to present invention was determined by using standardized stains obtainable from CFT (Center for Testmaterials) B.V., Vlaardingen, Netherlands ("CFT"), Eidgenössische Material- und Prüfanstalt Testmaterialien AG [Federal materials and testing agency, Testmaterials], St. Gallen, Switzerland ("EMPA") and Warwick Equest Ltd Unit 55, Consett Business Park, Consett, County Durham ("Equest").

A liquid washing agent with the following composition was used as base formulation (all values in weight percent):

**Table 15.**

| Chemical name | Active substance raw material [%] | Active substance detergent formulation [%] |
|---|---|---|
| Water demin. | 100 | Rest |
| Alkyl benzene sulfonic acid | 96 | 2-7 |
| Anionic surfactants | 70 | 6-10 |
| C12-C18 Fatty acid sodium salt | 30 | 1-4 |
| Nonionic surfactants | 100 | 4-7 |
| Phosphonates | 40 | 0.1-2 |
| Citric acid | 100 | 1-3 |
| NaOH | 50 | 1-4 |
| Boronic acid | 100 | 0.1-2 |
| Antifoaming agent | 100 | 0.01-1 |
| Glycerol | 100 | 1-3 |
| Enzymes | 100 | 0.1-2 |
| Preserving agent | 100 | 0.05-1 |
| Ethanol | 93 | 0.5-2 |
| Optical brightener | 90 | 0.01-1 |
| Perfume | 100 | 0.1-1 |
| Dye | 100 | 0.001-0.1 |

The pH of the detergent composition was between 8.2-8.6.

Based on this base formulation, liquid detergent compositions 1, 2 and 3 were prepared by adding respective enzymes as indicated below:
Composition 1: Enzyme according to SEQ ID NO: 3 (Man 1)
Composition 2: Enzyme according to SEQ ID NO: 9 (Man 3)
Composition 3: Enzyme according to SEQ ID NO: 12 (Man 5)

The wash was performed as follows according to the AISE Method: 3.5 kg Clean ballast cloth, 4 SBL Cloths, Miele washing machine, 20°C and 40°C Short program. All mannanases were added in the same amounts based on total protein content.

The dosing ratio of the liquid washing agent was 4.0 grams per liter of washing liquor. The washing procedure was performed for 60 minutes at a temperature of 20°C and 40°C, the water having a water hardness between 15.5 and 16.5° (German degrees of hardness).

The results obtained are the difference values between the remission units obtained with the detergents and the remission units obtained with the detergent containing the commercially available reference mannanase (Mannaway 4.0L, obtained from Novozymes). A positive value therefore indicates an improved wash performance of the detergent compositions comprising the mannanases of present invention compared to the same detergent composition comprising the reference mannanase. Within the washing test a large range of stains were tested.

The whiteness, i.e. the brightening of the stains, was determined photometrically as an indication of wash performance. A Minolta CM508d spectrometer device was used, which was calibrated beforehand using a white standard provided with the unit.

The results obtained are the difference values between the remission units obtained with the detergents and the remission units obtained with the detergent containing the enzyme combinations. A positive value therefore indicates an improved wash performance due to the enzyme combinations present in the detergent. Mannanases of the present invention in detergent compositions show improved performance on a variety of mannan comprising stains.

**Table 16.**

| Stain | Man1 (SEQ ID NO: 3) | Man3 (SEQ ID NO: 9) | Man5 (SEQ ID NO: 12) |
|---|---|---|---|
| Chocolate creme (EMPA 160 [CO]) | n.d. | 1.5 | 1.5 |
| Starch/Pigment (WFK 10R [CO]) | n.d. | 0.6 | 1.3 |
| Black currant juice (CFT CS12 [CO]) | 1.0 | 0.9 | 2.5 |
| Groundnutoil, pigments, high milk (CFT C1 0 [CO]) | n.d. | 0.9 | 1.0 |
| Blood (aged) (CFT CS01 [CO]) | n.d. | 1.5 | 1.1 |
| Chocolate Icecream L [CO] (EQUEST) | n.d. | n.d. | 1.2 |
| Cocoa [CO] (EQUEST) | 0.9 | 1.3 | n.d. |

### Example 15: Wash performance of powder detergent compositions not according to the invention

The wash performance of powder detergent compositions according to present invention was determined by using standardized stains obtainable from CFT (Center for Testmaterials) B.V., Vlaardingen, Netherlands ("CFT"), Eidgenössische Material- und Prüfanstalt Testmaterialien AG [Federal materials and testing agency, Testmaterials], St. Gallen, Switzerland ("EMPA") and Warwick Equest Ltd Unit 55, Consett Business Park, Consett, County Durham ("Equest").

A solid washing agent with the following composition was used as base formulation (all values in weight percent):

**Table 17.**

| Chemical Name | Active substance raw material [%] | Active substance detergent formulation [%] |
|---|---|---|
| Water demin. | 100 | 1-4 |
| Alkyl benzene sulfonic acid | 97 | 9-13 |
| Nonionic surfactants | 100 | 4-7 |
| Percarbonates | 88 | 9-13 |
| TAED | 92 | 1-5 |
| Phosphonates | 60 | 0.1-3 |
| Polyacrylates | 45 | 1-4 |
| Sodium silicate | 40 | 5-10 |
| Sodium carbonate | 100 | 18-22 |
| Carboxymethylcellulose | 69 | 1-4 |
| Soil release polymer | 100 | 0.1-1 |
| Optical brightener | 70 | 0.1-1 |
| Antifoaming agent | t.q. | 0.01-1 |
| Sodium sulfate | 100 | 22-30 |
| Enzymes | 100 | 0.1-1 |
| Perfume | 100 | 0.1-1 |
| NaOH | 100 | 0.1-1 |
| Rest | - | 1-4 |

Based on this base formulation, solid detergent compositions 4 and 5 were prepared by adding respective enzymes as indicated below:
Composition 4: Enzyme according to SEQ ID NO:3 (Man1)
Composition 5: Enzyme according to SEQ ID NO:12 (Man5)

The wash was performed as follows according to the AISE Method: 3.5 kg Clean ballast cloth, 4 SBL Cloths, Miele washing machine, 20°C and 40°C Short program. All mannanases were added in the same amounts based on total protein content.

The dosing ratio of the powder washing agent was 3.8 grams per liter of washing liquor. The composition of the detergent is listed in Table 17. The washing procedure was performed for 60 minutes at a temperature of 20°C and 40°C, the water having a water hardness between 15.5 and 16.5° (German degrees of hardness).

The whiteness, i.e. the brightening of the stains, was determined photometrically as an indication of wash performance. A Minolta CM508d spectrometer device was used, which was calibrated beforehand using a white standard provided with the unit.

The results obtained are the difference values between the remission units obtained with the detergents and the remission units obtained with the detergent containing the reference mannanase (Mannaway 4.0L, obtained from Novozymes). A positive value therefore indicates an improved wash performance of the mannanases in the detergent. Mannanases of the invention show improved performance on several stains in Table 18. Therefore, it is evident from Table 18 that mannanases according to the invention show improved wash performance.

**Table 18.**

| Stain | Man1 (SEQ ID NO: 3) | Man5 (SEQ ID NO: 12) |
|---|---|---|
| Milk/Carbon black (H-MR-B [CO]) | n.d. | 1.4 |
| Pudding (EMPA 165 [CO]) | 0.6 | 1.6 |
| Porridge (EMPA 163 [CO]) | n.d. | 0.5 |
| Sebum BEY with carbon black (CFT CS32 [CO]) | n.d. | 0.7 |
| Lipstick, diluted, Red (CFT CS216 [CO]) | n.d. | 0.9 |
| Chocolate drink, pure (CFT CS44 [CO]) | n.d. | 1.4 |
| Groundnutoil, pigments, high milk (CFT C10 [CO]) | 0.6 | 1.6 |
| Salad dressing, with natural black (CFT CS06 [CO]) | n.d. | 0.5 |

Without limiting the scope and interpretation of the patent claims, certain technical effects of one or more of the aspects or embodiments disclosed herein are listed in the following: a technical effect is degradation or modification of mannan. Another technical effect is provision of mannanase which has good storage stability and enzyme activity.

### References:

Bendtsen JD, Nielsen H, von Heijne G, and Brunak S. (2004) Improved prediction of signal peptides: SignalP 3.0. J. Mol.Biol. 340:783-795.
Gasteiger E., Hoogland C., Gattiker A., Duvaud S., Wilkins M.R., Appel R.D., Bairoch A.;Protein Identification and Analysis Tools on the ExPASy Server; (In) John M. Walker (ed): The Proteomics Protocols Handbook, Humana Press (2005). pp. 571-607.
Joutsjoki VV, Torkkeli TK, and Nevalainen KMH. (1993) Transformation of Trichoderma reesei with the Hormoconis resinae glucoamylase P (gamP) gene: production of a heterologous glucoamylase by Trichoderma reesei. Curr. Genet. 24: 223-228.
Karhunen T, Mäntylä M, Nevalainen KMH, and Suominen PL. (1993) High frequency one-step gene replacement in Trichoderma reesei. I. Endoglucanase I overproduction. Mol. Gen. Genet. 241: 515-522.
Nielsen H, Engelbrecht J, Brunak S, and von Heijne G. (1997) Identification of prokaryotic and eykaryotic signal peptides and prediction of their cleavage sites. Protein. Eng. 10: 1-6.
Nielsen H, and Krogh A. (1998) Prediction of signal peptides and signal anchors by a hidden Markov model. In: Proceedings of the Sixth International Conference on Intelligent Systems for Molecular Biology (ISMB 6), AAAI Press, Menlo Park, California, p. 122-130.
Paloheimo M, Mäntylä A, Kallio J, and Suominen P. (2003) Highyield production of a bacterial xylanase in the filamentous fungus Trichoderma reesei requires a carrier polypeptide with an intact domain structure. Appl. Env. Microbiol. 69: 7073-7082.
Penttilä M, Nevalainen H, Rättö M, Salminen E, and Knowles J. (1987) A versatile transformation system for the cellulolytic filamentous fungus Trichoderma reesei. Gene 61: 155-164.
Sambrook J, and Russell DW. (2001) Molecular cloning, a laboratory manual. Cold Spring Harbor Laboratory, New York, US.

## Claims

1. A recombinant polypeptide having mannanase activity and comprising an amino acid sequence, which has:
at least 92% sequence identity with Man13 (SEQ ID NO: 30).

2. A recombinant host cell comprising genetic elements that allow producing at least one recombinant polypeptide with mannanase activity, which has at least 92% sequence identity with Man13 (SEQ ID NO: 30) and
wherein the host cell is selected from a group consisting of fungal cells,
filamentous fungal cells from Division *Ascomycota,* Subdivision *Pezizomycotina*; preferably from the group consisting of members of the Class *Sordariomycetes,* Subclass *Hypocreomycetidae,* Orders *Hypocreales* and *Microascales* and *Aspergillus, Chrysosporium, Myceliophthora* and *Humicola*;
more preferably from the group consisting of Families *Hypocreacea, Nectriaceae, Clavicipitaceae, Microascaceae,* and Genera *Trichoderma* (anamorph of *Hypocrea), Fusarium, Gibberella, Nectria, Stachybotrys, Claviceps, Metarhizium, Villosiclava, Ophiocordyceps, Cephalosporium,* and *Scedosporium*;
more preferably from the group consisting of *Trichoderma reesei (Hypocrea jecorina), T. citrinoviridae, T. longibrachiatum, T. virens, T. harzianum, T. asperellum, T. atroviridae, T. parareesei, Fusarium oxysporum, F. gramineanum, F. pseudograminearum, F. venenatum, Gibberella fujikuroi, G. moniliformis, G.* zeaea, *Nectria (Haematonectria) haematococca, Stachybotrys chartarum, S. chlorohalonata, Claviceps purpurea, Metarhizium acridum, M. anisopliae, Villosiclava virens, Ophiocordyceps sinensis, Acremonium (Cephalosporium) chrysogenum,* and *Scedosporium apiospermum, and Aspergillus niger, Aspergillus awamori, Aspergillus oryzae, Chrysosporium lucknowense, Myceliophthora thermophila, Humicola insolens,* and *Humicola grisea,*
bacterial cells, preferably gram positive Bacilli such as *B. subtilis, B. licheniformis, B. megaterium, B. amyloliquefaciens, B. pumilus,* gram negative bacteria such as *Escherichia coli*, actinomycetales such as *Streptomyces* sp., and
yeasts such as *Saccharomyces cerevisiae, Pichia pastoris, Yarrowia lipolytica*, most preferably *Trichoderma reesei* or *Bacillus.*

3. The recombinant host cell of claim 2 wherein the recombinant polypeptide is a fusion protein which, in addition to having the amino acid sequence having mannanase activity, comprises at least one of:
an amino acid sequence providing a secretory signal sequence;
an amino acid sequence which facilitates purification, such as an affinity tag or His-tag;
an amino acid sequence which enhances production, such as an amino acid sequence which is a carrier or CBM;
an amino acid sequence having an enzyme activity; and
an amino acid sequence providing for the fusion protein with binding affinity, such as a carbohydrate binding moiety.

4. An enzyme composition comprising a recombinant polypeptide having mannanase activity and at least 92% sequence identity with Man13 (SEQ ID NO: 30);
and optionally at least one further mannanase having
at least 70% sequence identity with Man8 (SEQ ID NO: 15); or
at least 93% sequence identity with Man9 (SEQ ID NO: 18); or
at least 70% sequence identity with Man10 (SEQ ID NO: 21); or
at least 80% sequence identity with Man29 (SEQ ID NO: 39); or
at least 89% sequence identity with Man30 (SEQ ID NO: 42); or
100% sequence identity with Man1 (SEQ ID NO: 3); or
100% sequence identity with Man2 (SEQ ID NO: 6); or
100% sequence identity with Man3 (SEQ ID NO: 9); or
100% sequence identity with Man5 (SEQ ID NO: 12); or
100% sequence identity with Man11 (SEQ ID NO: 24); or
100% sequence identity with Man12 (SEQ ID NO: 27); or
100% sequence identity with Man17 (SEQ ID NO: 33); or
100% sequence identity with Man27 (SEQ ID NO: 36).

5. The enzyme composition of claim 4 further comprising:
a. at least one preservative selected from benzoic acid, sodium benzoate, hydroxybenzoate, citric acid, ascorbic acid, or a combination thereof;
b.optionally at least one polyol selected from propylene glycol, glycerol, a sugar, sugar alcohol, lactic acid, boric acid, boric acid derivative, aromatic borate ester, phenyl boronic acid derivative, peptide, or a combination thereof;
c. optionally at least one enzyme selected from proteases, amylases, cellulases, lipases, xylanases, mannanases, cutinases, esterases, phytases, DNAses, pectinases, pectinolytic enzymes, pectate lyases, carbohydrases, arabinases, galactanases, xanthanases, laccases, peroxidases and oxidases with or without a mediator, or a combination thereof; and
d. optionally at least one filler selected from maltodextrin, flour, sodium chloride, sulfate, sodium sulfate, or a combination thereof.

6. The enzyme composition of claims 4-5 in the form of a liquid composition or a solid composition such as solution, dispersion, paste, powder, granulate, coated granulate, tablet, cake, crystal, crystal slurry, gel, or pellet.

7. A method for producing mannanase comprising
a. cultivating the recombinant host cell of claims 2-3, wherein
i. the genetic elements comprise at least one control sequence which controls the production of the recombinant polypeptide in the recombinant host cell under conditions that allow production of the polypeptide;
ii. the genetic elements optionally comprise at least one sequence encoding a signal sequence for transporting the polypeptide outside the host cell; and
iii. cultivating is carried out in conditions allowing production of the polypeptide; and
b. recovering the mannanase.

8. A method for degrading or modifying mannan containing material comprising treating said mannan containing material with an effective amount of the recombinant polypeptide of claim 1 or the enzyme composition of claims 4-6.

9. The method of claim 8 wherein the mannan containing material is plant based material, textile, waste water, sewage, oil, or a combination thereof.

10. An animal feed comprising the recombinant polypeptide of claim 1 or the enzyme composition of claims 4-6 and at least one protein source of plant origin or a mannan containing product or by-product, and
a. Optionally at least one enzyme selected from protease, amylase, phytase, xylanase, endoglucanase, beta-glucanase, or a combination thereof; and
b. Optionally at least one filler selected from maltodextrin, flour, salt, sodium chloride, sulfate, sodium sulfate, or a combination thereof.

11. A feed supplement comprising the recombinant polypeptide of claim 1 or the enzyme composition of claims 4-6; and
a. Optionally at least one enzyme selected from protease, amylase, phytase, xylanase, endoglucanase, beta-glucanase, or a combination thereof; and
b. Optionally at least one filler selected from maltodextrin, flour, salt, sodium chloride, sulfate, sodium sulfate or a combination thereof.

12. A use of the animal feed of claim 10 or the feed supplement of claim 11 in:
a. feeding animals, preferably monogastric animals or ruminants; and/or
b. improving weight gain of animals.

13. A use of the recombinant polypeptide of claim 1 or the enzyme composition of claims 4-6 in a detergent.

14. The use of claim 13, wherein the detergent is a liquid detergent or a dry detergent, preferably in a form of a powder, bar, tablet, pouch, paste, gel, liquid, granule or granulate.

15. A use of the recombinant polypeptide of claim 1 or the enzyme composition of claims 4-6 in oil drilling.

16. A use of the recombinant polypeptide of claim 1 or the enzyme composition of claims 4-6 in processing coffee extract, fruit juice, pineapple juice, or soya milk.

## Patentansprüche

1. Rekombinantes Polypeptid mit Mannanaseaktivität und umfassend eine Aminosäuresequenz, die aufweist:
wenigstens 92 % Sequenzidentität mit Man13 (SEQ ID NO: 30).

2. Rekombinante Wirtszelle, umfassend genetische Elemente, die Produktion wenigstens eines rekombinanten Polypeptids mit Mannanaseaktivität ermöglichen, die wenigstens 92 % Sequenzidentität mit Man13 (SEQ ID NO: 30) aufweist und wobei die Wirtszelle ausgewählt ist aus einer Gruppe gebildet aus
Pilzzellen,
filamentösen Pilzzellen aus Abteilung Ascomycota, Unterabteilung Pezizomycotina;
bevorzugt aus der Gruppe gebildet aus Mitgliedern der Klasse Sordariomycetes, Unterklasse Hypocreomycetidae, Ordnungen Hypocreales und Microascales und Aspergillus, Chrysosporium, Myceliophthora und Humicola;
besonders bevorzugt aus der Gruppe gebildet aus Familien Hypocreacea, Nectriaceae, Clavicipitaceae, Microascaceae, und Gattungen Trichoderma (Anamorph von Hypocrea), Fusarium, Gibberella, Nectria, Stachybotrys, Claviceps, Metarhizium, Villosiclava, Ophiocordyceps, Cephalosporium und Scedosporium;
besonders bevorzugt aus der Gruppe gebildet aus Trichoderma reesei (Hypocrea jecorina), T. citrinoviridae, T. longibrachiatum, T. virens, T. harzianum, T. asperellum, T. atroviridae, T. parareesei, Fusarium oxysporum, F. gramineanum, F. pseudograminearum, F. venenatum, Gibberella fujikuroi, G. moniliformis, G. zeaea, Nectria (Haematonectria) haematococca, Stachybotrys chartarum, S. chlorohalonata, Claviceps purpurea, Metarhizium acridum, M. anisopliae, Villosiclava virens, Ophiocordyceps sinensis, Acremonium (Cephalosporium) chrysogenum und Scedosporium apiospermum und Aspergillus niger, Aspergillus awamori, Aspergillus oryzae, Chrysosporium lucknowense, Myceliophthora thermophila, Humicola insolens und Humicola grisea,
Bakterienzellen, bevorzugt grampositive Bacilli wie z.B. B. subtilis, B. licheniformis, B. megaterium, B. amyloliquefaciens, B. pumilus, gramnegative Bakterien wie z.B. Escherichia coli, Actinomycetales wie z.B. Streptomyces sp. und
Hefen wie z.B. Saccharomyces cerevisiae, Pichia pastoris, Yarrowia lipolytica,
am bevorzugtesten Trichoderma reesei oder Bacillus.

3. Rekombinante Wirtszelle nach Anspruch 2, wobei das rekombinante Polypeptid ein Fusionsprotein ist, das zusätzlich zu der Aminosäuresequenz mit Mannanaseaktivität wenigstens eines der folgenden umfasst:
eine Aminosäuresequenz, die eine sekretorische Signalsequenz bereitstellt;
eine Aminosäuresequenz, die Reinigung erleichtert, wie z.B. ein Affinitäts-Tag oder His-Tag;
eine Aminosäuresequenz, die Produktion steigert, wie z.B. eine Aminosäuresequenz, die ein Träger oder CBM ist;
eine Aminosäuresequenz mit einer Enzymaktivität; und
eine Aminosäuresequenz, die das Fusionsprotein mit Bindungsaffinität vorsieht, wie z.B. eine kohlenhydratbindende Einheit.

4. Enzymzusammensetzung, umfassend ein rekombinantes Polypeptid mit Mannanaseaktivität und wenigstens 92 % Sequenzidentität mit Man13 (SEQ ID NO: 30); und optional wenigstens eine weitere Mannanase mit
wenigstens 70 % Sequenzidentität mit Man8 (SEQ ID NO: 15); oder
wenigstens 93 % Sequenzidentität mit Man9 (SEQ ID NO: 18); oder
wenigstens 70 % Sequenzidentität mit Man10 (SEQ ID NO: 21); oder
wenigstens 80 % Sequenzidentität mit Man29 (SEQ ID NO: 39); oder
wenigstens 89 % Sequenzidentität mit Man30 (SEQ ID NO: 42); oder
100 % Sequenzidentität mit Man1 (SEQ ID NO: 3); oder
100 % Sequenzidentität mit Man2 (SEQ ID NO: 6); oder
100 % Sequenzidentität mit Man3 (SEQ ID NO: 9); oder
100 % Sequenzidentität mit Man5 (SEQ ID NO: 12); oder
100 % Sequenzidentität mit Man11 (SEQ ID NO: 24); oder
100 % Sequenzidentität mit Man12 (SEQ ID NO: 27); oder
100 % Sequenzidentität mit Man17 (SEQ ID NO: 33); oder
100 % Sequenzidentität mit Man27 (SEQ ID NO: 36).

5. Enzymzusammensetzung nach Anspruch 4, ferner umfassend:
a. wenigstens ein Konservierungsmittel, ausgewählt aus Benzoesäure, Natriumbenzoat, Hydroxybenzoat, Zitronensäure, Ascorbinsäure oder einer Kombination davon;
b. optional wenigstens ein Polyol ausgewählt aus Propylenglycol, Glycerin, einem Zucker, Zuckeralkohol, Milchsäure, Borsäure, Borsäurederivat, aromatischem Boratester, Phenylboronsäurederivat, Peptid oder einer Kombination davon;
c. optional wenigstens ein Enzym ausgewählt aus Proteasen, Amylasen, Cellulasen, Lipasen, Xylanasen, Mannanasen, Cutinasen, Esterasen, Phytasen, DNAsen, Pektinasen, pektinolytischen Enzymen, Pektatlyasen, Carbohydrasen, Arabinasen, Galactanasen, Xanthanasen, Laccasen, Peroxidasen und Oxidasen mit oder ohne einen Mediator oder eine Kombination davon; und
d. optional wenigstens einen Füllstoff ausgewählt aus Maltodextrin, Mehl, Natriumchlorid, Sulfat, Natriumsulfat oder einer Kombination davon.

6. Enzymzusammensetzung nach Ansprüchen 4-5 in der Form einer flüssigen Zusammensetzung oder einer festen Zusammensetzung wie z.B. Lösung, Dispersion, Paste, Pulver, Granulat, beschichtetes Granulat, Tablette, Kuchen, Kristall, Kristallaufschlämmung, Gel oder Pellet.

7. Verfahren zur Herstellung von Mannanase, umfassend
a. Kultivieren der rekombinanten Wirtszelle nach Ansprüchen 2-3, wobei
i. die genetischen Elemente wenigstens eine Kontrollsequenz umfassen, die die Produktion des rekombinanten Polypeptids in der rekombinanten Wirtszelle unter Bedingungen steuert, die Produktion des Polypeptids ermöglichen;
ii. die genetischen Elemente optional wenigstens eine Sequenz umfassen, die eine Signalsequenz zum Transportieren des Polypeptids außerhalb der Wirtszelle kodiert; und
iii. die Kultivierung unter Bedingungen durchgeführt wird, die Produktion des Polypeptids ermöglichen; und
b. Gewinnung der Mannanase.

8. Verfahren zum Abbau oder Modifizieren von Mannan enthaltendem Material, umfassend Behandeln des Mannan enthaltenden Materials mit einer wirksamen Menge des rekombinanten Polypeptids nach Anspruch 1 oder der Enzymzusammensetzung nach Ansprüchen 4-6.

9. Verfahren nach Anspruch 8, wobei das Mannan enthaltende Material auf Pflanzen basierendes Material, Textil, Abwasser, Schmutzwasser, Öl oder eine Kombination davon ist.

10. Tierfutter, umfassend das rekombinante Polypeptid nach Anspruch 1 oder die Enzymzusammensetzung nach Ansprüchen 4-6 und wenigstens eine Proteinquelle pflanzlichen Ursprungs oder ein Mannan enthaltendes Produkt oder Nebenprodukt, und
a. optional wenigstens ein Enzym ausgewählt aus Protease, Amylase, Phytase, Xylanase, Endoglucanase, Beta-Glucanase oder einer Kombination davon; und
b. optional wenigstens einen Füllstoff, ausgewählt aus Maltodextrin, Mehl, Salz, Natriumchlorid, Sulfat, Natriumsulfat oder einer Kombination davon.

11. Nahrungsergänzungsmittel, umfassend das rekombinante Polypeptid nach Anspruch 1 oder die Enzymzusammensetzung nach Ansprüchen 4-6; und
a. optional wenigstens ein Enzym ausgewählt aus Protease, Amylase, Phytase, Xylanase, Endoglucanase, Beta-Glucanase oder einer Kombination davon; und
b. optional wenigstens einen Füllstoff, ausgewählt aus Maltodextrin, Mehl, Salz, Natriumchlorid, Sulfat, Natriumsulfat oder einer Kombination davon.

12. Verwendung des Tierfutters nach Anspruch 10 oder des Nahrungsergänzungsmittels nach Anspruch 11 bei:
a. Fütterung von Tieren, bevorzugt monogastrischen Tieren oder Wiederkäuern; und / oder
b. Verbesserung der Gewichtszunahme von Tieren.

13. Verwendung des rekombinanten Polypeptids nach Anspruch 1 oder der Enzymzusammensetzung nach Ansprüchen 4-6 in einem Detergens.

14. Verwendung nach Anspruch 13, wobei das Detergens ein flüssiges Detergens oder ein trockenes Detergens ist, bevorzugt in einer Form eines Pulvers, eines Riegels, einer Tablette, eines Beutels, einer Paste, eines Gels, einer Flüssigkeit, eines Körnchens oder eines Granulats.

15. Verwendung des rekombinanten Polypeptids nach Anspruch 1 oder der Enzymzusammensetzung nach Ansprüchen 4-6 beim Ölbohren.

16. Verwendung des rekombinanten Polypeptids nach Anspruch 1 oder der Enzymzusammensetzung nach Ansprüchen 4-6 bei Verarbeitung von Kaffeeextrakt, Fruchtsaft, Ananassaft oder Sojamilch.

## Revendications

1. Un polypeptide recombinant ayant une activité mannanase et comprenant une séquence d'acides aminés, qui a :
au moins 92 % d'identité de séquence avec Man13 (SEQ ID NO : 30).

2. Une cellule hôte recombinante comprenant des éléments génétiques qui permettent de produire au moins un polypeptide recombinant à activité mannanase, qui a
au moins 92% d'identité de séquence avec Man13 (SEQ ID NO : 30) et
la cellule hôte étant choisie dans un groupe constitué
des cellules fongiques,
des cellules fongiques filamenteuses de la division *Ascomycota*, de la subdivision *Pezizomycotina* ; de préférence du groupe constitué des membres de la classe *Sordariomycetes*, sous-classe des *Hypocreomycetidae*, ordres des *Hypocreales* et *Microascales* et *Aspergillus*, *Chrysosporium*, *Myceliophthora* et *Humicola* ;
de façon encore préférée du groupe constitué des familles *Hypocreacea*, *Nectriaceae*, *Clavicipitaceae*, *Microascaceae* et Genre *Trichoderma* (anamorphe de *Hypocrea*), *Fusarium*, *Gibberella*, *Nectria*, *Stachybotrys*, *Claviceos*, *Metarhizium*, *Villosiclava*, *Ophiocordyceps*, *Cephalosporium* et *Scedosporium ;*
de façon encore préférée du groupe constitué de *Trichoderma reesei (Hypocrea jecorina), T. citrinoviridae, T. longibrachiatum*, *T. virens, T. harzianum, T. asperellum, T. atroviridae, T. parareesei, Fusarium oxysporum, F. gramineanum, F pseudograminearum, F. venenatum, Gibberella fujikuroi*, *G. moniliformis*, *G. zeaea*, *Nectria (Haematonectria) haematococca, Stachybotrys chartarum, S. chlorohalonata, Claviceps purpurea*, *Metarhizium acridum, M. anisopliae, Villosiclava virens, Ophiocordyceps sinensis, Acremonium (Cephalosporium) chrysogenum*, et *Scedosporium apiospermum*, et *Aspergillus niger*, *Aspergillus awamori*, *Aspergillus oryzae*, *Chrysosporium lucknowense, Myceliophthora thermophila*, *Humicola insolens*, et *Humicola grisea*,
des cellules bactériennes, de préférence des bacilles à Gram positif tels que *B*. *subtilis*, *B. licheniformis*, *B. Megaterium*, *B. Amyloliquefaciens*, *B. pumilus*, des bactéries à Gram négatif telles que *Escherichia coli*, des actinomycétales telles que *Streptomyces* sp., et
des levures telles que *Saccharomyces cerevisiae*, *Pichia pastoris*, *Yarrowia lipolytica,*
de façon la plus préférée *Trichoderma reesei* ou *Bacillus.*

3. La cellule hôte recombinante selon la revendication 2, dans laquelle le polypeptide recombinant est une protéine de fusion qui, en plus d'avoir la séquence d'acides aminés ayant une activité mannanase, comprend au moins une parmi :
une séquence d'acides aminés fournissant une séquence signal de sécrétion ;
une séquence d'acides aminés qui facilite la purification, telle qu'une étiquette d'affinité ou une étiquette His ;
une séquence d'acides aminés qui améliore la production, telle qu'une séquence d'acides aminés qui est un support ou CBM :
une séquence d'acides aminés ayant une activité enzymatique ; et
une séquence d'acides aminés fournissant la protéine de fusion avec une affinité de liaison, telle qu'un groupe de liaison glucidique.

4. Une composition enzymatique comprenant un polypeptide recombinant ayant une activité mannanase et au moins 92 % d'identité de séquence avec Man13 (SEQ ID NO : 30) : et
optionnellement, au moins une autre mannanase ayant
au moins 70 % d'identité de séquence avec Man8 (SEQ ID NO : 15) ; ou alors
au moins 93 % d'identité de séquence avec Man9 (SEQ ID NO : 18) : ou
au moins 70 % d'identité de séquence avec Man10 (SEQ ID NO : 21) ; ou alors
au moins 80 % d'identité de séquence avec Man29 (SEQ ID NO : 39) ; ou
au moins 89 % d'identité de séquence avec Man30 (SEQ ID NO : 42) : ou
100 % d'identité de séquence avec Man1 (SEQ ID NO : 3) : ou
100 % d'identité de séquence avec Man2 (SEQ ID NO : 6) : ou
100 % d'identité de séquence avec Man3 (SEQ ID NO : 9) : ou
100 % d'identité de séquence avec Man5 (SEQ ID NO : 12) : ou
100 % d'identité de séquence avec Man11 (SEQ ID NO : 24) : ou
100 % d'identité de séquence avec Man12 (SEQ ID NO : 27) ; ou
100 % d'identité de séquence avec Man17 (SEQ ID NO : 33) : ou
100 % d'identité de séquence avec Man27 (SEQ ID NO : 36).

5. La composition enzymatique selon la revendication 4 comprenant en outre :
a. au moins un conservateur choisi parmi l'acide benzoïque, le benzoate de sodium, l'hydroxybenzoate, l'acide citrique, l'acide ascorbique, ou une combinaison de ceux-ci ;
b. optionnellement, au moins un polyol choisi parmi le propylène glycol, le glycérol, un sucre, un alcool de sucre, l'acide lactique, l'acide borique, un dérivé d'acide borique, un ester de borate aromatique, un dérivé d'acide phénylboronique, un peptide, ou une combinaison de ceux-ci ;
c. optionnellement, au moins une enzyme choisie parmi les protéases, les amylases, les cellulases, les lipases, les xylanases, les mannanases, les cutinases, les estérases, les phytases, les ADNases, les pectinases, les enzymes pectinolytiques, les pectates lyases, les carbohydrases, les arabinases, les galactanases, les xanthanases, les laccases, les peroxydases et les oxydases avec ou sans médiateur, ou une combinaison de ceux-ci ; et
d. optionnellement, au moins une charge choisie parmi la maltodextrine, la farine, le chlorure de sodium, le sulfate, le sulfate de sodium, ou une combinaison de ceux-ci.

6. La composition enzymatique selon les revendications 4 à 5 sous la forme d'une composition liquide ou d'une composition solide telle qu'une solution, une dispersion, une pâte, une poudre, un granulé, un granulé enrobé, un comprimé, un gâteau, un cristal, une suspension cristalline, un gel ou une pastille.

7. Un procédé de production de mannanase comprenant
a. le fait de cultiver la cellule hôte recombinante selon les revendications 2 à 3, dans laquelle
i. les éléments génétiques comprennent au moins une séquence de contrôle qui contrôle la production du polypeptide recombinant dans la cellule hôte recombinante dans des conditions qui permettent la production du polypeptide ;
ii. les éléments génétiques comprennent optionnellement au moins une séquence codant pour une séquence signal de transport du polypeptide hors de la cellule hôte : et
iii. la culture est réalisée dans des conditions permettant la production du polypeptide ; et
b. le fait de récupérer la mannanase.

8. Un procédé pour dégrader ou modifier une matière contenant du mannane, comprenant le fait de traiter ladite matière contenant du mannane avec une quantité efficace du polypeptide recombinant selon la revendication 1 ou de la composition enzymatique selon les revendications 4 à 6.

9. Le procédé selon la revendication 8, dans lequel la matière contenant du mannane est une matière à base de plantes, du textile, des eaux usées, des eaux d'égout, de l'huile ou une combinaison de ceux-ci.

10. Un aliment pour animaux comprenant le polypeptide recombinant selon la revendication 1 ou la composition enzymatique selon les revendications 4 à 6 et au moins une source de protéines d'origine végétale ou un produit ou sous-produit contenant du mannane, et
a. optionnellement, au moins une enzyme choisie parmi la protéase, l'amylase, la phytase, la xylanase, l'endoglucanase, la bêta-glucanase, ou une combinaison de celles-ci ; et
b. optionnellement, au moins une charge choisie parmi la maltodextrine, la farine, le sel, le chlorure de sodium, le sulfate, le sulfate de sodium, ou une combinaison de ceux-ci.

11. Un complément alimentaire comprenant le polypeptide recombinant selon la revendication 1 ou la composition enzymatique selon les revendications 4 à 6 ; et
a. optionnellement, au moins une enzyme choisie parmi la protéase, l'amylase, la phytase, la xylanase, l'endoglucanase, la bêta-glucanase, ou une combinaison de celles-ci ; et
b. optionnellement, au moins une charge choisie parmi la maltodextrine, la farine, le sel, le chlorure de sodium, le sulfate, le sulfate de sodium ou une combinaison de ceux-ci.

12. Une utilisation de l'aliment pour animaux selon la revendication 10 ou du complément alimentaire selon la revendication 11 pour :
a. nourrir des animaux, de préférence des animaux monogastriques ou des ruminants ; et/ou
b. améliorer la prise de poids des animaux.

13. Une utilisation du polypeptide recombinant selon la revendication 1 ou de la composition enzymatique selon les revendications 4 à 6 dans un détergent.

14. L'utilisation selon la revendication 13, dans laquelle le détergent est un détergent liquide ou un détergent sec, de préférence sous forme de poudre, barre, tablette, sachet, pâte, gel, liquide, granule ou granulé.

15. Une utilisation du polypeptide recombinant selon la revendication 1 ou de la composition enzymatique selon les revendications 4 à 6 dans le forage pétrolier.

16. Une utilisation du polypeptide recombinant selon la revendication 1 ou de la composition enzymatique selon les revendications 4 à 6 dans le traitement d'extrait de café, de jus de fruit, de jus d'ananas ou de lait de soja.


### SEQUENCE LISTING

<110> AB Enzymes Oy
<120> Fungal mannanase
<130> 31470_ALLMAN-F
<160> 56
<170> PatentIn version 3.5
<210> 1
   <211> 1686
   <212> DNA
   <213> Malbranchea cinnamomea
<400> 1
   atgcacctca cgaagcacct gactctggcc ctcctgacgg gcgctgcggt tgcggctcct      60
   gggcgcggtc atcacggcaa gcctcccaag ggcttcgtca cgaccacagg cacggagttt     120
   cggcttgatg gcaagaagtt ttactttgca ggcagcaatg cctattactt cccgttcaac     180
   aacgtacggt cgctcctcgg tcttgtccag tagttcatgt gtggtggaca tactactaca     240
   tattgtacac acacacaaat agtacgtgct atctgtgcat tatgctgact gactgcacct     300
   agaaccaaac ggacgtcgag ctcggactcg cggcagccaa aagggccggg ttgagtgtct     360
   tccgcacctg gggtttccac gaccgaaacg tgacgacgat tccggggggc ctcccgcagt     420
   atggaggaga aggtgcgggc gagacggaag tggtcttcca gtggtgggac aatggaaagc     480
   cgaccatcaa cctggagccc tttgacaagg tcgtcgctgc ggccgagaag attggcatca     540
   agctgatcgt ggccctgacg aacaactggg ccgactacgg tggcatggat gtgtacacag     600
   tgaatctggg cgggcagtac catgacgatg tgcgtccttg acgacctgat atttttgttg     660
   acaacacctg gcaaggggaa gggggggggg ccaatggagt ggatttattc cgatactaaa     720
   taggacaggc acatgctgac ctgcttgctg cagttctacc gcctccccca gatcaaagcc     780
   gcatacaaga actacgtcaa ggagttcgtg acgcgctacc gcgactcccc cgtcatcatg     840
   gcctgggaac tcggcaacga gccacgctgc ggcgccgacg gcgtccgcaa cctgcctcgc     900
   agcgacagct gcgacccgca cctgctcacc agctggatcg acgagatgag cacgtacatc     960
   aagagcctcg acccgcacca cctcgtgacc tggggcggcg aaggcgggtt caacagggcc    1020
   tcggacgact gggcctacaa cggttccgac gggggagatt tcgacgcaga gcttgccttg    1080
   gacaatatcg actttggcgt gttccactcg tacccggact ggtggaccaa gaccgtcgag    1140
   tggaccgacc agtggatcag ggaccatgcg gagagcatga ggaaggccgg caagccggtc    1200
   gtccacgagg aatatggtat tcatgcatcc ctgtcccttg cgctcgagcc gcatttttta    1260
   tttttgttat ttccctctaa aaaaaaaaac ctggacatgg gaaaactttc taacgaaata    1320
   aaactgttta caaacaggct ggttgaccga tgaagcccgg ctcgaatacc tcggcaccgt    1380
   ctccaacatt accagactcg aggccgtcgg cgggtggcag acgatcagcg tcaaggagcg    1440
   catgtccgat atgttctggc aatggggcta ctctggatac tcctatggga gaaatcatga    1500
   cgacggcttc actatctatg ttgacgagta cgtcctcctt tttttttttt tcgtccttct    1560
   gatgcagacc tacgaggaac gctcccgtac taattggatc gatcattgcc gtgtagcccg    1620
   gaagcgaagc agttgatcta caagcatgcc aaggaggtca acaggctgaa ccgtggacgc    1680
   cattga                                                               1686
<210> 2
   <211> 1233
   <212> DNA
   <213> Malbranchea cinnamomea
<400> 2
   atgcacctca cgaagcacct gactctggcc ctcctgacgg gcgctgcggt tgcggctcct      60
   gggcgcggtc atcacggcaa gcctcccaag ggcttcgtca cgaccacagg cacggagttt     120
   cggcttgatg gcaagaagtt ttactttgca ggcagcaatg cctattactt cccgttcaac     180
   aacaaccaaa cggacgtcga gctcggactc gcggcagcca aaagggccgg gttgagtgtc     240
   ttccgcacct ggggtttcca cgaccgaaac gtgacgacga ttccgggggg cctcccgcag     300
   tatggaggag aaggtgcggg cgagacggaa gtggtcttcc agtggtggga caatggaaag     360
   ccgaccatca acctggagcc ctttgacaag gtcgtcgctg cggccgagaa gattggcatc     420
   aagctgatcg tggccctgac gaacaactgg gccgactacg gtggcatgga tgtgtacaca     480
   gtgaatctgg gcgggcagta ccatgacgat ttctaccgcc tcccccagat caaagccgca     540
   tacaagaact acgtcaagga gttcgtgacg cgctaccgcg actcccccgt catcatggcc     600
   tgggaactcg gcaacgagcc acgctgcggc gccgacggcg tccgcaacct gcctcgcagc     660
   gacagctgcg acccgcacct gctcaccagc tggatcgacg agatgagcac gtacatcaag     720
   agcctcgacc cgcaccacct cgtgacctgg ggcggcgaag gcgggttcaa cagggcctcg     780
   gacgactggg cctacaacgg ttccgacggg ggagatttcg acgcagagct tgccttggac     840
   aatatcgact ttggcgtgtt ccactcgtac ccggactggt ggaccaagac cgtcgagtgg     900
   accgaccagt ggatcaggga ccatgcggag agcatgagga aggccggcaa gccggtcgtc     960
   cacgaggaat atggctggtt gaccgatgaa gcccggctcg aatacctcgg caccgtctcc    1020
   aacattacca gactcgaggc cgtcggcggg tggcagacga tcagcgtcaa ggagcgcatg    1080
   tccgatatgt tctggcaatg gggctactct ggatactcct atgggagaaa tcatgacgac    1140
   ggcttcacta tctatgttga cgacccggaa gcgaagcagt tgatctacaa gcatgccaag    1200
   gaggtcaaca ggctgaaccg tggacgccat tga                                 1233
<210> 3
   <211> 410
   <212> PRT
   <213> Malbranchea cinnamomea
<400> 3
   Met His Leu Thr Lys His Leu Thr Leu Ala Leu Leu Thr Gly Ala Ala
   1               5                   10                  15
   Val Ala Ala Pro Gly Arg Gly His His Gly Lys Pro Pro Lys Gly Phe
               20                  25                  30
   Val Thr Thr Thr Gly Thr Glu Phe Arg Leu Asp Gly Lys Lys Phe Tyr
           35                  40                  45
   Phe Ala Gly Ser Asn Ala Tyr Tyr Phe Pro Phe Asn Asn Asn Gln Thr
       50                  55                  60
   Asp Val Glu Leu Gly Leu Ala Ala Ala Lys Arg Ala Gly Leu Ser Val
   65                  70                  75                  80
   Phe Arg Thr Trp Gly Phe His Asp Arg Asn Val Thr Thr Ile Pro Gly
                   85                  90                  95
   Gly Leu Pro Gln Tyr Gly Gly Glu Gly Ala Gly Glu Thr Glu Val Val
               100                 105                 110
   Phe Gln Trp Trp Asp Asn Gly Lys Pro Thr Ile Asn Leu Glu Pro Phe
           115                 120                 125
   Asp Lys Val Val Ala Ala Ala Glu Lys Ile Gly Ile Lys Leu Ile Val
       130                 135                 140
   Ala Leu Thr Asn Asn Trp Ala Asp Tyr Gly Gly Met Asp Val Tyr Thr
   145                 150                 155                 160
   Val Asn Leu Gly Gly Gln Tyr His Asp Asp Phe Tyr Arg Leu Pro Gln
                   165                 170                 175
   Ile Lys Ala Ala Tyr Lys Asn Tyr Val Lys Glu Phe Val Thr Arg Tyr
               180                 185                 190
   Arg Asp Ser Pro Val Ile Met Ala Trp Glu Leu Gly Asn Glu Pro Arg
           195                 200                 205
   Cys Gly Ala Asp Gly Val Arg Asn Leu Pro Arg Ser Asp Ser Cys Asp
       210                 215                 220
   Pro His Leu Leu Thr Ser Trp Ile Asp Glu Met Ser Thr Tyr Ile Lys
   225 230 235 240
   Ser Leu Asp Pro His His Leu Val Thr Trp Gly Gly Glu Gly Gly Phe
   245 250 255
   Asn Arg Ala Ser Asp Asp Trp Ala Tyr Asn Gly Ser Asp Gly Gly Asp
   260 265 270
   Phe Asp Ala Glu Leu Ala Leu Asp Asn Ile Asp Phe Gly Val Phe His
   275 280 285
   Ser Tyr Pro Asp Trp Trp Thr Lys Thr Val Glu Trp Thr Asp Gln Trp
   290 295 300
   Ile Arg Asp His Ala Glu Ser Met Arg Lys Ala Gly Lys Pro Val Val
   305 310 315 320
   His Glu Glu Tyr Gly Trp Leu Thr Asp Glu Ala Arg Leu Glu Tyr Leu
   325 330 335
   Gly Thr Val Ser Asn Ile Thr Arg Leu Glu Ala Val Gly Gly Trp Gln
   340 345 350
   Thr Ile Ser Val Lys Glu Arg Met Ser Asp Met Phe Trp Gln Trp Gly
   355 360 365
   Tyr Ser Gly Tyr Ser Tyr Gly Arg Asn His Asp Asp Gly Phe Thr Ile
   370 375 380
   Tyr Val Asp Asp Pro Glu Ala Lys Gln Leu Ile Tyr Lys His Ala Lys
   385 390 395 400
   Glu Val Asn Arg Leu Asn Arg Gly Arg His
   405 410
<210> 4
   <211> 1330
   <212> DNA
   <213> Verticillium dahliae
<400> 4
   atgcatccac gcatctcgac acttctctcg ctgggcatgg ccctcgcagg ccgtgaggtt 60
   gaggcgcagt ttgtctcggt gaacgggcag cacttcgcgg tcgacggcaa ggacttcatc 120
   ttcgccggca gcaacgccta ctactggcct tttagcaacg tacgtgacct gtcacttgtc 180
   gattctgtcc gggcagtggt gatgggcgac tgacatgggt tcagacacaa gcggacattg 240
   agctcggcat gaaggccgcc ctcgacgctg ggctcactgt cttccggacg tgggggttca 300
   acgaggccaa ctcgacatat gacccgaacg gccttccacg ctacggtcag caggacccgg 360
   caacggtgtt ccagacgtgg tcgcctggtg gaacggtcga ggtcaacctg gcaccgctgg 420
   acaaggtggt cgcggccgcg gaaaagacgg gcatcaagct catcgtgacg ctgacgaaca 480
   actgggccga ctacggcggc atggacgtgt acacaacgaa ccttggtttc cggtaccacg 540
   acgatgtgag tctgccgagc agtagggccg acatctggtg ccccgtgatg tgactgacat 600
   gtaacgccgc acacagttct accgtcagcc gaccatcaag gcggcgtaca agaagtatct 660
   ctccgaggtt gtcgggcggt acgccgactc gccagccatt ttcgcgtggg aggtcgccaa 720
   cgagttgcgg tgcgcggccg acgggacgcg gaacctgcct tcgagcggtg actgtacgcc 780
   ggaacttctg ctcgagtggg tggacgagat ctccacgcac atcaagagcg ttgacgcgaa 840
   ccaccttgtg gccacgggtg gcgagggagc cttcaatcgc cagtcggacg atcacttcta 900
   caacggcggc gatggcaacg actttgacgg cgaactcaaa ctgcggaaca ttgactttgg 960
   cgtcttccac tcgtaccccg actggtggag caagacggtc gaatggacaa accagtggat 1020
   tcgcgaccac gccgaggcgg gccgtacagc gggcaagccg gtggtgcatg aggaatacgg 1080
   ctggatgacg gacaaggcgc gtcaggagca gcttggaaag acggccgaca agggacggat 1140
   cgaggtgttg ggtgggtggc aggccatctc gattgaggag aagatatcgg acatgttctg 1200
   gcagtttggc ttctctggat attcatcggg acgtaaccac gatgacggat tcaccatctt 1260
   cttggatgat gaggaggcaa agccgctggt gtacgatcac gcaaaggcca tgaacggtgt 1320
   tcttgcatga 1330
<210> 5
   <211> 1194
   <212> DNA
   <213> Verticillium dahliae
<400> 5
   atgcatccac gcatctcgac acttctctcg ctgggcatgg ccctcgcagg ccgtgaggtt      60
   gaggcgcagt ttgtctcggt gaacgggcag cacttcgcgg tcgacggcaa ggacttcatc     120
   ttcgccggca gcaacgccta ctactggcct tttagcaaca cacaagcgga cattgagctc     180
   ggcatgaagg ccgccctcga cgctgggctc actgtcttcc ggacgtgggg gttcaacgag     240
   gccaactcga catatgaccc gaacggcctt ccacgctacg gtcagcagga cccggcaacg     300
   gtgttccaga cgtggtcgcc tggtggaacg gtcgaggtca acctggcacc gctggacaag     360
   gtggtcgcgg ccgcggaaaa gacgggcatc aagctcatcg tgacgctgac gaacaactgg     420
   gccgactacg gcggcatgga cgtgtacaca acgaaccttg gtttccggta ccacgacgat     480
   ttctaccgtc agccgaccat caaggcggcg tacaagaagt atctctccga ggttgtcggg     540
   cggtacgccg actcgccagc cattttcgcg tgggaggtcg ccaacgagtt gcggtgcgcg     600
   gccgacggga cgcggaacct gccttcgagc ggtgactgta cgccggaact tctgctcgag     660
   tgggtggacg agatctccac gcacatcaag agcgttgacg cgaaccacct tgtggccacg     720
   ggtggcgagg gagccttcaa tcgccagtcg gacgatcact tctacaacgg cggcgatggc     780
   aacgactttg acggcgaact caaactgcgg aacattgact ttggcgtctt ccactcgtac     840
   cccgactggt ggagcaagac ggtcgaatgg acaaaccagt ggattcgcga ccacgccgag     900
   gcgggccgta cagcgggcaa gccggtggtg catgaggaat acggctggat gacggacaag     960
   gcgcgtcagg agcagcttgg aaagacggcc gacaagggac ggatcgaggt gttgggtggg    1020
   tggcaggcca tctcgattga ggagaagata tcggacatgt tctggcagtt tggcttctct    1080
   ggatattcat cgggacgtaa ccacgatgac ggattcacca tcttcttgga tgatgaggag    1140
   gcaaagccgc tggtgtacga tcacgcaaag gccatgaacg gtgttcttgc atga          1194
<210> 6
   <211> 397
   <212> PRT
   <213> Verticillium dahliae
<400> 6
   Met His Pro Arg Ile Ser Thr Leu Leu Ser Leu Gly Met Ala Leu Ala
   1               5                   10                  15
   Gly Arg Glu Val Glu Ala Gln Phe Val Ser Val Asn Gly Gln His Phe
               20                  25                  30
   Ala Val Asp Gly Lys Asp Phe Ile Phe Ala Gly Ser Asn Ala Tyr Tyr
           35                  40                  45
   Trp Pro Phe Ser Asn Thr Gln Ala Asp Ile Glu Leu Gly Met Lys Ala
       50                  55                  60
   Ala Leu Asp Ala Gly Leu Thr Val Phe Arg Thr Trp Gly Phe Asn Glu
   65                  70                  75                  80
   Ala Asn Ser Thr Tyr Asp Pro Asn Gly Leu Pro Arg Tyr Gly Gln Gln
                   85                  90                  95
   Asp Pro Ala Thr Val Phe Gln Thr Trp Ser Pro Gly Gly Thr Val Glu
               100                 105                 110
   Val Asn Leu Ala Pro Leu Asp Lys Val Val Ala Ala Ala Glu Lys Thr
           115                 120                 125
   Gly Ile Lys Leu Ile Val Thr Leu Thr Asn Asn Trp Ala Asp Tyr Gly
       130                 135                 140
   Gly Met Asp Val Tyr Thr Thr Asn Leu Gly Phe Arg Tyr His Asp Asp
   145                 150                 155                 160
   Phe Tyr Arg Gln Pro Thr Ile Lys Ala Ala Tyr Lys Lys Tyr Leu Ser
                   165                 170                 175
   Glu Val Val Gly Arg Tyr Ala Asp Ser Pro Ala Ile Phe Ala Trp Glu
               180                 185                 190
   Val Ala Asn Glu Leu Arg Cys Ala Ala Asp Gly Thr Arg Asn Leu Pro
           195                 200                 205
   Ser Ser Gly Asp Cys Thr Pro Glu Leu Leu Leu Glu Trp Val Asp Glu
       210                 215                 220
   Ile Ser Thr His Ile Lys Ser Val Asp Ala Asn His Leu Val Ala Thr
   225                 230                 235                 240
   Gly Gly Glu Gly Ala Phe Asn Arg Gln Ser Asp Asp His Phe Tyr Asn
                   245                 250                 255
   Gly Gly Asp Gly Asn Asp Phe Asp Gly Glu Leu Lys Leu Arg Asn Ile
               260                 265                 270
   Asp Phe Gly Val Phe His Ser Tyr Pro Asp Trp Trp Ser Lys Thr Val
           275                 280                 285
   Glu Trp Thr Asn Gln Trp Ile Arg Asp His Ala Glu Ala Gly Arg Thr
       290                 295                 300
   Ala Gly Lys Pro Val Val His Glu Glu Tyr Gly Trp Met Thr Asp Lys
   305                 310                 315                 320
   Ala Arg Gln Glu Gln Leu Gly Lys Thr Ala Asp Lys Gly Arg Ile Glu
                   325                 330                 335
   Val Leu Gly Gly Trp Gln Ala Ile Ser Ile Glu Glu Lys Ile Ser Asp
               340                 345                 350
   Met Phe Trp Gln Phe Gly Phe Ser Gly Tyr Ser Ser Gly Arg Asn His
           355                 360                 365
   Asp Asp Gly Phe Thr Ile Phe Leu Asp Asp Glu Glu Ala Lys Pro Leu
       370                 375                 380
   Val Tyr Asp His Ala Lys Ala Met Asn Gly Val Leu Ala
   385 390 395
<210> 7
   <211> 1388
   <212> DNA
   <213> Verticillium dahliae
<400> 7
   atgcgtttcg acattctctc cgtcggtctg ctggccactg gcgccatggc gcgctgtgtg      60
   cccaagggtt tcgtcacgac caagggcacc aagttcaggt tggacggcaa ggacttcccc     120
   tttgccggca gcaatgctta ctacttcccc tttgacaacg ttagtttctc ctggtcctga     180
   agcgaggcgg aacagctgca gaacgagaga gaagcattca cactgacacg ctgctgcgct     240
   agaaccaagc tgatgtcgag gctggtctga ccgccgccaa ggacgccggg ctcaaggtgt     300
   tccgcacctg gggcttcaac gacaagaacg cgacctcgga tttccagaac ggcatgccca     360
   agtacggcgg cgagggcgcc ggcgccaccg aggtgattct gcaaaagtgg tacgacaacg     420
   gcacctccgt catcaacctc gagccctttg acaaggtcgt cgacgccgcc accaaggttg     480
   gcatcaagct gctcgtcgcc ttcaccaaca actgggccga ctacggtggc atggacgtgt     540
   acaccgtcaa cctcggcggc aagtaccacg acgatgtatg ttcctctccc ttatcccatg     600
   ttgggtccct ggccctcatc ccatgttggg cttggggatt tctcccattc ctcgactcgc     660
   actaacatgc gcagttctac cgcgtccccg cgataaagga cgcctacaag cgctacgtca     720
   aggcctttgt cgagcgctac aaggactcgc ccaccatctt cgcctgggag ctcatgaacg     780
   agccccgctg cggcgccgac ggcgtccgca acctcccccg cagccccgcc ggctgcaacc     840
   cggccgtcat gtccgcctgg accgacgaga tgtcgacctt tgtccgcagc atcgacccct     900
   accacctcat cacctggggc ggcgagggcg ccttcaaccg cgagtccgac gactgggcct     960
   acaacggcgc cgacggctcc gactttgacc acgagatgac cctcaagaac attgactttg    1020
   gcaccttcca cctctacccc gactggtgga gcaagacggt cgagtggtcg aaccagtgga    1080
   tcgtcgacca cgccgccgtc ggccgcgacg tcggcaagcc cgtcgtcttt gaggagtacg    1140
   gctggctcca gcccgacaag cgcctcgagt acctcggcca ggtccgcaac gagacgaggc    1200
   tcgaggtcat cagccactgg caggagctgt ccatcgaggg cggcatgccc gacatgtact    1260
   ggcagtacgg ctactgcggc tactcgtacg gctgcaacca caacgacggc ttcaccattt    1320
   acctcaacga caccgaggcc gacgtcctca tctaccagca cgctgccaag gtcaacggcg    1380
   agaactag                                                             1388
<210> 8
   <211> 1206
   <212> DNA
   <213> Verticillium dahliae
<400> 8
   atgcgtttcg acattctctc cgtcggtctg ctggccactg gcgccatggc gcgctgtgtg        60
   cccaagggtt tcgtcacgac caagggcacc aagttcaggt tggacggcaa ggacttcccc       120
   tttgccggca gcaatgctta ctacttcccc tttgacaaca accaagctga tgtcgaggct       180
   ggtctgaccg ccgccaagga cgccgggctc aaggtgttcc gcacctgggg cttcaacgac       240
   aagaacgcga cctcggattt ccagaacggc atgcccaagt acggcggcga gggcgccggc       300
   gccaccgagg tgattctgca aaagtggtac gacaacggca cctccgtcat caacctcgag       360
   ccctttgaca aggtcgtcga cgccgccacc aaggttggca tcaagctgct cgtcgccttc       420
   accaacaact gggccgacta cggtggcatg gacgtgtaca ccgtcaacct cggcggcaag       480
   taccacgacg atttctaccg cgtccccgcg ataaaggacg cctacaagcg ctacgtcaag       540
   gcctttgtcg agcgctacaa ggactcgccc accatcttcg cctgggagct catgaacgag       600
   ccccgctgcg gcgccgacgg cgtccgcaac ctcccccgca gccccgccgg ctgcaacccg       660
   gccgtcatgt ccgcctggac cgacgagatg tcgacctttg tccgcagcat cgacccctac       720
   cacctcatca cctggggcgg cgagggcgcc ttcaaccgcg agtccgacga ctgggcctac       780
   aacggcgccg acggctccga ctttgaccac gagatgaccc tcaagaacat tgactttggc       840
   accttccacc tctaccccga ctggtggagc aagacggtcg agtggtcgaa ccagtggatc       900
   gtcgaccacg ccgccgtcgg ccgcgacgtc ggcaagcccg tcgtctttga ggagtacggc       960
   tggctccagc ccgacaagcg cctcgagtac ctcggccagg tccgcaacga gacgaggctc      1020
   gaggtcatca gccactggca ggagctgtcc atcgagggcg gcatgcccga catgtactgg      1080
   cagtacggct actgcggcta ctcgtacggc tgcaaccaca acgacggctt caccatttac      1140
   ctcaacgaca ccgaggccga cgtcctcatc taccagcacg ctgccaaggt caacggcgag      1200
   aactag                                                                 1206
<210> 9
   <211> 401
   <212> PRT
   <213> Verticillium dahliae
<400> 9
   Met Arg Phe Asp Ile Leu Ser Val Gly Leu Leu Ala Thr Gly Ala Met
   1               5                   10                  15
   Ala Arg Cys Val Pro Lys Gly Phe Val Thr Thr Lys Gly Thr Lys Phe
               20                  25                  30
   Arg Leu Asp Gly Lys Asp Phe Pro Phe Ala Gly Ser Asn Ala Tyr Tyr
           35                  40                  45
   Phe Pro Phe Asp Asn Asn Gln Ala Asp Val Glu Ala Gly Leu Thr Ala
   50 55 60
   Ala Lys Asp Ala Gly Leu Lys Val Phe Arg Thr Trp Gly Phe Asn Asp
   65 70 75 80
   Lys Asn Ala Thr Ser Asp Phe Gln Asn Gly Met Pro Lys Tyr Gly Gly
   85 90 95
   Glu Gly Ala Gly Ala Thr Glu Val Ile Leu Gln Lys Trp Tyr Asp Asn
   100 105 110
   Gly Thr Ser Val Ile Asn Leu Glu Pro Phe Asp Lys Val Val Asp Ala
   115 120 125
   Ala Thr Lys Val Gly Ile Lys Leu Leu Val Ala Phe Thr Asn Asn Trp
   130 135 140
   Ala Asp Tyr Gly Gly Met Asp Val Tyr Thr Val Asn Leu Gly Gly Lys
   145 150 155 160
   Tyr His Asp Asp Phe Tyr Arg Val Pro Ala Ile Lys Asp Ala Tyr Lys
   165 170 175
   Arg Tyr Val Lys Ala Phe Val Glu Arg Tyr Lys Asp Ser Pro Thr Ile
   180 185 190
   Phe Ala Trp Glu Leu Met Asn Glu Pro Arg Cys Gly Ala Asp Gly Val
   195 200 205
   Arg Asn Leu Pro Arg Ser Pro Ala Gly Cys Asn Pro Ala Val Met Ser
   210 215 220
   Ala Trp Thr Asp Glu Met Ser Thr Phe Val Arg Ser Ile Asp Pro Tyr
   225 230 235 240
   His Leu Ile Thr Trp Gly Gly Glu Gly Ala Phe Asn Arg Glu Ser Asp
   245 250 255
   Asp Trp Ala Tyr Asn Gly Ala Asp Gly Ser Asp Phe Asp His Glu Met
   260 265 270
   Thr Leu Lys Asn Ile Asp Phe Gly Thr Phe His Leu Tyr Pro Asp Trp
   275 280 285
   Trp Ser Lys Thr Val Glu Trp Ser Asn Gln Trp Ile Val Asp His Ala
   290 295 300
   Ala Val Gly Arg Asp Val Gly Lys Pro Val Val Phe Glu Glu Tyr Gly
   305 310 315 320
   Trp Leu Gln Pro Asp Lys Arg Leu Glu Tyr Leu Gly Gln Val Arg Asn
   325 330 335
   Glu Thr Arg Leu Glu Val Ile Ser His Trp Gln Glu Leu Ser Ile Glu
   340 345 350
   Gly Gly Met Pro Asp Met Tyr Trp Gln Tyr Gly Tyr Cys Gly Tyr Ser
   355 360 365
   Tyr Gly Cys Asn His Asn Asp Gly Phe Thr Ile Tyr Leu Asn Asp Thr
   370 375 380
   Glu Ala Asp Val Leu Ile Tyr Gln His Ala Ala Lys Val Asn Gly Glu
   385 390 395 400
   Asn
<210> 10
   <211> 1397
   <212> DNA
   <213> Melanocarpus albomyces
<400> 10
   atgctgttct cggccccgca gcttgtcatt accagtctcc tggcctctac ggccgcagcc 60
   gctccaagcc gagctgtgca ggctcgacag aacgccgggt tcgtcacggt ggaggacggt 120
   aaattcaagc tcgacggcaa agacttccac ttcgctggaa gcaacgctta ctatttccct 180
   ttcaatgggg ttcgtgggaa ttgaggcatc ggtcgttttg tctgacatgg gtgctaacca 240
   tccgtacctg cttgcggctc actgtaatag ggacaagacg acgtcgagaa gggtttgatg 300
   gcggccaaga acgccggcct cacggttttc cgtacatggg gcttcaatga caagaacgtc 360
   acctacgtcc ctggcggctt gccgcaatat ggcggcgagg gagctggccc ttccgaggtc 420
   gtgttccagt ggtggcacga taacggcacc tctactattg acgtcacggg ctttgacaag 480
   gtcgtggatg ccgctagcaa ggtgggcatc aagttgatcg tggccttgac caacaactgg 540
   gccgactacg gcggcatgga tgtctacacg gtcaacctcg gggggcaata ccacgatgat 600
   gtaaggctgt cttgtccatc cgcctacaat cgttcgtgtc cacggtcatc gctaactccc 660
   gtcagttcta cacagtgccc aggatcaagg acgcattcaa gcgatatgtc aaagagatgg 720
   tgacccgcta caaggattct cccaccatct tcgcgtggga gctcgccaac gagcctcgct 780
   gtggtgctga cggtgttcgc aatctgccgc gtagcgacaa ctgcaacccg caggtcctcg 840
   gcgagtgggt ggccgagatg agccagtaca tcaagagcct cgaccccaac cacctggtga 900
   catggggcgg cgaaggcggc ttcaaccggg agtcggacga ctgggcctac aacggcagcg 960
   atggtggcga ctttgatcac gagatttccc tggacaccat cgactttggt gtattccatt 1020
   cctatcccga ctggtggagc aagacggtcg agtggacaga ccagtggatc cgggatcacg 1080
   cagaagccgg ccggaaagcc ggtaagcccg tggtccacga ggagtacggc tggctaacgc 1140
   ccgaagcccg gctggaatac gtcggcatcg tcgataaccg ctcaagggtc gaagtcatgg 1200
   gacaatggca aaggacgacg gtcgaggaga agctggctgg aagcatgtat tggcaatacg 1260
   gcttctccga ctactcatat ggtcgcaacc acgatgatgg cttcaccatc tacctggacg 1320
   accccgaggc cgaagtgcta gtataccagc atgctgtaga gatgcagagc ctcaatagcg 1380
   aggcgagatg tgactaa 1397
<210> 11
   <211> 1251
   <212> DNA
   <213> Melanocarpus albomyces
<400> 11
   atgctgttct cggccccgca gcttgtcatt accagtctcc tggcctctac ggccgcagcc 60
   gctccaagcc gagctgtgca ggctcgacag aacgccgggt tcgtcacggt ggaggacggt 120
   aaattcaagc tcgacggcaa agacttccac ttcgctggaa gcaacgctta ctatttccct 180
   ttcaatgggg gacaagacga cgtcgagaag ggtttgatgg cggccaagaa cgccggcctc 240
   acggttttcc gtacatgggg cttcaatgac aagaacgtca cctacgtccc tggcggcttg 300
   ccgcaatatg gcggcgaggg agctggccct tccgaggtcg tgttccagtg gtggcacgat 360
   aacggcacct ctactattga cgtcacgggc tttgacaagg tcgtggatgc cgctagcaag 420
   gtgggcatca agttgatcgt ggccttgacc aacaactggg ccgactacgg cggcatggat 480
   gtctacacgg tcaacctcgg ggggcaatac cacgatgatt tctacacagt gcccaggatc 540
   aaggacgcat tcaagcgata tgtcaaagag atggtgaccc gctacaagga ttctcccacc 600
   atcttcgcgt gggagctcgc caacgagcct cgctgtggtg ctgacggtgt tcgcaatctg 660
   ccgcgtagcg acaactgcaa cccgcaggtc ctcggcgagt gggtggccga gatgagccag 720
   tacatcaaga gcctcgaccc caaccacctg gtgacatggg gcggcgaagg cggcttcaac 780
   cgggagtcgg acgactgggc ctacaacggc agcgatggtg gcgactttga tcacgagatt 840
   tccctggaca ccatcgactt tggtgtattc cattcctatc ccgactggtg gagcaagacg 900
   gtcgagtgga cagaccagtg gatccgggat cacgcagaag ccggccggaa agccggtaag 960
   cccgtggtcc acgaggagta cggctggcta acgcccgaag cccggctgga atacgtcggc 1020
   atcgtcgata accgctcaag ggtcgaagtc atgggacaat ggcaaaggac gacggtcgag 1080
   gagaagctgg ctggaagcat gtattggcaa tacggcttct ccgactactc atatggtcgc 1140
   aaccacgatg atggcttcac catctacctg gacgaccccg aggccgaagt gctagtatac 1200
   cagcatgctg tagagatgca gagcctcaat agcgaggcga gatgtgacta a 1251
<210> 12
   <211> 416
   <212> PRT
   <213> Melanocarpus albomyces
<400> 12
   Met Leu Phe Ser Ala Pro Gln Leu Val Ile Thr Ser Leu Leu Ala Ser
   1               5                   10                  15
   Thr Ala Ala Ala Ala Pro Ser Arg Ala Val Gln Ala Arg Gln Asn Ala
               20                  25                  30
   Gly Phe Val Thr Val Glu Asp Gly Lys Phe Lys Leu Asp Gly Lys Asp
           35                  40                  45
   Phe His Phe Ala Gly Ser Asn Ala Tyr Tyr Phe Pro Phe Asn Gly Gly
       50                  55                  60
   Gln Asp Asp Val Glu Lys Gly Leu Met Ala Ala Lys Asn Ala Gly Leu
   65                  70                  75                  80
   Thr Val Phe Arg Thr Trp Gly Phe Asn Asp Lys Asn Val Thr Tyr Val
                   85                  90                  95
   Pro Gly Gly Leu Pro Gln Tyr Gly Gly Glu Gly Ala Gly Pro Ser Glu
               100                 105                 110
   Val Val Phe Gln Trp Trp His Asp Asn Gly Thr Ser Thr Ile Asp Val
           115                 120                 125
   Thr Gly Phe Asp Lys Val Val Asp Ala Ala Ser Lys Val Gly Ile Lys
       130                 135                 140
   Leu Ile Val Ala Leu Thr Asn Asn Trp Ala Asp Tyr Gly Gly Met Asp
   145                 150                 155                 160
   Val Tyr Thr Val Asn Leu Gly Gly Gln Tyr His Asp Asp Phe Tyr Thr
                   165                 170                 175
   Val Pro Arg Ile Lys Asp Ala Phe Lys Arg Tyr Val Lys Glu Met Val
               180                 185                 190
   Thr Arg Tyr Lys Asp Ser Pro Thr Ile Phe Ala Trp Glu Leu Ala Asn
               195                 200                 205
   Glu Pro Arg Cys Gly Ala Asp Gly Val Arg Asn Leu Pro Arg Ser Asp
       210                 215                 220
   Asn Cys Asn Pro Gln Val Leu Gly Glu Trp Val Ala Glu Met Ser Gln
   225                 230                 235                 240
   Tyr Ile Lys Ser Leu Asp Pro Asn His Leu Val Thr Trp Gly Gly Glu
                   245                 250                 255
   Gly Gly Phe Asn Arg Glu Ser Asp Asp Trp Ala Tyr Asn Gly Ser Asp
               260                 265                 270
   Gly Gly Asp Phe Asp His Glu Ile Ser Leu Asp Thr Ile Asp Phe Gly
           275                 280                 285
   Val Phe His Ser Tyr Pro Asp Trp Trp Ser Lys Thr Val Glu Trp Thr
       290                 295                 300
   Asp Gln Trp Ile Arg Asp His Ala Glu Ala Gly Arg Lys Ala Gly Lys
   305                 310                 315                 320
   Pro Val Val His Glu Glu Tyr Gly Trp Leu Thr Pro Glu Ala Arg Leu
                   325                 330                 335
   Glu Tyr Val Gly Ile Val Asp Asn Arg Ser Arg Val Glu Val Met Gly
               340                 345                 350
   Gln Trp Gln Arg Thr Thr Val Glu Glu Lys Leu Ala Gly Ser Met Tyr
           355                 360                 365
   Trp Gln Tyr Gly Phe Ser Asp Tyr Ser Tyr Gly Arg Asn His Asp Asp
       370                 375                 380
   Gly Phe Thr Ile Tyr Leu Asp Asp Pro Glu Ala Glu Val Leu Val Tyr
   385                 390                 395                 400
   Gln His Ala Val Glu Met Gln Ser Leu Asn Ser Glu Ala Arg Cys Asp
                   405                 410                 415
<210> 13
   <211> 1520
   <212> DNA
   <213> Acremonium alcalophilum
<400> 13
   atgatttcca agtctctcct cgcactcggc gcggccgcca tggccagcgt cgaggttgct        60
   gcgcaggctc cttctggctt cgcgacgatt gcaaacggtc agttccagtt ggacggcagt       120
   cccctcgtct ttgccggtag caatgcctac tactggccct tcaatgcggt atgtttctcc       180
   cctccccttt acgcagagtg gcatctccta accaggacgc gacagtgggg cacggatcac       240
   taccaggatg tcgtggtggg catggaggca gcgctcgatg ctggcctcaa ggtgatgcgc       300
   acctgggctt tccacgacca gaaccggacc accatctccg gcggactgcc gcagtacaac       360
   actggggcgg aggagacggt gatgcaattc ttcaaccccg atgggaccgt taccatcgac       420
   ctcagcgtgc tcgatgtggt gattcgggcg gccgaggaca cgggcatgaa gctcgtcctg       480
   gccctgacga ataactgggc cgattacggc ggcatggacg tgtataccgt gaacctcggc       540
   ggtcgttacc acgacgatgt aagtttttgt cccaggggga gatgtcaaaa caaacactct       600
   aacccctcca gttctaccgc atgccgacca tcaaggcggc gttcaaaaac tacatcaccc       660
   aagtcgtcgg acgctacaag gactcgccgg ccgtgttcgc gtgggaggtg gccaacgagg       720
   cccgctgcgg cgccgacgga acgcgcaacc tgccccgcgg cccggactgc acgccggagc       780
   tgctgatcga gtggctcgac gaaatgtcga cgcacatcaa gtccatcgac ccgcaccacc       840
   tcgtgaccac gggcagcgag ggccacttca accgcgactc gagcgactgg gcctacaacg       900
   gctccgacgg caacgacttt gacgccgagc tggccctgcc caacatcgac tttggcacct       960
   tccactccta cccggactgg tggagcaaga cggtcgagtg gaccgagcag tggatcgtcg      1020
   accacgccga cgccgcccgc gccctgggca agcccgtcgt ccacgaggag tacggctggc      1080
   tggacgtcgc cacccgcgtc gaggtgctgg gccgctggca ggccatttcc cgcgacgagc      1140
   tcatgcccga catgttctgg cagtttggct actccggcta ctccacgggc cggaaccaca      1200
   acgacggctt caccatctac ctcgacgagg ccgaggctca gccccttgtc taccagcacg      1260
   ccgccctggt caacgctctt ctcggcggcg gcggcagccc tcctcctacc tctccccctc       1320
   ccacttctcc tcctcccact tcgcctcccc ctatcactcc ccctcctatc actcctcctc      1380
   caatcacacc acctcctcct agccccggca aggcccagcg atggggccag tgcggaggcg      1440
   agaactggac cggccccgtg gaatgcgagg acccttgggt ctgcacccac cagaacccgt     1500
   ggtactcgca gtgtctgtaa                                                  1520
<210> 14
   <211> 1410
   <212> DNA
   <213> Acremonium alcalophilum
<400> 14
   atgatctcca agagcctcct ggccctcggc gctgccgcca tggctagcgt cgaggtcgct      60
   gctcaggctc cctccggctt cgccaccatc gccaacggcc agttccagct cgacggctcc     120
   cctctcgtct ttgctggcag caacgcctac tactggccgt tcaacgcctg gggcaccgac     180
   cactaccagg acgtcgtcgt cggcatggag gctgctctgg acgctggcct caaggtcatg     240
   cgcacctggg ccttccacga ccagaaccgc accaccatct ccggcggcct ccctcagtac     300
   aacacgggcg ccgaggagac ggtcatgcag ttcttcaacc ctgacggcac cgtcacgatc     360
   gacctgtcgg tcctggacgt cgtcatccgc gccgccgagg acacgggcat gaagctcgtc     420
   ctggccctca cgaacaactg ggccgactac ggtggcatgg acgtctacac cgtcaacctc     480
   ggcggccgat accacgacga cttctaccgc atgcctacca tcaaggccgc cttcaagaac     540
   tacatcacgc aggtcgtcgg ccgctacaag gactcgccgg ccgtctttgc ttgggaggtc     600
   gccaacgagg ctcgctgcgg cgctgacggc acccgcaacc tgcctcgcgg cccggactgc     660
   acgccggagc tcctgatcga gtggctcgac gagatgagca cccacatcaa gtcgatcgac     720
   cctcaccacc tggtcacgac cggcagcgag ggccacttca accgcgacag ctcggactgg     780
   gcctacaacg gctcggacgg caacgacttc gacgccgagc tggccctccc gaacatcgac     840
   ttcggcacct tccacagcta ccccgactgg tggtcgaaga ccgtcgagtg gacggagcag     900
   tggatcgtcg accacgctga cgctgctcgc gctctcggca agcccgtcgt ccacgaggag     960
   tacggctggc tggacgtcgc tacccgcgtc gaggtcctgg gccgctggca ggctatcagc    1020
   cgcgacgagc tcatgcctga catgttctgg cagttcggct actcgggcta ctccaccggc    1080
   cgcaaccaca acgacggctt cacgatctac ctcgacgagg ctgaggctca gcctctggtc    1140
   taccagcacg ccgccctcgt caacgctctc ctcggcggcg gcggcagccc tcctcccacc    1200
   agccctcctc ccacgtcgcc ccctccgacc agccctcctc ccatcacgcc ccctccgatc    1260
   acccctcctc ccattacgcc ccctccgccc agccctggca aggctcagcg ctggggccag    1320
   tgcggcggcg agaactggac gggccctgtc gagtgcgagg acccttgggt ctgcacgcac    1380
   cagaaccctt ggtactccca gtgcctgtaa                                     1410
<210> 15
   <211> 469
   <212> PRT
   <213> Acremonium alcalophilum
<400> 15
   Met Ile Ser Lys Ser Leu Leu Ala Leu Gly Ala Ala Ala Met Ala Ser
   1               5                   10                  15
   Val Glu Val Ala Ala Gln Ala Pro Ser Gly Phe Ala Thr Ile Ala Asn
               20                  25                  30
   Gly Gln Phe Gln Leu Asp Gly Ser Pro Leu Val Phe Ala Gly Ser Asn
           35                  40                  45
   Ala Tyr Tyr Trp Pro Phe Asn Ala Trp Gly Thr Asp His Tyr Gln Asp
       50                  55                  60
   Val Val Val Gly Met Glu Ala Ala Leu Asp Ala Gly Leu Lys Val Met
   65                  70                  75                  80
   Arg Thr Trp Ala Phe His Asp Gln Asn Arg Thr Thr Ile Ser Gly Gly
                   85                  90                  95
   Leu Pro Gln Tyr Asn Thr Gly Ala Glu Glu Thr Val Met Gln Phe Phe
               100                 105                 110
   Asn Pro Asp Gly Thr Val Thr Ile Asp Leu Ser Val Leu Asp Val Val
           115                 120                 125
   Ile Arg Ala Ala Glu Asp Thr Gly Met Lys Leu Val Leu Ala Leu Thr
       130                 135                 140
   Asn Asn Trp Ala Asp Tyr Gly Gly Met Asp Val Tyr Thr Val Asn Leu
   145                 150                 155                 160
   Gly Gly Arg Tyr His Asp Asp Phe Tyr Arg Met Pro Thr Ile Lys Ala
                   165                 170                 175
   Ala Phe Lys Asn Tyr Ile Thr Gln Val Val Gly Arg Tyr Lys Asp Ser
               180                 185                 190
   Pro Ala Val Phe Ala Trp Glu Val Ala Asn Glu Ala Arg Cys Gly Ala
           195                 200                 205
   Asp Gly Thr Arg Asn Leu Pro Arg Gly Pro Asp Cys Thr Pro Glu Leu
       210                 215                 220
   Leu Ile Glu Trp Leu Asp Glu Met Ser Thr His Ile Lys Ser Ile Asp
   225                 230                 235                 240
   Pro His His Leu Val Thr Thr Gly Ser Glu Gly His Phe Asn Arg Asp
                   245                 250                 255
   Ser Ser Asp Trp Ala Tyr Asn Gly Ser Asp Gly Asn Asp Phe Asp Ala
               260                 265                 270
   Glu Leu Ala Leu Pro Asn Ile Asp Phe Gly Thr Phe His Ser Tyr Pro
           275                 280                 285
   Asp Trp Trp Ser Lys Thr Val Glu Trp Thr Glu Gln Trp Ile Val Asp
       290                 295                 300
   His Ala Asp Ala Ala Arg Ala Leu Gly Lys Pro Val Val His Glu Glu
   305                 310                 315                 320
   Tyr Gly Trp Leu Asp Val Ala Thr Arg Val Glu Val Leu Gly Arg Trp
                   325                 330                 335
   Gln Ala Ile Ser Arg Asp Glu Leu Met Pro Asp Met Phe Trp Gln Phe
               340                 345                 350
   Gly Tyr Ser Gly Tyr Ser Thr Gly Arg Asn His Asn Asp Gly Phe Thr
           355                 360                 365
   Ile Tyr Leu Asp Glu Ala Glu Ala Gln Pro Leu Val Tyr Gln His Ala
       370                 375                 380
   Ala Leu Val Asn Ala Leu Leu Gly Gly Gly Gly Ser Pro Pro Pro Thr
   385                 390                 395                 400
   Ser Pro Pro Pro Thr Ser Pro Pro Pro Thr Ser Pro Pro Pro Ile Thr
                   405                 410                 415
   Pro Pro Pro Ile Thr Pro Pro Pro Ile Thr Pro Pro Pro Pro Ser Pro
               420                 425                 430
   Gly Lys Ala Gln Arg Trp Gly Gln Cys Gly Gly Glu Asn Trp Thr Gly
           435                 440                 445
   Pro Val Glu Cys Glu Asp Pro Trp Val Cys Thr His Gln Asn Pro Trp
       450                 455                 460
   Tyr Ser Gln Cys Leu
   465
<210> 16
   <211> 1438
   <212> DNA
   <213> Fusarium equiseti
<400> 16
   atgaagttct cctcaatcat cgccgcagct tttgccagct ccccagcact ggctgtccca      60
   gctggcttcg tcaccacgga cggcacaaag ttctctcttg acggaaaaga cttctttttc      120
   gcaggcacta acgcttactt ccttccgttc aacatcgtat gtgctctatc aaatatcaca     180
   ccacgctact aaccaactct cgcagtgggg aacagaccat tacaaggatg tcaagctcgg     240
   tcttgaagcc gccaaggatg ctggtctgaa ggttgtccgt acctgggcct tccatgacaa     300
   caacaggacg ttctcagagg gcggactgcc aaagtataac actggtgctg aggacactgt     360
   ttttcagtgg tttgagaagg atggaagtgt caagattgat ctgagcaagc ttgatgtagt     420
   tgttgaagct gccgaagcta ctaacatgaa gttgattctg gctctgacca acaactgggc     480
   tgattatggt ggtatggacg tgtataccgt caacctcggt ggtaaatacc acgacgatgt     540
   gagtccccca gctcatctta gcaatcttgt aactgaccaa gcacagttct accgtctccc     600
   tgctatcaag aaggccttca aaaactacat caagaccgtt gtcaaccggt acaaagattc     660
   ccccgctgtc atggcctggg aaattgccaa tgaaccccgc tgcggcgccg atggtgtccg     720
   aaacctccct cgcggccctg attgcactcc caagacaatc acttcctggg tcgacgagat     780
   gagcacctac atcaagtcca tcgacgcaga ccatctcgtc accaccggaa gtgaaggtgg     840
   cttcaaccgc aagtcagatg actggaccta caacggcgct gacggaaacg atttcgacgc     900
   cgagcttaag ctccccaaca tcgacttcaa cacgttccac tcctaccctc aatattggag     960
   caagaccgcc gattgggttg ttcagtggat caaggaccat gacgcagctg gcaagactgt    1020
   tggaaagcca gttatccatg aagaatacgg ctggcaagac aagtcaaccc gggtcgccac    1080
   catcagcaaa tggcagaagg cttctctgga cttggaaatg agtgacttgt actggcaatt    1140
   cggattcgcc ggcttctcgt atggcaagaa ccacgatgat ggaaacacga tttatctcga    1200
   agacgacgag gctcagcctt tggtatacga gcatgctgcc cgcgtcaacg ggggagaaac    1260
   cacgccaaag ccgacggcta ccactactgg atcaactcct cccaagccca ctgttactga    1320
   ggggcccaag gtcggtagat atgggcagtg tggtggttct tcttggactg gacctaccgc    1380
   ttgtgaatcc ccatacactt gcaaggctca gaaccagtgg tactctcagt gtctataa      1438
<210> 17
   <211> 1341
   <212> DNA
   <213> Fusarium equiseti
<400> 17
   atgaagttca gctcgatcat cgctgccgcc tttgcttcca gccctgctct ggctgtccct      60
   gccggctttg tcaccaccga cggcaccaag ttctccctgg acggcaagga cttcttcttc     120
   gccggcacga acgcctactt cctccctttc aacatctggg gcaccgacca ctacaaggac     180
   gtcaagctgg gcctggaggc tgctaaggac gctggcctga aggtcgtccg cacctgggcc     240
   ttccacgaca acaaccgcac cttctcggag ggcggcctcc ctaagtacaa caccggcgcc     300
   gaggacaccg tcttccagtg gttcgagaag gacggctccg tcaagatcga cctgtcgaag     360
   ctggacgtcg tcgtcgaggc cgccgaggcc acgaacatga agctcatcct ggccctcacc     420
   aacaactggg ccgactacgg tggcatggac gtctacacgg tcaacctggg cggcaagtac     480
   cacgacgact tctaccgcct ccccgccatc aagaaggcct tcaagaacta catcaagacc     540
   gtcgtcaacc gctacaagga ctcgcccgcc gtcatggctt gggagatcgc caacgagcct     600
   cgctgcggcg ctgacggcgt ccgcaacctg cctcgcggcc ctgactgcac ccctaagacg     660
   atcacctcgt gggtcgacga gatgagcacc tacatcaagt cgatcgacgc cgaccacctg     720
   gtcacgaccg gcagcgaggg cggcttcaac cgcaagtcgg acgactggac gtacaacggc     780
   gctgacggca acgactttga cgctgagctg aagctcccga acatcgactt caacacgttc     840
   cactcgtacc cccagtactg gtccaagacc gccgactggg tcgtccagtg gatcaaggac     900
   cacgacgctg ccggcaagac ggtcggcaag ccggtcatcc acgaggagta cggctggcag     960
   gacaagagca cccgcgtcgc caccatcagc aagtggcaga aggcctcgct ggacctcgag    1020
   atgtccgacc tgtactggca gttcggcttc gccggcttct cctacggcaa gaaccacgac    1080
   gacggcaaca ccatctacct ggaggacgac gaggctcagc ctctggtcta cgagcacgct    1140
   gctcgcgtca acggcggcga gacgacccct aagcccaccg ctaccaccac gggctcgacc    1200
   cctcccaagc ctaccgtcac ggagggccct aaggtcggcc gctacggcca gtgcggcggc    1260
   tcgtcgtgga ccggccctac ggtctgcgag tccccgtaca cctgcaaggc ccagaaccag    1320
   tggtacagcc agtgcctcta a                                              1341
<210> 18
   <211> 446
   <212> PRT
   <213> Fusarium equiseti
<400> 18
   Met Lys Phe Ser Ser Ile Ile Ala Ala Ala Phe Ala Ser Ser Pro Ala
   1               5                   10                  15
   Leu Ala Val Pro Ala Gly Phe Val Thr Thr Asp Gly Thr Lys Phe Ser
               20                  25                  30
   Leu Asp Gly Lys Asp Phe Phe Phe Ala Gly Thr Asn Ala Tyr Phe Leu
           35                  40                  45
   Pro Phe Asn Ile Trp Gly Thr Asp His Tyr Lys Asp Val Lys Leu Gly
       50                  55                  60
   Leu Glu Ala Ala Lys Asp Ala Gly Leu Lys Val Val Arg Thr Trp Ala
   65                  70                  75                  80
   Phe His Asp Asn Asn Arg Thr Phe Ser Glu Gly Gly Leu Pro Lys Tyr
                   85                  90                  95
   Asn Thr Gly Ala Glu Asp Thr Val Phe Gln Trp Phe Glu Lys Asp Gly
               100                 105                 110
   Ser Val Lys Ile Asp Leu Ser Lys Leu Asp Val Val Val Glu Ala Ala
           115                 120                 125
   Glu Ala Thr Asn Met Lys Leu Ile Leu Ala Leu Thr Asn Asn Trp Ala
   130 135 140
   Asp Tyr Gly Gly Met Asp Val Tyr Thr Val Asn Leu Gly Gly Lys Tyr
   145 150 155 160
   His Asp Asp Phe Tyr Arg Leu Pro Ala Ile Lys Lys Ala Phe Lys Asn
   165 170 175
   Tyr Ile Lys Thr Val Val Asn Arg Tyr Lys Asp Ser Pro Ala Val Met
   180 185 190
   Ala Trp Glu Ile Ala Asn Glu Pro Arg Cys Gly Ala Asp Gly Val Arg
   195 200 205
   Asn Leu Pro Arg Gly Pro Asp Cys Thr Pro Lys Thr Ile Thr Ser Trp
   210 215 220
   Val Asp Glu Met Ser Thr Tyr Ile Lys Ser Ile Asp Ala Asp His Leu
   225 230 235 240
   Val Thr Thr Gly Ser Glu Gly Gly Phe Asn Arg Lys Ser Asp Asp Trp
   245 250 255
   Thr Tyr Asn Gly Ala Asp Gly Asn Asp Phe Asp Ala Glu Leu Lys Leu
   260 265 270
   Pro Asn Ile Asp Phe Asn Thr Phe His Ser Tyr Pro Gln Tyr Trp Ser
   275 280 285
   Lys Thr Ala Asp Trp Val Val Gln Trp Ile Lys Asp His Asp Ala Ala
   290 295 300
   Gly Lys Thr Val Gly Lys Pro Val Ile His Glu Glu Tyr Gly Trp Gln
   305 310 315 320
   Asp Lys Ser Thr Arg Val Ala Thr Ile Ser Lys Trp Gln Lys Ala Ser
   325 330 335
   Leu Asp Leu Glu Met Ser Asp Leu Tyr Trp Gln Phe Gly Phe Ala Gly
   340 345 350
   Phe Ser Tyr Gly Lys Asn His Asp Asp Gly Asn Thr Ile Tyr Leu Glu
   355 360 365
   Asp Asp Glu Ala Gln Pro Leu Val Tyr Glu His Ala Ala Arg Val Asn
       370                 375                 380
   Gly Gly Glu Thr Thr Pro Lys Pro Thr Ala Thr Thr Thr Gly Ser Thr
   385                 390                 395                 400
   Pro Pro Lys Pro Thr Val Thr Glu Gly Pro Lys Val Gly Arg Tyr Gly
                   405                 410                 415
   Gln Cys Gly Gly Ser Ser Trp Thr Gly Pro Thr Val Cys Glu Ser Pro
               420                 425                 430
   Tyr Thr Cys Lys Ala Gln Asn Gln Trp Tyr Ser Gln Cys Leu
           435                 440                 445
<210> 19
   <211> 1539
   <212> DNA
   <213> Acremonium thermophilum
<400> 19
   atgttgggcc atcgcgtctt cgcgccgctg gcgctgctcg cacagcgggt gctgggcggc 60
   cccgtcgctc gtgacacgcc tgcggggttc gtgaccgtcg agggcgacaa gttccagctc 120
   gatggcaagg actttttctt tgcaggcacc aatgcctact acttcccgtt catgacggtg 180
   cgttcgacac cagtctcttc cctcctaccc tccatctacg actccaagct gacagcgcga 240
   cgcggcagag cgcagacgac gtggagaaag gcatgacggc ggcccgggag gccgggctca 300
   gggtgattcg gacgtggggc ttcaacgaga gaaacgtgac cagcgtcgcg ggcgggctgc 360
   ccgcctacgg caacgagggc gcgggcccca gcgacgccgt gttccagtac tggtatggca 420
   acggctcgac ggtcatcgat ctgacgccct tcgacaaggt ggtcagctcg gccgagaagg 480
   tgggcatcaa gctggtggtg gccctcacca acaactgggc cgactacggc ggcatggacg 540
   tctacacggt caatctgaac ggcacctacc acgacgacgt acgtgtgtcc tctgtttact 600
   atgtcgttca tcttttttat tctctattat ccttctttct tattgtcttt gatccttctt 660
   tttgattatt ttattttatt ttattttact tcattttact tcattttttt atttcatttt 720
   tatttcattt ttatttcatt tttatttttt cttttgcgac atacactgag ctgactcgag 780
   ctgccgtctc ttcagttcta cagactgccc cgcatcaagg acgccttcaa gcgctacgtc 840
   caggccgtcg tcacgcggtt caggcactcg cccgccatca tggcctggga gctggccaac 900
   gagccccgct gcggcgccga cggcgcgcgc aacctgccgc gcagcgacag ctgcacggcg 960
   gccgtgctgg acgcatggat cgacgagatg agccggtacg tcaagagcct ggacccgcac 1020
   cacctggtca cctggggcgg cgagggcgag ttcgaccgcg cgtcgtcgga cgactgggcc 1080
   tacagcggcg cggacggtgg cgacttcgag cgcgagctgg cgctgcccgc cgtcgacttc 1140
   ggcaccttcc actcgtaccc ggactggtgg ggcaagacgg tcgagtggac gagccagtgg 1200
   atccgcgacc acgccgaggc gggtcgcgcg gcgggcaagc ccgtcgtgca cgaggagtac 1260
   ggatggatga cgcccgaggc gcggctggcc aacctgggca cggtggccaa ccagacgcgg 1320
   gtcgaggtgc tgggccggtg gcagaacatc accctcgagg cgcggatgcc ggacatgtac 1380
   tggcagttcg gctactccca ctacagcatc ggccggaacg acgacgacgg cttcaccatc 1440
   tacctggacg acgcggaagc ccagccgctc gtgtacgagc acgccaaggc tgtcaatagg 1500
   ttgaacagcg catgtggcag ccgcagacga cggcactag 1539
<210> 20
   <211> 1251
   <212> DNA
   <213> Acremonium thermophilum
<400> 20
   atgctcggcc accgcgtctt cgcccctctg gccctcctgg ctcagcgcgt cctcggcggc      60
   cctgtcgccc gcgacacgcc tgccggcttt gtcaccgtcg agggcgacaa gttccagctg     120
   gacggcaagg acttcttctt cgccggcacc aacgcctact acttcccgtt catgacgtcg     180
   gccgacgacg tcgagaaggg catgaccgcc gcccgcgagg ctggcctgcg cgtcatccgc     240
   acctggggct tcaacgagcg caacgtcacc tcggtcgccg gcggcctccc tgcctacggc     300
   aacgagggcg ccggccccag cgacgccgtc ttccagtact ggtacggcaa cggctcgacc     360
   gtcatcgacc tgacgccctt cgacaaggtc gtcagctcgg ccgagaaggt cggcatcaag     420
   ctcgtcgtcg ccctgaccaa caactgggcc gactacggtg gcatggacgt ctacaccgtc     480
   aacctgaacg gcacgtacca cgacgacttc taccgcctcc ctcgcatcaa ggacgccttc     540
   aagcgctacg tccaggctgt cgtcacccgc ttccgccaca gccctgccat catggcctgg     600
   gagctcgcca acgagcctcg ctgcggcgct gacggcgctc gcaacctccc tcgcagcgac     660
   tcctgcaccg ccgccgtcct cgacgcctgg atcgacgaga tgtcgcgcta cgtcaagtcc     720
   ctcgaccccc accacctggt cacctggggc ggcgagggcg agtttgaccg cgcttcctcg     780
   gacgactggg cctacagcgg cgctgacggc ggcgactttg agcgcgagct ggctctgcct     840
   gccgtcgact ttggcacctt ccactcctac ccggactggt ggggcaagac cgtcgagtgg     900
   acgagccagt ggattcgcga ccacgctgag gctggccgcg ctgccggcaa gcccgtcgtc     960
   cacgaggagt acggctggat gacccctgag gctcgcctgg ctaacctcgg cacggtcgct    1020
   aaccagaccc gcgtcgaggt cctgggccgc tggcagaaca tcacgctcga ggcccgcatg    1080
   ccggacatgt actggcagtt cggctacagc cactactcga tcggccgcaa cgacgacgac    1140
   ggcttcacca tctacctcga cgacgctgag gctcagcctc tcgtctacga gcacgccaag    1200
   gctgtcaacc gcctcaactc ggcttgcggc agccgccgcc gccgccacta a             1251
<210> 21
   <211> 416
   <212> PRT
   <213> Acremonium thermophilum
<400> 21
   Met Leu Gly His Arg Val Phe Ala Pro Leu Ala Leu Leu Ala Gln Arg
   1               5                   10                  15
   Val Leu Gly Gly Pro Val Ala Arg Asp Thr Pro Ala Gly Phe Val Thr
               20                  25                  30
   Val Glu Gly Asp Lys Phe Gln Leu Asp Gly Lys Asp Phe Phe Phe Ala
           35                  40                  45
   Gly Thr Asn Ala Tyr Tyr Phe Pro Phe Met Thr Ser Ala Asp Asp Val
       50                  55                  60
   Glu Lys Gly Met Thr Ala Ala Arg Glu Ala Gly Leu Arg Val Ile Arg
   65                  70                  75                  80
   Thr Trp Gly Phe Asn Glu Arg Asn Val Thr Ser Val Ala Gly Gly Leu
                   85                  90                  95
   Pro Ala Tyr Gly Asn Glu Gly Ala Gly Pro Ser Asp Ala Val Phe Gln
               100                 105                 110
   Tyr Trp Tyr Gly Asn Gly Ser Thr Val Ile Asp Leu Thr Pro Phe Asp
           115                 120                 125
   Lys Val Val Ser Ser Ala Glu Lys Val Gly Ile Lys Leu Val Val Ala
       130                 135                 140
   Leu Thr Asn Asn Trp Ala Asp Tyr Gly Gly Met Asp Val Tyr Thr Val
   145                 150                 155                 160
   Asn Leu Asn Gly Thr Tyr His Asp Asp Phe Tyr Arg Leu Pro Arg Ile
                   165                 170                 175
   Lys Asp Ala Phe Lys Arg Tyr Val Gln Ala Val Val Thr Arg Phe Arg
               180                 185                 190
   His Ser Pro Ala Ile Met Ala Trp Glu Leu Ala Asn Glu Pro Arg Cys
           195                 200                 205
   Gly Ala Asp Gly Ala Arg Asn Leu Pro Arg Ser Asp Ser Cys Thr Ala
       210                 215                 220
   Ala Val Leu Asp Ala Trp Ile Asp Glu Met Ser Arg Tyr Val Lys Ser
   225                 230                 235                 240
   Leu Asp Pro His His Leu Val Thr Trp Gly Gly Glu Gly Glu Phe Asp
                   245                 250                 255
   Arg Ala Ser Ser Asp Asp Trp Ala Tyr Ser Gly Ala Asp Gly Gly Asp
               260                 265                 270
   Phe Glu Arg Glu Leu Ala Leu Pro Ala Val Asp Phe Gly Thr Phe His
           275                 280                 285
   Ser Tyr Pro Asp Trp Trp Gly Lys Thr Val Glu Trp Thr Ser Gln Trp
       290                 295                 300
   Ile Arg Asp His Ala Glu Ala Gly Arg Ala Ala Gly Lys Pro Val Val
   305                 310                 315                 320
   His Glu Glu Tyr Gly Trp Met Thr Pro Glu Ala Arg Leu Ala Asn Leu
                   325                 330                 335
   Gly Thr Val Ala Asn Gln Thr Arg Val Glu Val Leu Gly Arg Trp Gln
               340                 345                 350
   Asn Ile Thr Leu Glu Ala Arg Met Pro Asp Met Tyr Trp Gln Phe Gly
           355                 360                 365
   Tyr Ser His Tyr Ser Ile Gly Arg Asn Asp Asp Asp Gly Phe Thr Ile
       370                 375                 380
   Tyr Leu Asp Asp Ala Glu Ala Gln Pro Leu Val Tyr Glu His Ala Lys
   385                 390                 395                 400
   Ala Val Asn Arg Leu Asn Ser Ala Cys Gly Ser Arg Arg Arg Arg His
                   405                 410                 415
<210> 22
   <211> 1586
   <212> DNA
   <213> Verticillium dahliae
<400> 22
   atgcgttcgt cccttctcgc tctggccctg gccctcgccg gccagcaggt cgcggcgcag      60
   gtgcctgccg gcttcgccac gaccaagggc aacaagttcc aggtcgacgg caaggatttc     120
   atcttcgccg gtagcaacgc ctactactgg cccttcaatg tggtatgcaa gcctccaagc     180
   tcgccttgat ccatggcttt gtactaacga gcatccctag tggggaaccg accactactc     240
   ggatgtcgtc gtcggcatgg acgctgctct cgatgctggc ctcaaggtca tccgaacctg 300
   ggccttccac gaccagaacg ccacgaccat ctctggcggc ttgccccgct acggcaccgg 360
   aggtgaggcg accatcttcc agaagttcaa cgccgatgga accgtcacca tcgaccttaa 420
   gctcctcgac attgtcgtca aggctgccga ggacaccggc atcaagctga tcctggccct 480
   caccaacaac tgggccgatt acggcggcat ggatgtctac accgtcaacc gtggcggccg 540
   ctaccacgac gatgtgagtg ctgccctcgg gctttatgtg gttccatact aattctctcc 600
   agttttaccg tctgcccgcc atcaagacgg ccttcaagaa ctacgtctcc accgtcgtca 660
   gccgctacaa gaactccccc gccatctttg cctgggaggt ggccaacgag gcccgctgcg 720
   gcgccgatgg cacccgcaac ctgccccgcg gcccggcctg cacgaccgac ctgatcatcg 780
   actggctcga cgagatgtcg acgtacatca agtccattga cgccaaccat ctcgtgacca 840
   ccggtaccga gggccacttc aaccgcgcct cgtccgactg ggcgtacaac ggctccgacg 900
   gcaacgactt tgacgccgag ctcgagctgc ccaacgtcga ctttggcacc ttccactcgt 960
   accccgactg gtggtccaag acgcccgcct ggaccgacac gtggatcgtc gaccacgccg 1020
   ttgccgcccg cgccgccggc aagcccgtca tccacgagga gtacggctgg ctcacggcct 1080
   cgaagcgcca ggagtacctc ggccagacga gcccctttgg ccgcgtcgag gtcctctcgc 1140
   gctggcagga catttccctg cgcgaggaga tgcccgacct ctactggcag tttggctttg 1200
   gcggctactc gtacggcaag aaccacgacg acggcttcac catctacctc gaggacgccg 1260
   aggcccagcc cctcgtcttc cagcacgccg cccgcgtcaa cgccctctac ggcagccccc 1320
   agcccaccac caccgtcgcc ccgcccgcgc ccacgaccac gagcaggccc gccactacga 1380
   ccacgtcccg gcccgccacc accagcacgt cccggcccgc caccaccacc agcaccgtcg 1440
   ccccgcccac caccaccacg gcccccgccg gcccgaccca ggtccgctgg ggccagtgcg 1500
   gcggccaggg ctggaccggc cccaaggtct gcgctgcgcc gtacacgtgc caggtgcaga 1560
   acgcttggta ctcgcagtgc ctctaa 1586
<210> 23
   <211> 1479
   <212> DNA
   <213> Verticillium dahliae
<400> 23
   atgcgcagca gcctgctggc tctggccctc gctctggctg gccagcaggt cgccgcccag      60
   gtccctgccg gctttgctac caccaagggc aacaagttcc aggtcgacgg caaggacttc     120
   atcttcgccg gcagcaacgc ctactactgg ccgttcaacg tctggggcac cgaccactac     180
   tcggacgtcg tcgtcggcat ggacgctgct ctggacgctg gcctgaaggt catccgcacc     240
   tgggccttcc acgaccagaa cgctaccacc atcagcggcg gcctccctcg ctacggcacg     300
   ggcggcgagg ctaccatctt ccagaagttc aacgccgacg gcacggtcac catcgacctg     360
   aagctcctgg acatcgtcgt caaggccgcc gaggacacgg gcatcaagct gatcctcgcc     420
   ctgaccaaca actgggccga ctacggtggc atggacgtct acaccgtcaa ccgaggcggc     480
   cgataccacg acgacttcta ccgcctcccg gccatcaaga cggccttcaa gaactacgtc     540
   agcaccgtcg tctcgcgcta caagaactcg cctgctatct ttgcttggga ggtcgccaac     600
   gaggctcgct gcggcgctga cggcacccgc aacctccctc gcggccctgc ttgcaccacc     660
   gacctcatca tcgactggct ggacgagatg tccacgtaca tcaagagcat cgacgccaac     720
   cacctggtca cgaccggcac ggagggccac ttcaaccgcg cttcctcgga ctgggcttac     780
   aacggctcgg acggcaacga cttcgacgcc gagctcgagc tgcccaacgt cgacttcggc     840
   acgttccact cgtaccctga ctggtggtcc aagacgcctg cttggaccga cacctggatc     900
   gtcgaccacg ctgtcgctgc tcgcgctgcc ggcaagcctg tcatccacga ggagtacggc     960
   tggctcaccg ctagcaagcg ccaggagtac ctcggccaga cctccccttt tggccgcgtc    1020
   gaggtcctct cgcgctggca ggacatcagc ctccgcgagg agatgcctga cctgtactgg    1080
   cagttcggct tcggtggcta ctcctacggc aagaaccacg acgacggctt caccatctac    1140
   ctggaggacg ctgaggctca gcctctggtc ttccagcacg ctgctcgcgt caacgctctg    1200
   tacggcagcc ctcagcctac caccacggtc gcccctcccg ctcctacgac cacgtcccgc    1260
   ccggccacca cgaccacgag ccgccctgcc accacgtcga cctcccgccc ggctaccacg    1320
   acctcgaccg tcgcccctcc cacgaccacg accgcccctg ccggccccac ccaggtccgc    1380
   tggggccagt gcggtggcca gggctggacg ggccccaagg tctgcgccgc cccttacacc    1440
   tgccaggtcc agaacgcctg gtactcccag tgcctgtaa                           1479
<210> 24
   <211> 492
   <212> PRT
   <213> Verticillium dahliae
<400> 24
   Met Arg Ser Ser Leu Leu Ala Leu Ala Leu Ala Leu Ala Gly Gln Gln
   1 5 10 15
   Val Ala Ala Gln Val Pro Ala Gly Phe Ala Thr Thr Lys Gly Asn Lys
   20 25 30
   Phe Gln Val Asp Gly Lys Asp Phe Ile Phe Ala Gly Ser Asn Ala Tyr
   35 40 45
   Tyr Trp Pro Phe Asn Val Trp Gly Thr Asp His Tyr Ser Asp Val Val
   50 55 60
   Val Gly Met Asp Ala Ala Leu Asp Ala Gly Leu Lys Val Ile Arg Thr
   65                  70                  75                  80
   Trp Ala Phe His Asp Gln Asn Ala Thr Thr Ile Ser Gly Gly Leu Pro
                   85                  90                  95
   Arg Tyr Gly Thr Gly Gly Glu Ala Thr Ile Phe Gln Lys Phe Asn Ala
               100                 105                 110
   Asp Gly Thr Val Thr Ile Asp Leu Lys Leu Leu Asp Ile Val Val Lys
           115                 120                 125
   Ala Ala Glu Asp Thr Gly Ile Lys Leu Ile Leu Ala Leu Thr Asn Asn
       130                 135                 140
   Trp Ala Asp Tyr Gly Gly Met Asp Val Tyr Thr Val Asn Arg Gly Gly
   145                 150                 155                 160
   Arg Tyr His Asp Asp Phe Tyr Arg Leu Pro Ala Ile Lys Thr Ala Phe
                   165                 170                 175
   Lys Asn Tyr Val Ser Thr Val Val Ser Arg Tyr Lys Asn Ser Pro Ala
               180                 185                 190
   Ile Phe Ala Trp Glu Val Ala Asn Glu Ala Arg Cys Gly Ala Asp Gly
           195                 200                 205
   Thr Arg Asn Leu Pro Arg Gly Pro Ala Cys Thr Thr Asp Leu Ile Ile
       210                 215                 220
   Asp Trp Leu Asp Glu Met Ser Thr Tyr Ile Lys Ser Ile Asp Ala Asn
   225                 230                 235                 240
   His Leu Val Thr Thr Gly Thr Glu Gly His Phe Asn Arg Ala Ser Ser
                   245                 250                 255
   Asp Trp Ala Tyr Asn Gly Ser Asp Gly Asn Asp Phe Asp Ala Glu Leu
               260                 265                 270
   Glu Leu Pro Asn Val Asp Phe Gly Thr Phe His Ser Tyr Pro Asp Trp
           275                 280                 285
   Trp Ser Lys Thr Pro Ala Trp Thr Asp Thr Trp Ile Val Asp His Ala
       290                 295                 300
   Val Ala Ala Arg Ala Ala Gly Lys Pro Val Ile His Glu Glu Tyr Gly
   305                 310                 315                 320
   Trp Leu Thr Ala Ser Lys Arg Gln Glu Tyr Leu Gly Gln Thr Ser Pro
   325 330 335
   Phe Gly Arg Val Glu Val Leu Ser Arg Trp Gln Asp Ile Ser Leu Arg
   340 345 350
   Glu Glu Met Pro Asp Leu Tyr Trp Gln Phe Gly Phe Gly Gly Tyr Ser
   355 360 365
   Tyr Gly Lys Asn His Asp Asp Gly Phe Thr Ile Tyr Leu Glu Asp Ala
   370 375 380
   Glu Ala Gln Pro Leu Val Phe Gln His Ala Ala Arg Val Asn Ala Leu
   385 390 395 400
   Tyr Gly Ser Pro Gln Pro Thr Thr Thr Val Ala Pro Pro Ala Pro Thr
   405 410 415
   Thr Thr Ser Arg Pro Ala Thr Thr Thr Thr Ser Arg Pro Ala Thr Thr
   420 425 430
   Ser Thr Ser Arg Pro Ala Thr Thr Thr Ser Thr Val Ala Pro Pro Thr
   435 440 445
   Thr Thr Thr Ala Pro Ala Gly Pro Thr Gln Val Arg Trp Gly Gln Cys
   450 455 460
   Gly Gly Gln Gly Trp Thr Gly Pro Lys Val Cys Ala Ala Pro Tyr Thr
   465 470 475 480
   Cys Gln Val Gln Asn Ala Trp Tyr Ser Gln Cys Leu
   485 490
<210> 25
   <211> 1275
   <212> DNA
   <213> Malbranchea cinnamomea
<400> 25
   atgaagttat catccttcgc tttacccctg ttagcagggc taagttcttc agctcccttg        60
   gaatcgcggc agagtggctt gtctcccttc gcaggtacca atgcctactg gcttccgttc       120
   ctcacaaatg acgcagacgt ggaggccagc ttccaggcgt gagtcaactt tactactcac       180
   accccaggta acaatcatta aattgagata ctaaatgttg aactgacata gtatgaaaaa       240
   cgccggtatg aaagtggttc gaacctgggc gttcaacgac aatactgagt gccaggaaat       300
   ccattttcag tgctggaaaa atggacaacc tacaatcaac accggagaaa acgggctgca       360
   acgtcttgat gtaattgttc gaaccgcaga agcccatgga atacagttga ttctgccatt       420
   tggtattgtt tcctctcaat attgttccaa tcctaccctt gctaataata atatgtattc       480
   gacagtaaac aactggggtg attatggcgg gatggatgtc tatgtccaac agcttggtgg       540
   caacggtcat tcttcctttt ataccgatgc tgcaattcag gatgcgtata agaactatgt       600
   cagaactatt gtgaaccgct acaaaaattc gtccgccatt ttcgcttggg aacttgccaa       660
   tgagccacgc tgccaaggct gcgacacgtc catcatcacg gaatgggcgt cgaacatgag       720
   cgcattcgtt aaatctctcg acccatcgca ctatgttgtc cttggtgacg agggtttttt       780
   caatcgacct ggtgacccgt cgtaccctta ccaaggcggc gaaggtgttg acttcgaggc       840
   caatctgaag atcagcacac tagacttcgg gatcttccac atgtacatta ccccctgggg       900
   acagacgtac gattggggaa atcagtggat cgccgaccac tctgcggctt gcgaggctgc       960
   tggaaagcca tgcatcttag aagagtatgg agtggatcag gatggtgact ttagaaccac      1020
   ttggatgaca aattggcaca acaccttgct ggagagcccc ggtgtcccag ccgatatgtt      1080
   ttggcagttt ggtttacagt tgagttatgg gcccaaccat gatgatgggg taggttcaac      1140
   gccgttgttt cgcgccgtta ctgttcaact aaggaaacct tgatgctaat aatacaatag      1200
   tacaccattt acaatttcga ggacaacttt cagccggtcg ttgtcgattg ggccgctact      1260
   cgaaacgcaa gctag                                                       1275
<210> 26
   <211> 1068
   <212> DNA
   <213> Malbranchea cinnamomea
<400> 26
   atgaagttat catccttcgc tttacccctg ttagcagggc taagttcttc agctcccttg        60
   gaatcgcggc agagtggctt gtctcccttc gcaggtacca atgcctactg gcttccgttc        120
   ctcacaaatg acgcagacgt ggaggccagc ttccaggcta tgaaaaacgc cggtatgaaa        180
   gtggttcgaa cctgggcgtt caacgacaat actgagtgcc aggaaatcca ttttcagtgc        240
   tggaaaaatg gacaacctac aatcaacacc ggagaaaacg ggctgcaacg tcttgatgta        300
   attgttcgaa ccgcagaagc ccatggaata cagttgattc tgccatttgt aaacaactgg        360
   ggtgattatg gcgggatgga tgtctatgtc caacagcttg gtggcaacgg tcattcttcc        420
   ttttataccg atgctgcaat tcaggatgcg tataagaact atgtcagaac tattgtgaac        480
   cgctacaaaa attcgtccgc cattttcgct tgggaacttg ccaatgagcc acgctgccaa        540
   ggctgcgaca cgtccatcat cacggaatgg gcgtcgaaca tgagcgcatt cgttaaatct        600
   ctcgacccat cgcactatgt tgtccttggt gacgagggtt ttttcaatcg acctggtgac        660
   ccgtcgtacc cttaccaagg cggcgaaggt gttgacttcg aggccaatct gaagatcagc        720
   acactagact tcgggatctt ccacatgtac attaccccct ggggacagac gtacgattgg        780
   ggaaatcagt ggatcgccga ccactctgcg gcttgcgagg ctgctggaaa gccatgcatc        840
   ttagaagagt atggagtgga tcaggatggt gactttagaa ccacttggat gacaaattgg        900
   cacaacacct tgctggagag ccccggtgtc ccagccgata tgttttggca gtttggttta        960
   cagttgagtt atgggcccaa ccatgatgat gggtacacca tttacaattt cgaggacaac       1020
   tttcagccgg tcgttgtcga ttgggccgct actcgaaacg caagctag                    1068
<210> 27
   <211> 355
   <212> PRT
   <213> Malbranchea cinnamomea
<400> 27
   Met Lys Leu Ser Ser Phe Ala Leu Pro Leu Leu Ala Gly Leu Ser Ser
   1               5                   10                  15
   Ser Ala Pro Leu Glu Ser Arg Gln Ser Gly Leu Ser Pro Phe Ala Gly
               20                  25                  30
   Thr Asn Ala Tyr Trp Leu Pro Phe Leu Thr Asn Asp Ala Asp Val Glu
           35                  40                  45
   Ala Ser Phe Gln Ala Met Lys Asn Ala Gly Met Lys Val Val Arg Thr
       50                  55                  60
   Trp Ala Phe Asn Asp Asn Thr Glu Cys Gln Glu Ile His Phe Gln Cys
   65                  70                  75                  80
   Trp Lys Asn Gly Gln Pro Thr Ile Asn Thr Gly Glu Asn Gly Leu Gln
                   85                  90                  95
   Arg Leu Asp Val Ile Val Arg Thr Ala Glu Ala His Gly Ile Gln Leu
               100                 105                 110
   Ile Leu Pro Phe Val Asn Asn Trp Gly Asp Tyr Gly Gly Met Asp Val
           115                 120                 125
   Tyr Val Gln Gln Leu Gly Gly Asn Gly His Ser Ser Phe Tyr Thr Asp
       130                 135                 140
   Ala Ala Ile Gln Asp Ala Tyr Lys Asn Tyr Val Arg Thr Ile Val Asn
   145                 150                 155                 160
   Arg Tyr Lys Asn Ser Ser Ala Ile Phe Ala Trp Glu Leu Ala Asn Glu
                   165                 170                 175
   Pro Arg Cys Gln Gly Cys Asp Thr Ser Ile Ile Thr Glu Trp Ala Ser
   180 185 190
   Asn Met Ser Ala Phe Val Lys Ser Leu Asp Pro Ser His Tyr Val Val
   195 200 205
   Leu Gly Asp Glu Gly Phe Phe Asn Arg Pro Gly Asp Pro Ser Tyr Pro
   210 215 220
   Tyr Gln Gly Gly Glu Gly Val Asp Phe Glu Ala Asn Leu Lys Ile Ser
   225 230 235 240
   Thr Leu Asp Phe Gly Ile Phe His Met Tyr Ile Thr Pro Trp Gly Gln
   245 250 255
   Thr Tyr Asp Trp Gly Asn Gln Trp Ile Ala Asp His Ser Ala Ala Cys
   260 265 270
   Glu Ala Ala Gly Lys Pro Cys Ile Leu Glu Glu Tyr Gly Val Asp Gln
   275 280 285
   Asp Gly Asp Phe Arg Thr Thr Trp Met Thr Asn Trp His Asn Thr Leu
   290 295 300
   Leu Glu Ser Pro Gly Val Pro Ala Asp Met Phe Trp Gln Phe Gly Leu
   305 310 315 320
   Gln Leu Ser Tyr Gly Pro Asn His Asp Asp Gly Tyr Thr Ile Tyr Asn
   325 330 335
   Phe Glu Asp Asn Phe Gln Pro Val Val Val Asp Trp Ala Ala Thr Arg
   340 345 350
   Asn Ala Ser
   355
<210> 28
   <211> 1192
   <212> DNA
   <213> Aspergillus nishimurae
<400> 28
   atgaagttct ccttcctcac tgttgccagc gtcctgtcgg ccctctcggc ccaggtgctg 60
   gccgccccca aacccagcgc aaaattcgcc agcacgtcgg ggctgcaatt ctccatcgac 120
   ggtagaacag gctactttgc aggctccaac tcctactgga tcggcttcct gaccaacaat 180
   gccgacgtgg acctggtctt cgaccacatg aagcaatccg ggctcaagat cctgcgcgtc 240
   tggggattca acgacgtcaa cgcggtcccc ggcgcgggaa cggtgtacta ccagctccac 300
   gtcaacggaa cctcgacaat caacacgggc ccggatgggc tgcagcggct ggactatgtc 360
   gtgtcctcgg cggagaagcg cggcatcaag ctcatcatca actttgtcaa caactgggat 420
   gattacggtg ggatgaatgc atatgtcaag gcgtacggcg agacggacca taatgcgttc 480
   tattcaaaca aggacatcca ggaagcgtat aggcggtata tcaatgccgt ggtgtcgcgg 540
   tacatcaact cgcccgcggt gtttgcgtgg gagctggcca acgaaccccg gtgcaagggg 600
   tgtgatacgg acgtgctgta tcagtggatc aagtcaacga gtcaatacat caagtcgctg 660
   gacaagaggc acatggtctg cattggtgac ggtcggtccc tgcccttttg catatgacat 720
   ctcttcccta ctgacagagg actaacatgt agagggcttc gggctcgacc tcctctccga 780
   cggcagctac ccctacacct acgtcgaagg cagcaacttc acccgcaacc tcgccatccc 840
   caccatcgac tttggcacct tccacctgta cccggacagc tggggcacca cgcacgaatg 900
   gggcaacggg tgggtgcgct cgcacggcgc cgcgtgcaaa gccgccggta agccctgtct 960
   gttggaggag tacggcgtta cctccgacca ctgcgcggtc gagacgccgt ggcaggagac     1020
   ggcgttgaat acaacggggg tctcggggga tttgtactgg cagtatggcg atactctgag     1080
   cacggggctg tcgccgaacg atgggaatac gatctactac ggcacggagg acttcaagtg 1140
   tatagtgacg gaccatgtgg cggccatcaa cgcaaagcag gggtgggttt ga 1192
<210> 29
   <211> 1131
   <212> DNA
   <213> Aspergillus nishimurae
<400> 29
   atgaagttct cgttcctcac ggtcgcttcg gtcctctcgg ctctgagcgc tcaggtcctg      60
   gctgctccta agccttcggc taagtttgct tccacctcgg gcctgcagtt cagcatcgac     120
   ggccgcaccg gctactttgc tggctcgaac tcctactgga tcggcttcct cacgaacaac     180
   gccgacgtcg acctggtctt cgaccacatg aagcagtccg gcctcaagat cctgcgcgtc     240
   tggggcttca acgacgtcaa cgctgtccct ggcgctggca ccgtctacta ccagctgcac     300
   gtcaacggta cgtcgaccat caacaccggc cctgacggcc tccagcgcct ggactacgtc     360
   gtcagctcgg ccgagaagcg cggcatcaag ctcatcatca acttcgtcaa caactgggac     420
   gactacggtg gcatgaacgc ctacgtcaag gcctacggcg agacggacca caacgccttc     480
   tacagcaaca aggacatcca agaggcctac cgccgataca tcaacgccgt cgtcagccgc     540
   tacatcaaca gcccggccgt ctttgcttgg gagctggcta acgagcctcg ctgcaagggc     600
   tgcgacaccg acgtcctgta ccagtggatc aagagcacgt cgcagtacat caagtcgctc     660
   gacaagcgcc acatggtctg catcggcgac gagggctttg gcctcgacct cctgtcggac     720
   ggcagctacc cttacaccta cgtcgagggc tccaacttca cgcgcaacct cgccatccct     780
   accatcgact tcggcacgtt ccacctgtac cctgacagct ggggcaccac ccacgagtgg     840
   ggcaacggct gggtccgctc ccacggcgct gcttgcaagg ctgccggcaa gccttgcctc     900
   ctggaggagt acggcgtcac gtcggaccac tgcgctgtcg agacgccttg gcaggagacg     960
   gctctcaaca cgaccggcgt ctcgggcgac ctgtactggc agtacggcga cacgctgagc    1020
   accggcctgt cccctaacga cggcaacacc atctactacg gcacggagga cttcaagtgc    1080
   atcgtcaccg accacgtcgc cgccatcaac gccaagcagg gctgggtcta g             1131
<210> 30
   <211> 376
   <212> PRT
   <213> Aspergillus nishimurae
<400> 30
   Met Lys Phe Ser Phe Leu Thr Val Ala Ser Val Leu Ser Ala Leu Ser
   1               5                   10                  15
   Ala Gln Val Leu Ala Ala Pro Lys Pro Ser Ala Lys Phe Ala Ser Thr
               20                  25                  30
   Ser Gly Leu Gln Phe Ser Ile Asp Gly Arg Thr Gly Tyr Phe Ala Gly
           35                  40                  45
   Ser Asn Ser Tyr Trp Ile Gly Phe Leu Thr Asn Asn Ala Asp Val Asp
       50                  55                  60
   Leu Val Phe Asp His Met Lys Gln Ser Gly Leu Lys Ile Leu Arg Val
   65                  70                  75                  80
   Trp Gly Phe Asn Asp Val Asn Ala Val Pro Gly Ala Gly Thr Val Tyr
                   85                  90                  95
   Tyr Gln Leu His Val Asn Gly Thr Ser Thr Ile Asn Thr Gly Pro Asp
               100                 105                 110
   Gly Leu Gln Arg Leu Asp Tyr Val Val Ser Ser Ala Glu Lys Arg Gly
           115                 120                 125
   Ile Lys Leu Ile Ile Asn Phe Val Asn Asn Trp Asp Asp Tyr Gly Gly
       130                 135                 140
   Met Asn Ala Tyr Val Lys Ala Tyr Gly Glu Thr Asp His Asn Ala Phe
   145                 150                 155                 160
   Tyr Ser Asn Lys Asp Ile Gln Glu Ala Tyr Arg Arg Tyr Ile Asn Ala
                   165                 170                 175
   Val Val Ser Arg Tyr Ile Asn Ser Pro Ala Val Phe Ala Trp Glu Leu
               180                 185                 190
   Ala Asn Glu Pro Arg Cys Lys Gly Cys Asp Thr Asp Val Leu Tyr Gln
           195                 200                 205
   Trp Ile Lys Ser Thr Ser Gln Tyr Ile Lys Ser Leu Asp Lys Arg His
       210                 215                 220
   Met Val Cys Ile Gly Asp Glu Gly Phe Gly Leu Asp Leu Leu Ser Asp
   225                 230                 235                 240
   Gly Ser Tyr Pro Tyr Thr Tyr Val Glu Gly Ser Asn Phe Thr Arg Asn
                   245                 250                 255
   Leu Ala Ile Pro Thr Ile Asp Phe Gly Thr Phe His Leu Tyr Pro Asp
               260                 265                 270
   Ser Trp Gly Thr Thr His Glu Trp Gly Asn Gly Trp Val Arg Ser His
           275                 280                 285
   Gly Ala Ala Cys Lys Ala Ala Gly Lys Pro Cys Leu Leu Glu Glu Tyr
       290                 295                 300
   Gly Val Thr Ser Asp His Cys Ala Val Glu Thr Pro Trp Gln Glu Thr
   305                 310                 315                 320
   Ala Leu Asn Thr Thr Gly Val Ser Gly Asp Leu Tyr Trp Gln Tyr Gly
                   325                 330                 335
   Asp Thr Leu Ser Thr Gly Leu Ser Pro Asn Asp Gly Asn Thr Ile Tyr
               340                 345                 350
   Tyr Gly Thr Glu Asp Phe Lys Cys Ile Val Thr Asp His Val Ala Ala
           355                 360                 365
   Ile Asn Ala Lys Gln Gly Trp Val
       370                 375
<210> 31
   <211> 1761
   <212> DNA
   <213> Melanocarpus albomyces
<400> 31
   atgggcgtca ggtcgctccg atccatcttc tgcagcgtcg tggctcttac tgcaggggca 60
   cacgcggccc cgtatctgaa gagcagccgt gcgagcagcg attctgtgac cttggaggcc 120
   gaggatgcag tgctcagcgg gacacacgtt gagacggagc accctggttt caccggtacg 180
   gccattctcc atctgcgact aggcggcacc caggactcag cgctaactgc cgtcgcaggg 240
   accggctacg tcacgggctt cgacgaggcg actgacaagg tcaccttcca cgtcgacagc 300
   gatgccacca agctgtacga cctgagcatc cgcgtcgccg ccatctacgg cgagaagcgg 360
   accaccgtcg tcctcaacgg cggcgcgaac agcgaggtgt acttcccggc cagcgacacc 420
   tttgacgaca tcgccgccgg ccaggtcctg ctggaggagg gcgccaacac gatcgacatc 480
   gtgagcaact ggggatggtg cgtcgacgat ggttgcgtca cgttcccgct tctccgaaag 540
   ccgggctgct aactctgccc gtctaggtac ctcatcgact ccatcacgct gaccccctcg 600
   ccgccgcggc cgccgcacaa catcaacgag gcgcccgtca acgcggccgc cgacgcgaac 660
   gcccgggcgc tgtacgacta cctccgctcc atctacggca agaagatcct gtcgggccag 720
   caggagctct cgtacgcgga ctggatcgcg cagcagacgg gcaagacgcc ggcgctggtg 780
   tcggtcgacc tgatggacta ctcgccgtcg cgggtggagc ggggcacggt gggcacggcg 840
   gtggaggagg cgatcgcgca ccacgagcgg ggcggcatcg tgtcggtgct gtggcactgg 900
   aacgcgccgg cggggctgta cgacacggac gagaacccgt ggtggagcgg cttctacacg 960
   cgggcgaccg acttcgacgt ggaggcggcg ctgagctcgc cggccaacgc caactacacg 1020
   ctgctggtgc gcgacatcga cgccatcgcc gagcagctca agcggctgcg cgacgccggc 1080
   gtgcccgtcc tcttccgccc gctgcacgag gccgagggcg gctggttctg gtggggcgcg 1140
   aagggccccg agccggccaa gaagctgtgg gcgctgctct acgaccgcct gaccaaccac 1200
   caccagatcc acaacctgct ctgggtctgg aactcgatcc tgcccgagtg gtatccgggc 1260
   gacgacaccg tcgacattct cagcgccgac gtctacgccc agggcaacgg ggtgagtcct 1320
   ctccgtcttc ttcttcttct tcttctttgc tcgtgcccat tctgtcgccg gagatcactg 1380
   gctaacccgg ttcgctttcc accaccacca ccagcccatg tcgacgcagt acaaccagct 1440
   catcgagctg ggccaggaca agaagatgat cgccgcgacc gaggtgggcg cggcgccgct 1500
   gccggacctg ctgcaggcct acgaggccca ctggctgtgg ttcgccgtct ggggcgacac 1560
   cttcatcaac aaccccgact ggaactcgcc agagacgctg cgcacggtac gtctatggtc 1620
   tattttttta aacctccgtg acatggaaaa agcagggacc gtcgaggtgt cgttggctga 1680
   caaaaaatga cgctgctgca gatctaccac catgattacg tcctcacgct cgatgagatt 1740
   cagggctggc ggggcaactg a 1761
<210> 32
   <211> 1431
   <212> DNA
   <213> Melanocarpus albomyces
<400> 32
   atgggcgtca ggtcgctccg atccatcttc tgcagcgtcg tggctcttac tgcaggggca      60
   cacgcggccc cgtatctgaa gagcagccgt gcgagcagcg attctgtgac cttggaggcc     120
   gaggatgcag tgctcagcgg gacacacgtt gagacggagc accctggttt caccgggacc     180
   ggctacgtca cgggcttcga cgaggcgact gacaaggtca ccttccacgt cgacagcgat     240
   gccaccaagc tgtacgacct gagcatccgc gtcgccgcca tctacggcga gaagcggacc     300
   accgtcgtcc tcaacggcgg cgcgaacagc gaggtgtact tcccggccag cgacaccttt     360
   gacgacatcg ccgccggcca ggtcctgctg gaggagggcg ccaacacgat cgacatcgtg     420
   agcaactggg gatggtacct catcgactcc atcacgctga ccccctcgcc gccgcggccg     480
   ccgcacaaca tcaacgaggc gcccgtcaac gcggccgccg acgcgaacgc ccgggcgctg     540
   tacgactacc tccgctccat ctacggcaag aagatcctgt cgggccagca ggagctctcg     600
   tacgcggact ggatcgcgca gcagacgggc aagacgccgg cgctggtgtc ggtcgacctg     660
   atggactact cgccgtcgcg ggtggagcgg ggcacggtgg gcacggcggt ggaggaggcg     720
   atcgcgcacc acgagcgggg cggcatcgtg tcggtgctgt ggcactggaa cgcgccggcg     780
   gggctgtacg acacggacga gaacccgtgg tggagcggct tctacacgcg ggcgaccgac     840
   ttcgacgtgg aggcggcgct gagctcgccg gccaacgcca actacacgct gctggtgcgc     900
   gacatcgacg ccatcgccga gcagctcaag cggctgcgcg acgccggcgt gcccgtcctc     960
   ttccgcccgc tgcacgaggc cgagggcggc tggttctggt ggggcgcgaa gggccccgag    1020
   ccggccaaga agctgtgggc gctgctctac gaccgcctga ccaaccacca ccagatccac    1080
   aacctgctct gggtctggaa ctcgatcctg cccgagtggt atccgggcga cgacaccgtc    1140
   gacattctca gcgccgacgt ctacgcccag ggcaacgggc ccatgtcgac gcagtacaac    1200
   cagctcatcg agctgggcca ggacaagaag atgatcgccg cgaccgaggt gggcgcggcg    1260
   ccgctgccgg acctgctgca ggcctacgag gcccactggc tgtggttcgc cgtctggggc    1320
   gacaccttca tcaacaaccc cgactggaac tcgccagaga cgctgcgcac gatctaccac    1380
   catgattacg tcctcacgct cgatgagatt cagggctggc ggggcaactg a             1431
<210> 33
   <211> 476
   <212> PRT
   <213> Melanocarpus albomyces
<400> 33
   Met Gly Val Arg Ser Leu Arg Ser Ile Phe Cys Ser Val Val Ala Leu
   1               5                   10                  15
   Thr Ala Gly Ala His Ala Ala Pro Tyr Leu Lys Ser Ser Arg Ala Ser
               20                  25                  30
   Ser Asp Ser Val Thr Leu Glu Ala Glu Asp Ala Val Leu Ser Gly Thr
           35                  40                  45
   His Val Glu Thr Glu His Pro Gly Phe Thr Gly Thr Gly Tyr Val Thr
       50                  55                  60
   Gly Phe Asp Glu Ala Thr Asp Lys Val Thr Phe His Val Asp Ser Asp
   65                  70                  75                  80
   Ala Thr Lys Leu Tyr Asp Leu Ser Ile Arg Val Ala Ala Ile Tyr Gly
                   85                  90                  95
   Glu Lys Arg Thr Thr Val Val Leu Asn Gly Gly Ala Asn Ser Glu Val
               100                 105                 110
   Tyr Phe Pro Ala Ser Asp Thr Phe Asp Asp Ile Ala Ala Gly Gln Val
           115                 120                 125
   Leu Leu Glu Glu Gly Ala Asn Thr Ile Asp Ile Val Ser Asn Trp Gly
       130                 135                 140
   Trp Tyr Leu Ile Asp Ser Ile Thr Leu Thr Pro Ser Pro Pro Arg Pro
   145                 150                 155                 160
   Pro His Asn Ile Asn Glu Ala Pro Val Asn Ala Ala Ala Asp Ala Asn
                   165                 170                 175
   Ala Arg Ala Leu Tyr Asp Tyr Leu Arg Ser Ile Tyr Gly Lys Lys Ile
               180                 185                 190
   Leu Ser Gly Gln Gln Glu Leu Ser Tyr Ala Asp Trp Ile Ala Gln Gln
           195                 200                 205
   Thr Gly Lys Thr Pro Ala Leu Val Ser Val Asp Leu Met Asp Tyr Ser
       210                 215                 220
   Pro Ser Arg Val Glu Arg Gly Thr Val Gly Thr Ala Val Glu Glu Ala
   225                 230                 235                 240
   Ile Ala His His Glu Arg Gly Gly Ile Val Ser Val Leu Trp His Trp
                   245                 250                 255
   Asn Ala Pro Ala Gly Leu Tyr Asp Thr Asp Glu Asn Pro Trp Trp Ser
               260                 265                 270
   Gly Phe Tyr Thr Arg Ala Thr Asp Phe Asp Val Glu Ala Ala Leu Ser
   275 280 285
   Ser Pro Ala Asn Ala Asn Tyr Thr Leu Leu Val Arg Asp Ile Asp Ala
   290 295 300
   Ile Ala Glu Gln Leu Lys Arg Leu Arg Asp Ala Gly Val Pro Val Leu
   305 310 315 320
   Phe Arg Pro Leu His Glu Ala Glu Gly Gly Trp Phe Trp Trp Gly Ala
   325 330 335
   Lys Gly Pro Glu Pro Ala Lys Lys Leu Trp Ala Leu Leu Tyr Asp Arg
   340 345 350
   Leu Thr Asn His His Gln Ile His Asn Leu Leu Trp Val Trp Asn Ser
   355 360 365
   Ile Leu Pro Glu Trp Tyr Pro Gly Asp Asp Thr Val Asp Ile Leu Ser
   370 375 380
   Ala Asp Val Tyr Ala Gln Gly Asn Gly Pro Met Ser Thr Gln Tyr Asn
   385 390 395 400
   Gln Leu Ile Glu Leu Gly Gln Asp Lys Lys Met Ile Ala Ala Thr Glu
   405 410 415
   Val Gly Ala Ala Pro Leu Pro Asp Leu Leu Gln Ala Tyr Glu Ala His
   420 425 430
   Trp Leu Trp Phe Ala Val Trp Gly Asp Thr Phe Ile Asn Asn Pro Asp
   435 440 445
   Trp Asn Ser Pro Glu Thr Leu Arg Thr Ile Tyr His His Asp Tyr Val
   450 455 460
   Leu Thr Leu Asp Glu Ile Gln Gly Trp Arg Gly Asn
   465 470 475
<210> 34
   <211> 1360
   <212> DNA
   <213> Acremonium thermophilum
<400> 34
   atgcgttatc tgaccgctct gacccttggg ctcggggccg ctatggctac ggctctccca        60
   gccgcaaagg cagggtcagc agacggcgcc tcgaccttcc caagcgccaa tggcactcgt       120
   ttcaccatcg acggccagac tgcctacttt gctgggtcta acagctactg gatcggcttc       180
   ctgacgaaca acaacgacgt cgacaccgtg ctggaccaca tctcgcagtc cggtctcaag       240
   atcctgcggg tgtgggggtt caacgacgtc acccgcaacc cgggctcggg caccgtctgg       300
   taccagatgc tctcctcctc gggctcgcag atcaacacgg gtgccaacgg cctgcagcgc       360
   ttggactacg tcgtcaagtc ggccgagacc cgcggcgtca agctcatcgt caacttcgtc       420
   aacaactggg acgactacgg cggcatggcc gcctacgtgt cggccttcgg cggctcccac       480
   gagacctggt acaccaatac gcgggcgcag gaccagtacc gcaagtacat cgccgccgtc       540
   gtgggtcggt accgcaactc ctccgccatc ttcgcctggg agctcgccaa cgaaccccgc       600
   tgcaaggggt gcggcaccga cgtcatctac aagtgggcca ccgacacgtc tgcctacata       660
   cgagggctcg atccggagca catgatcacc ctcggcgacg aagggtttgg gcttcccggc       720
   gatacgacgt acccgtacgg tacgagcgag ggtgtcgact tcgtcaagaa tctgggcatc       780
   aaagacctgg actttggcac ttttcacatg tacccttcga gttgtgagtg tttcttcttt        840
   gccttcgaat ttttacccga agggtgcgtt gttgttgtgt ctaaagttat aggcatggga       900
   actgggagct aacagaaacc ttcttcatag ggggcgtttc gaacagcttt ggccccggtt        960
   ggattcaaaa tcacgccaaa gcctgcgccg ctgccaataa accctgcctt ctagaagaat      1020
   gtaagaagca actggtctga cccctcccta tccagtgatc cctattcgat cggcctctgt      1080
   atttgggccc gatcaggatt gtacaacgct aacagcacaa aacccttccg cagacggtgt      1140
   cggttcggac cactgcaacg tcgagaagag ctggcagcag gtatcactgg cgctggctac      1200
   agggggcatg ggaggcgacc tattctggca gtggggtgac aagctcagca acgggcaatc      1260
   ggccaacgac ggcaacacca tctattacgg ctccagcgac gggcagtgtc tcatcactga      1320
   ccatgtcaaa gccatcgagg cggcggaggc agcacagtga                             1360
<210> 35
   <211> 1140
   <212> DNA
   <213> Acremonium thermophilum
<400> 35
   atgcgttatc tgaccgctct gacccttggg ctcggggccg ctatggctac ggctctccca     60
   gccgcaaagg cagggtcagc agacggcgcc tcgaccttcc caagcgccaa tggcactcgt    120
   ttcaccatcg acggccagac tgcctacttt gctgggtcta acagctactg gatcggcttc    180
   ctgacgaaca acaacgacgt cgacaccgtg ctggaccaca tctcgcagtc cggtctcaag    240
   atcctgcggg tgtgggggtt caacgacgtc acccgcaacc cgggctcggg caccgtctgg    300
   taccagatgc tctcctcctc gggctcgcag atcaacacgg gtgccaacgg cctgcagcgc    360
   ttggactacg tcgtcaagtc ggccgagacc cgcggcgtca agctcatcgt caacttcgtc    420
   aacaactggg acgactacgg cggcatggcc gcctacgtgt cggccttcgg cggctcccac    480
   gagacctggt acaccaatac gcgggcgcag gaccagtacc gcaagtacat cgccgccgtc    540
   gtgggtcggt accgcaactc ctccgccatc ttcgcctggg agctcgccaa cgaaccccgc    600
   tgcaaggggt gcggcaccga cgtcatctac aagtgggcca ccgacacgtc tgcctacata    660
   cgagggctcg atccggagca catgatcacc ctcggcgacg aagggtttgg gcttcccggc    720
   gatacgacgt acccgtacgg tacgagcgag ggtgtcgact tcgtcaagaa tctgggcatc    780
   aaagacctgg actttggcac ttttcacatg tacccttcga gttggggcgt ttcgaacagc    840
   tttggccccg gttggattca aaatcacgcc aaagcctgcg ccgctgccaa taaaccctgc    900
   cttctagaag aatacggtgt cggttcggac cactgcaacg tcgagaagag ctggcagcag    960
   gtatcactgg cgctggctac agggggcatg ggaggcgacc tattctggca gtggggtgac   1020
   aagctcagca acgggcaatc ggccaacgac ggcaacacca tctattacgg ctccagcgac   1080
   gggcagtgtc tcatcactga ccatgtcaaa gccatcgagg cggcggaggc agcacagtga   1140
<210> 36
   <211> 379
   <212> PRT
   <213> Acremonium thermophilum
<400> 36
   Met Arg Tyr Leu Thr Ala Leu Thr Leu Gly Leu Gly Ala Ala Met Ala
   1               5                   10                  15
   Thr Ala Leu Pro Ala Ala Lys Ala Gly Ser Ala Asp Gly Ala Ser Thr
               20                  25                  30
   Phe Pro Ser Ala Asn Gly Thr Arg Phe Thr Ile Asp Gly Gln Thr Ala
           35                  40                  45
   Tyr Phe Ala Gly Ser Asn Ser Tyr Trp Ile Gly Phe Leu Thr Asn Asn
       50                  55                  60
   Asn Asp Val Asp Thr Val Leu Asp His Ile Ser Gln Ser Gly Leu Lys
   65                  70                  75                  80
   Ile Leu Arg Val Trp Gly Phe Asn Asp Val Thr Arg Asn Pro Gly Ser
                   85                  90                  95
   Gly Thr Val Trp Tyr Gln Met Leu Ser Ser Ser Gly Ser Gln Ile Asn
               100                 105                 110
   Thr Gly Ala Asn Gly Leu Gln Arg Leu Asp Tyr Val Val Lys Ser Ala
           115                 120                 125
   Glu Thr Arg Gly Val Lys Leu Ile Val Asn Phe Val Asn Asn Trp Asp
       130                 135                 140
   Asp Tyr Gly Gly Met Ala Ala Tyr Val Ser Ala Phe Gly Gly Ser His
   145                 150                 155                 160
   Glu Thr Trp Tyr Thr Asn Thr Arg Ala Gln Asp Gln Tyr Arg Lys Tyr
                   165                 170                 175
   Ile Ala Ala Val Val Gly Arg Tyr Arg Asn Ser Ser Ala Ile Phe Ala
               180                 185                 190
   Trp Glu Leu Ala Asn Glu Pro Arg Cys Lys Gly Cys Gly Thr Asp Val
           195                 200                 205
   Ile Tyr Lys Trp Ala Thr Asp Thr Ser Ala Tyr Ile Arg Gly Leu Asp
       210                 215                 220
   Pro Glu His Met Ile Thr Leu Gly Asp Glu Gly Phe Gly Leu Pro Gly
   225                 230                 235                 240
   Asp Thr Thr Tyr Pro Tyr Gly Thr Ser Glu Gly Val Asp Phe Val Lys
                   245                 250                 255
   Asn Leu Gly Ile Lys Asp Leu Asp Phe Gly Thr Phe His Met Tyr Pro
               260                 265                 270
   Ser Ser Trp Gly Val Ser Asn Ser Phe Gly Pro Gly Trp Ile Gln Asn
           275                 280                 285
   His Ala Lys Ala Cys Ala Ala Ala Asn Lys Pro Cys Leu Leu Glu Glu
       290                 295                 300
   Tyr Gly Val Gly Ser Asp His Cys Asn Val Glu Lys Ser Trp Gln Gln
   305                 310                 315                 320
   Val Ser Leu Ala Leu Ala Thr Gly Gly Met Gly Gly Asp Leu Phe Trp
                   325                 330                 335
   Gln Trp Gly Asp Lys Leu Ser Asn Gly Gln Ser Ala Asn Asp Gly Asn
               340                 345                 350
   Thr Ile Tyr Tyr Gly Ser Ser Asp Gly Gln Cys Leu Ile Thr Asp His
           355                 360                 365
   Val Lys Ala Ile Glu Ala Ala Glu Ala Ala Gln
       370                 375
<210> 37
   <211> 1371
   <212> DNA
   <213> Plectosphaerella cucumerina
<400> 37
   atgcgtctgg aactcctctc ggcagccggc ctcctcgccg ccggtgctgc tgcgcgctgc      60
   gtgcccaagg gcttcgtcac gacaaaaggc accgagttct acctcgacgg caagccattc     120
   cgcttcgccg gtagcaacgc ctactacttc cccttcaaca acgtacgtct cctgcctggg     180
   tggtgggtat ttgtctgtca gtggctaaca tggcatagaa ccaagccgat gttgaaaagg     240
   gcctgaacgc ggccaaggac gccggtctca aggtgttccg tacctggggt ttcaacgaca     300
   agaacgtgac gagcgactac gtcaacggtc tgccgcagta cggtggcgag ggcgcgggtg     360
   acacggagat tgtgttccag cggtgggaga atggcaagtc gaccatcaac ctcgagccct     420
   ttgacaaggt cgttgctgcg gcggaggcga cgggcatcaa gctcatcgtt gccttgacca     480
   acaactgggc cgactacggc ggtatggacg tgtacaccat taacttgggc tacaagtacc     540
   acgacgatgt aagttcatca tggaagcagt ccatgtcctt ggagtgtacc tgacatctgt     600
   gcacagttct acaaggagcc cgagatcaag gacgcgttca agcgctacgt caaggagttt     660
   gtgactcgct acaaggactc gccgtccatt ttcgcgtggg agctggccaa cgagccccgc     720
   tgcggcgctg atggcaagcg caacctgcct cgcagcgctg acggctgcaa ccccgaggtc     780
   atgtcggcgt ggaccgagga gctgagcgac tacgtccgct ccattgaccc gtaccatctc     840
   atcacctggg gtggcgaggg tgctttcaac agggcctcgg aggactgggc ctacaacggc     900
   gctgacggct ccgactttga ccacgagatg accctgaagg tgagcctagt tccctctctt     960
   ctttcattaa catcatgcta accctgccag aacatcgact ttggcacata ccacctgtac    1020
   cccgactggt ggtccaagac ggtcgagtgg tcgaaccagt ggatcctgga ccacgccgag    1080
   gtgggcgtca cggccaacaa gcccgtcgtg ttcgaggagt acggctggct cgaggccgac    1140
   aagcgcctcg agtggctcgg tcaggtgcgc aacgagacgc gcgtggaggt catcagccac    1200
   tggctcgagc tggcgcgcga ggtcaatcac ggcgcgatgt actggcagtt tggatactgc    1260
   gggtacagct acgggtgcaa ccacaacgat gggttcacta tttacctgga gaatgaggag    1320
   gcgcaggagc tggtgtatga ccatgctgct attgtcaatg gcgaggcata g             1371
<210> 38
   <211> 1206
   <212> DNA
   <213> Plectosphaerella cucumerina
<400> 38
   atgcgcctcg agctcctgtc cgccgccggc ctcctggctg ccggcgctgc cgcccgctgc 60
   gtccccaagg gcttcgtcac cacgaagggc accgagttct acctggacgg caagcctttc 120
   cgcttcgccg gctcgaacgc ctactacttc ccgttcaaca acaaccaggc tgacgtcgag 180
   aagggcctca acgctgctaa ggacgctggc ctgaaggtct tccgcacctg gggcttcaac 240
   gacaagaacg tcacgagcga ctacgtcaac ggcctccctc agtacggcgg cgagggcgcc 300
   ggcgacaccg agatcgtctt ccagcgctgg gagaacggca agtcgacgat caacctggag 360
   cccttcgaca aggtcgtcgc cgccgccgag gctaccggca tcaagctcat cgtcgccctg 420
   acgaacaact gggccgacta cggtggcatg gacgtctaca ccatcaacct cggctacaag 480
   taccacgacg acttctacaa ggagcctgag atcaaggacg ccttcaagcg ctacgtcaag 540
   gagttcgtca cgcgctacaa ggacagcccg tcgatcttcg cctgggagct ggctaacgag 600
   cctcgctgcg gcgctgacgg caagcgcaac ctccctcgca gcgccgacgg ctgcaaccct 660
   gaggtcatgt cggcctggac cgaggagctc tccgactacg tccgcagcat cgacccgtac 720
   cacctgatca cctggggcgg cgagggcgct ttcaaccgcg cttcggagga ctgggcttac 780
   aacggcgctg acggcagcga ctttgaccac gagatgaccc tcaagaacat cgacttcggc 840
   acgtaccacc tgtaccccga ctggtggtcg aagaccgtcg agtggtccaa ccagtggatt 900
   ctcgaccacg ctgaggtcgg cgtcaccgct aacaagcctg tcgtcttcga ggagtacggc 960
   tggctcgagg ctgacaagcg cctggagtgg ctcggccagg tccgcaacga gacgcgcgtc 1020
   gaggtcatca gccactggct ggagctggct cgcgaggtca accacggcgc catgtactgg 1080
   cagttcggct actgcggcta ctcctacggc tgcaaccaca acgacggctt cacgatctac 1140
   ctcgagaacg aggaagccca ggagctggtc tacgaccacg ctgctatcgt caacggcgag 1200
   gcctag 1206
<210> 39
   <211> 401
   <212> PRT
   <213> Plectosphaerella cucumerina
<400> 39
   Met Arg Leu Glu Leu Leu Ser Ala Ala Gly Leu Leu Ala Ala Gly Ala
   1               5                   10                  15
   Ala Ala Arg Cys Val Pro Lys Gly Phe Val Thr Thr Lys Gly Thr Glu
               20                  25                  30
   Phe Tyr Leu Asp Gly Lys Pro Phe Arg Phe Ala Gly Ser Asn Ala Tyr
           35                  40                  45
   Tyr Phe Pro Phe Asn Asn Asn Gln Ala Asp Val Glu Lys Gly Leu Asn
       50                  55                  60
   Ala Ala Lys Asp Ala Gly Leu Lys Val Phe Arg Thr Trp Gly Phe Asn
   65                  70                  75                  80
   Asp Lys Asn Val Thr Ser Asp Tyr Val Asn Gly Leu Pro Gln Tyr Gly
                   85                  90                  95
   Gly Glu Gly Ala Gly Asp Thr Glu Ile Val Phe Gln Arg Trp Glu Asn
               100                 105                 110
   Gly Lys Ser Thr Ile Asn Leu Glu Pro Phe Asp Lys Val Val Ala Ala
           115                 120                 125
   Ala Glu Ala Thr Gly Ile Lys Leu Ile Val Ala Leu Thr Asn Asn Trp
       130                 135                 140
   Ala Asp Tyr Gly Gly Met Asp Val Tyr Thr Ile Asn Leu Gly Tyr Lys
   145                 150                 155                 160
   Tyr His Asp Asp Phe Tyr Lys Glu Pro Glu Ile Lys Asp Ala Phe Lys
                   165                 170                 175
   Arg Tyr Val Lys Glu Phe Val Thr Arg Tyr Lys Asp Ser Pro Ser Ile
               180                 185                 190
   Phe Ala Trp Glu Leu Ala Asn Glu Pro Arg Cys Gly Ala Asp Gly Lys
           195                 200                 205
   Arg Asn Leu Pro Arg Ser Ala Asp Gly Cys Asn Pro Glu Val Met Ser
       210                 215                 220
   Ala Trp Thr Glu Glu Leu Ser Asp Tyr Val Arg Ser Ile Asp Pro Tyr
   225                 230                 235                 240
   His Leu Ile Thr Trp Gly Gly Glu Gly Ala Phe Asn Arg Ala Ser Glu
                   245                 250                 255
   Asp Trp Ala Tyr Asn Gly Ala Asp Gly Ser Asp Phe Asp His Glu Met
               260                 265                 270
   Thr Leu Lys Asn Ile Asp Phe Gly Thr Tyr His Leu Tyr Pro Asp Trp
           275                 280                 285
   Trp Ser Lys Thr Val Glu Trp Ser Asn Gln Trp Ile Leu Asp His Ala
       290                 295                 300
   Glu Val Gly Val Thr Ala Asn Lys Pro Val Val Phe Glu Glu Tyr Gly
   305                 310                 315                 320
   Trp Leu Glu Ala Asp Lys Arg Leu Glu Trp Leu Gly Gln Val Arg Asn
   325 330 335
   Glu Thr Arg Val Glu Val Ile Ser His Trp Leu Glu Leu Ala Arg Glu
   340 345 350
   Val Asn His Gly Ala Met Tyr Trp Gln Phe Gly Tyr Cys Gly Tyr Ser
   355 360 365
   Tyr Gly Cys Asn His Asn Asp Gly Phe Thr Ile Tyr Leu Glu Asn Glu
   370 375 380
   Glu Ala Gln Glu Leu Val Tyr Asp His Ala Ala Ile Val Asn Gly Glu
   385 390 395 400
   Ala
<210> 40
   <211> 1459
   <212> DNA
   <213> Gymnascella citrina
<400> 40
   atgcagctct cgaagctctt gacttacgct cttctcaccg gcgctgccgt tgcagctcct      60
   ggtcgtggcg gtggccatca cggcaggtct ccgcgcggct tcgtgacgac aaaagggacc     120
   gagttcaggc ttgacgggaa gaaatttcac ttcgctggca gcaacgctta ttacttccct     180
   ttcaacaacg tacgtttccg cataccatgt tactacggac ctagctaact cgcggtcaga     240
   accaatccga cgtcgagctc gggcttgcag cagccaaaaa ggccggtctc aacgtcttcc     300
   gcacatgggg cttcaatgac cgcaacgtga ccaccgaccc caatggactc ccacagtatg     360
   gaggcgaagg ggcgggtgag actggagtcg ttttccagtg gtgggataac gggaagccga     420
   ctatcgacct ggagccgttt gacaaggtgg tagctgctgc tgagaaggtt ggaatcaagc     480
   tcgtcgtggc tctgacgaat aactgggcgg attacggtgg catggacgtg tataccgtta     540
   accttggtgg gaagtatcac gacgatgtta gtgaacccct gcagaaaaga cgaagaagag     600
   aaactaacag cactcctagt tctaccgcct ccctcaaata aagcaggcgt acaagaacta     660
   cgtcaaggaa ttcgtaaccc gctaccgcga ctcccccacc atcatggcct gggaactcgg    720
   caacgaaccc cgctgcggtg ccgacggcgt gcgcaacctg cctcgcagcg acgactgcga     780
   cccgcacctc ttgacatcct ggatcgacga gatgagcacg tacatcaaga gccttgatcc     840
   gcaccacctg gtgacgtggg gcggagaggg agggttcaac agagagtcgg atgactgggc     900
   gtataacggg agcgatggag gggattttga cgccgagctt gcgctgaaga atatcgactt       960
   cggaaccttc cattcgtatc cggattggtg gtcgaagacg gtcgagtgga cggatcagtg 1020
   ggtgagggac cacggggaga gccagaggaa gggccggaaa ccggttgttc atgaggaata 1080
   tggtgaatag tactacctcg agcatcttgc ccggatcgca aaactaactc tgattgaaca 1140
   caggctggct cactccggaa gcacgctggg ataacctagg caccgtggtg aacatcacca 1200
   gaaccgaagc cgtcggcgga tggcagaaga tcagcgtcaa ggagcgcatg tccgacatgt 1260
   tttggcaatg ggggttctct gggtattcgt atggaagaaa ccacgacgac gggttcacca 1320
   tcttcgtcga cgagtaagtc cgcttcattc atcctcgtgg gaaacatttt gttgacgctt 1380
   gtaatcaaat agccccgaag cgaagcagct catctaccgc cacgccaaag aggttaatcg 1440
   actgaacaga cgtcgctag 1459
<210> 41
   <211> 1236
   <212> DNA
   <213> Gymnascella citrina
<400> 41
   atgcagctct ccaagctcct gacctacgcc ctcctgaccg gcgccgccgt cgctgctcct 60
   ggccgaggcg gcggccacca cggccgcagc cctcgcggct tcgtcaccac gaagggcacc 120
   gagttccgcc tggacggcaa gaagttccac ttcgccggca gcaacgccta ctacttcccg 180
   ttcaacaaca accagagcga cgtcgagctg ggcctggccg ccgccaagaa ggctggcctc 240
   aacgtcttcc gcacctgggg cttcaacgac cgcaacgtca ccacggaccc caacggcctg 300
   cctcagtacg gcggcgaggg cgctggcgag acgggcgtcg tcttccagtg gtgggacaac 360
   ggcaagccga cgatcgacct ggagcctttt gacaaggtcg tcgccgccgc cgagaaggtc 420
   ggcatcaagc tcgtcgtcgc cctgaccaac aactgggccg actacggtgg catggacgtc 480
   tacacggtca acctcggcgg caagtaccac gacgacttct accgcctgcc ccagatcaag 540
   caggcctaca agaactacgt caaggagttc gtcacccgct accgcgactc gccgacgatc 600
   atggcctggg agctcggcaa cgagcctcgc tgcggcgctg acggcgtccg caacctccct 660
   cgcagcgacg actgcgaccc tcacctcctg acctcctgga tcgacgagat gtccacgtac 720
   atcaagagcc tcgaccccca ccacctggtc acctggggcg gcgagggcgg cttcaaccgc 780
   gagtccgacg actgggctta caacggcagc gacggcggcg actttgacgc tgagctcgcc 840
   ctgaagaaca tcgacttcgg cacgttccac tcgtaccctg actggtggtc caagacggtc 900
   gagtggaccg accagtgggt ccgcgaccac ggcgagtcgc agcgcaaggg ccgcaagcct 960
   gtcgtccacg aggagtacgg ctggctcacc cctgaggctc gctgggacaa cctgggcacg 1020
   gtcgtcaaca tcacgcgcac cgaggctgtc ggcggctggc agaagatcag cgtcaaggag 1080
   cgcatgtcgg acatgttctg gcagtggggc ttcagcggct actcgtacgg ccgcaaccac 1140
   gacgacggct tcaccatctt cgtcgacgac cctgaggcca agcagctcat ctaccgccac 1200
   gccaaggaag tcaaccgcct caaccgccgc cgatag 1236
<210> 42
   <211> 411
   <212> PRT
   <213> Gymnascella citrina
<400> 42
   Met Gln Leu Ser Lys Leu Leu Thr Tyr Ala Leu Leu Thr Gly Ala Ala
   1 5 10 15
   Val Ala Ala Pro Gly Arg Gly Gly Gly His His Gly Arg Ser Pro Arg
   20 25 30
   Gly Phe Val Thr Thr Lys Gly Thr Glu Phe Arg Leu Asp Gly Lys Lys
   35 40 45
   Phe His Phe Ala Gly Ser Asn Ala Tyr Tyr Phe Pro Phe Asn Asn Asn
   50 55 60
   Gln Ser Asp Val Glu Leu Gly Leu Ala Ala Ala Lys Lys Ala Gly Leu
   65 70 75 80
   Asn Val Phe Arg Thr Trp Gly Phe Asn Asp Arg Asn Val Thr Thr Asp
   85 90 95
   Pro Asn Gly Leu Pro Gln Tyr Gly Gly Glu Gly Ala Gly Glu Thr Gly
   100 105 110
   Val Val Phe Gln Trp Trp Asp Asn Gly Lys Pro Thr Ile Asp Leu Glu
   115 120 125
   Pro Phe Asp Lys Val Val Ala Ala Ala Glu Lys Val Gly Ile Lys Leu
   130 135 140
   Val Val Ala Leu Thr Asn Asn Trp Ala Asp Tyr Gly Gly Met Asp Val
   145 150 155 160
   Tyr Thr Val Asn Leu Gly Gly Lys Tyr His Asp Asp Phe Tyr Arg Leu
   165 170 175
   Pro Gln Ile Lys Gln Ala Tyr Lys Asn Tyr Val Lys Glu Phe Val Thr
   180 185 190
   Arg Tyr Arg Asp Ser Pro Thr Ile Met Ala Trp Glu Leu Gly Asn Glu
   195 200 205
   Pro Arg Cys Gly Ala Asp Gly Val Arg Asn Leu Pro Arg Ser Asp Asp
       210                 215                 220
   Cys Asp Pro His Leu Leu Thr Ser Trp Ile Asp Glu Met Ser Thr Tyr
   225                 230                 235                 240
   Ile Lys Ser Leu Asp Pro His His Leu Val Thr Trp Gly Gly Glu Gly
                   245                 250                 255
   Gly Phe Asn Arg Glu Ser Asp Asp Trp Ala Tyr Asn Gly Ser Asp Gly
               260                 265                 270
   Gly Asp Phe Asp Ala Glu Leu Ala Leu Lys Asn Ile Asp Phe Gly Thr
           275                 280                 285
   Phe His Ser Tyr Pro Asp Trp Trp Ser Lys Thr Val Glu Trp Thr Asp
       290                 295                 300
   Gln Trp Val Arg Asp His Gly Glu Ser Gln Arg Lys Gly Arg Lys Pro
   305                 310                 315                 320
   Val Val His Glu Glu Tyr Gly Trp Leu Thr Pro Glu Ala Arg Trp Asp
                   325                 330                 335
   Asn Leu Gly Thr Val Val Asn Ile Thr Arg Thr Glu Ala Val Gly Gly
               340                 345                 350
   Trp Gln Lys Ile Ser Val Lys Glu Arg Met Ser Asp Met Phe Trp Gln
           355                 360                 365
   Trp Gly Phe Ser Gly Tyr Ser Tyr Gly Arg Asn His Asp Asp Gly Phe
       370                 375                 380
   Thr Ile Phe Val Asp Asp Pro Glu Ala Lys Gln Leu Ile Tyr Arg His
   385                 390                 395                 400
   Ala Lys Glu Val Asn Arg Leu Asn Arg Arg Arg
                   405                 410
<210> 43
   <211> 53
   <212> DNA
   <213> Artificial
<220>
   <223> oligonucleotide
<400> 43
   acccaatagt caaccgcgga ctgcgcatca tgcacctcac gaagcacctg act 53
<210> 44
   <211> 36
   <212> DNA
   <213> Artificial
<220>
   <223> oligonucleotide
<400> 44
   gttactgcag ggatcctcaa tggcgtccac ggttca 36
<210> 45
   <211> 60
   <212> DNA
   <213> artificial
<220>
   <223> oligonucleotide
<400> 45
   agtcaaccgc ggactgcgca tcatgaagtt atcatccttc gctttacccc tgttagcagg 60
<210> 46
   <211> 52
   <212> DNA
   <213> artificial
<220>
   <223> oilgonucleotide
<400> 46
   cgcgccggat ccctagcttg cgtttcgagt agcggcccaa tcgacaacga cc 52
<210> 47
   <211> 48
   <212> DNA
   <213> artificial
<220>
   <223> oligonucleotide
<400> 47
   acccaatagt caaccgcgga ctgcgcatca tgcgtttcga cattctct 48
<210> 48
   <211> 37
   <212> DNA
   <213> artificial
<220>
   <223> oligonucleotide
<400> 48
   gttactgcag ggatccctag ttctcgccgt tgacctt 37
<210> 49
   <211> 53
   <212> DNA
   <213> artificial
<220>
   <223> oligonucleotide
<400> 49
   acccaatagt caaccgcgga ctgcgcatca tgcatccacg catctcgaca ctt 53
<210> 50
   <211> 38
   <212> DNA
   <213> artificial
<220>
   <223> oligonucleotide
<400> 50
   gttactgcag ggatcctcat gcaagaacac cgttcatg 38
<210> 51
   <211> 53
   <212> DNA
   <213> artificial
<220>
   <223> oligonucleotide
<400> 51
   acccaatagt caaccgcgga ctgcgcatca tgctgttctc ggccccgcag ctt 53
<210> 52
   <211> 39
   <212> DNA
   <213> artificial
<220>
   <223> oligonucleotide
<400> 52
   gttactgcag ggatccttag tcacatctcg cctcgctat 39
<210> 53
   <211> 47
   <212> DNA
   <213> artificial
<220>
   <223> oligonucleotide
<400> 53
   acccaatagt caaccgcgga ctgcgcatca tgggcgtcag gtcactc 47
<210> 54
   <211> 41
   <212> DNA
   <213> artificial
<220>
   <223> oligonucleotide
<400> 54
   gttactgcag ggatcctcag ttgccccgcc agccctgaat c 41
<210> 55
   <211> 60
   <212> DNA
   <213> artificial
<220>
   <223> oligonucleotide
<400> 55
   agtcaaccgc ggactgcgca tcatgcgtta tctgaccgct ctgacccttg ggctcggggc 60
<210> 56
   <211> 54
   <212> DNA
   <213> artificial
<220>
   <223> oligonucleotide
<400> 56
   cgcgccggat cctcactgtg ctgcctccgc cgcctcgatg gctttgacat ggtc 54